# EUROPEAN PATENT APPLICATION

(11) **EP 2 017 277 A1**
(43) Date of publication of application: **21.01.2009**
(21) Application number: 07111460.7
(22) Date of filing: 29.06.2007
(51) Int. Cl.: C07D 471/04, A61K 31/4188, A61P 35/00

(54) **Thiophene-imidazopyridines**

(71) Applicant: 4SC AG, 82152 Planegg - Martinsried (DE)
(72) Inventor: The designation of the inventor has not yet been filed
(74) Representative: Vossius & Partner

(57) **Abstract**

The invention relates to thiophene-imidazopyridine compounds according to formula (I), 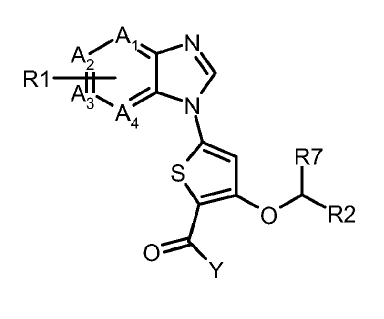 wherein the substituents and symbols are as defined in the description. The compounds are inhibitors of PIk1.

## Description

### Field of application of the invention

The invention relates to thiophene-imidazopyridine compounds which can be used in the pharmaceutical industry for the manufacture of pharmaceutical compositions. The invention further relates to the contribution made to the art by the finding that said thiophene-imidazopyridine compounds are potent inhibitors of polo-like kinase 1 (PIk1). This activity is known to result in many cases in a cell cycle-dependent, anti-proliferative and apoptosis inducing activity.
The invention also relates to the use of these compounds for the therapy of hyperproliferative diseases, in particular human cancer.

### Known technical background

Tumor cells are characterized by a partial or complete loss of control of the cell cycle. This loss of cell cycle control results in excessive cell division activity and, thus, in uncontrolled growth. It is known that PIk1, the human orthologue of polo kinase of Drosophila, is an essential regulator of the M-phase of the cell cycle. Targeted interference with Plk1 function in cancer cells such as antisense molecules, siRNA, or antibody microinjection is known to result in mitotic arrest of the cells followed by the onset of cell death. Moreover, it was found that Plk1 is over-expressed in a wide variety of human cancers including, but not limited to breast, prostate, stomach or ovaries.

Inhibitors of Plk1 are known from WO 03/93249, WO 2004/014899, WO 2004/043936, WO 2004/074244, WO 2004/087652, WO 2005/019193, WO 2005/037827, WO 2005/042505, WO 2005/075470, WO 2005/123736, WO 2006/008028, WO 2006/018185, WO 2006/018222, WO 2006/021544, WO 2006/021547, WO 2006/025567, WO 2006/049339, WO 2007/030359, WO 2007/030361 and WO 2007/030366.

Insufficient susceptibility to known medicines of many tumor types requires the development of novel compounds as chemotherapeutic agents such as, for example, Plk1 inhibiting compounds interfering with cancer cell cycle and/or proliferation. Thus, subject of the present invention are novel Plk1 inhibitors.

### Description of the invention

It has now been found that the thiophene-imidazopyridine compounds described in detail below are characterized by surprising and advantageous properties such as, among others, the selective inhibition of Plk1 enzyme. It can be expected that among these Plk1 inhibitors there will be compounds that selectively inhibit proliferation and induce cell death in proliferating cancer cells while being inactive on arrested cells. Moreover it was observed that many of the thiophene-imidazopyridine compounds arrest proliferating cancer cells in mitosis.

Accordingly, the invention relates to compounds of formula (I) 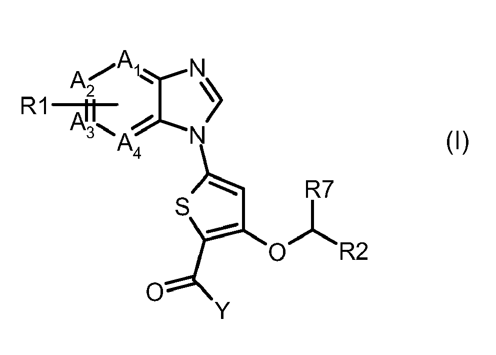 wherein
(l)
R1 is -H, -CH₂OH, -CH₂N(R3)R4, -C(O)N(R5)R6, -OCH₃, -F, -Cl, -Br, -CH₂SO₂CH₃, or -CH₂O-piperidyl, the piperidyl group optionally being N-substituted by C1-C4 alkyl,
and wherein R1 is attached to either A₂ or to A₃;
R2 is phenyl optionally having 1 to 3 substituents independently selected from -F, -Cl, -Br, -I, -CN, -OH, -NO₂, -SO₂CH₃, C1-C4 alkyl optionally being substituted with 1 to 3 F atoms, C1-C4 alkoxy optionally being substituted with 1 to 3 F atoms, C1-C4 alkylamino, and C1-C4 dialkylamino;
A₁, A₂, A₃ and A₄ independently are CR1, CH or N,
wherein one of A₁, A₂, A₃ and A₄ is N, one of A₂ and A₃ is CR1, and all others are CH;
Y is -OH or -NH₂;
R3 is -H, -SO₂CH₃, C1-C4 alkyl, C2-C3 alkyl substituted with -OR8, C2-C3 alkyl substituted with -N(R8)R9, C3-C6 cycloalkyl, or saturated four- to six-membered heterocyclyl with at least one ring atom being N, the heterocyclyl group optionally being N-substituted with C1-C4 alkyl, phenyl, pyridyl, or pyrimidyl;
R4 is -H, -SO₂CH₃, C1-C4 alkyl, C2-C3 alkyl substituted with -OR8, C2-C3 alkyl substituted with -N(R8)R9, C3-C6 cycloalkyl, or saturated four- to six-membered heterocyclyl with at least one ring atom being N, the heterocyclyl group optionally being N-substituted with C1-C4 alkyl, phenyl, pyridyl, or pyrimidyl;
or
R3 and R4, together with the nitrogen atom they are bound to, form a saturated four- to seven-membered heterocycle, the heterocycle optionally containing one more heteroatom which is N, O or S, S optionally being oxidized to a -SO- group or a -SO₂- group,
and the heterocycle optionally being substituted by one or two substituents which independently are -OH, -F, amino, C1-C4 alkylamino, C1-C4 dialkylamino, C1-C4 alkyl optionally being substituted with 1 to 3 F atoms, C1-C4 acyl, benzoyl, phenoxy, 1-carboxamidyl optionally being substituted with C1-C4 alkyl or phenyl, 1-carboximidamidyl, sulfonyl optionally being substituted with C1-C4 alkyl or phenyl, 2-aminoethyl, 2-N-(C1-C4 alkyl)aminoethyl, 2-N,N-di(C1-C4 alkyl)aminoethyl, phenyl, pyridyl, pyrimidyl, benzyl, allyl, cyclopropyl, cyclobutyl, cyclopentyl, cyclohexyl, cyanomethyl, 2-cyanoethyl, 2-methansulfonylethyl, hydroxymethyl, 2-hydroxyethyl, 3-hydroxypropyl, (C1-C4 alkoxy)methyl, 2-(C1-C4 alkoxy)ethyl, 3-(C1-C4 alkoxy)propyl, N-methylpiperidyl, 1-phenylethyl, pyridylmethyl, N-methylpiperidylmethyl, 2-morpholinylethyl, morpholinocarbonylethyl, N,N-dimethylcarbonylmethyl, anilinocarbonylmethyl, N-methylanilinocarbonylmethyl, N-pyrrolidinocarbonylmethyl, N,N-dimethylsulfonyl, N-methylpiperazinyl, N-methylpiperazinylcarbonyl, or oxo,
or
R3 and R4, together with the nitrogen atom they are bound to, form a bridged system which is 2,5-diazabicyclo[2.2.1]hept-2-yl or 5-methyl-2,5-diazabicyclo [2.2.1]hept-2-yl;
R5 is -H, methyl, C1-C4 alkoxyethyl, or four- to six-membered N-ethylheterocyclyl;
R6 is -H, methyl, C1-C4 alkoxyethyl, or four- to six-membered N-ethylheterocyclyl;
R7 is -H, methyl, ethyl, -CH₂OH or -CF₃;
R8 is -H or C1-C4 alkyl; and
R9 is -H or C1-C4 alkyl;
or
R8 and R9, together with the nitrogen atom they are bound to, form a saturated four- to six-membered heterocycle, the heterocycle optionally containing one more heteroatom which is N, O or S, S optionally being oxidized to a -SO-group or a *-*SO₂*-* group;
a salt of one of the compounds, a stereoisomer of one of the compounds, or a salt of a stereoisomer of one of the compounds.

In a preferred embodiment, the invention relates to compounds of formula (I), wherein
R1 is -H, -CH₂OH, -CH₂N(R3)R4, -C(O)N(R5)R6, -OCH₃, -F, -Cl, -Br, -CH₂SO₂CH₃, or -CH₂O-piperidyl, the piperidyl group optionally being N-substituted by C1-C4 alkyl,
and wherein R1 is attached to either A₂ or to A₃;
R2 is phenyl optionally having 1 to 3 substituents independently selected from -F, -Cl, -Br, I, -CN, -SO₂CH₃, C1-C4 alkyl optionally being substituted with 1 to 3 F atoms, C1-C4 alkoxy optionally being substituted with 1 to 3 F atoms, C1-C4 alkylamino, and C1-C4 dialkylamino;
A₁ and A₄ are C, one of A₂ and A₃ is CR1 and the other of A₂ and A₃ is N;
Y is -OH or -NH₂;
R3 is -H, -SO₂CH₃, C1-C4 alkyl, C2-C3 alkyl substituted with -OR8, C2-C3 alkyl substituted with -N(R8)R9, C3-C6 cycloalkyl, piperidine-4-yl optionally being N-substituted with C1-C4 alkyl, phenyl, pyridyl, or pyrimidyl, or piperidine-3-yl optionally being N-substituted with C1-C4 alkyl, phenyl, pyridyl, or pyrimidyl;
R4 is -H, -SO₂CH₃, C1-C4 alkyl, C2-C3 alkyl substituted with -OR8, C2-C3 alkyl substituted with -N(R8)R9, C3-C6 cycloalkyl, piperidine-4-yl optionally being N-substituted with C1-C4 alkyl, phenyl, pyridyl, or pyrimidyl, or piperidine-3-yl optionally being N-substituted with C1-C4 alkyl, phenyl, pyridyl, or pyrimidyl;
or
R3 and R4, together with the nitrogen atom they are bound to, form a saturated four- to seven-membered heterocycle, the heterocycle optionally containing one more heteroatom which is N, O or S, S optionally being oxidized to a -SO- group or a -SO₂- group,
and the heterocycle optionally being substituted by one or two substituents which independently are -OH, -F, amino, C1-C4 alkylamino, C1-C4 dialkylamino, C1-C4 alkyl optionally being substituted with 1 to 3 F atoms, C1-C4 acyl, benzoyl, phenoxy, 1-carboxamidyl optionally being substituted with C1-C4 alkyl or phenyl, 1-carboximidamidyl, sulfonyl optionally being substituted with C1-C4 alkyl or phenyl, 2-aminoethyl, 2-N-(C1-C4 alkyl)aminoethyl, 2-N,N-di(C1-C4 alkyl)aminoethyl, phenyl, pyridyl, pyrimidyl, benzyl, allyl, cyclopropyl, cyclobutyl, cyclopentyl, cyclohexyl, cyanomethyl, 2-cyanoethyl, 2-methansulfonylethyl, hydroxymethyl, 2-hydroxyethyl, 3-hydroxypropyl, (C1-C4 alkoxy)methyl, 2-(C1-C4 alkoxy)ethyl, 3-(C1-C4 alkoxy)propyl, N-methylpiperidyl, 1-phenylethyl, pyridylmethyl, N-methylpiperidylmethyl, 2-morpholinylethyl, morpholinocarbonylethyl, N,N-dimethylcarbonylmethyl, anilinocarbonylmethyl, N-methylanilinocarbonylmethyl, N-pyrrolidinocarbonylmethyl, N,N-dimethylsulfonyl, N-methylpiperazinyl, N-methylpiperazinylcarbonyl, or oxo,
or
R3 and R4, together with the nitrogen atom they are bound to, form a bridged system which is 2,5-diazabicyclo[2.2.1]hept-2-yl or 5-methyl-2,5-diazabicyclo [2.2.1]hept-2-yl;
R5 is -H, methyl, C1-C4 alkoxyethyl, or four- to six-membered N-ethylheterocyclyl;
R6 is -H, methyl, C1-C4 alkoxyethyl, or four- to six-membered N-ethylheterocyclyl;
R7 is -H, methyl, ethyl, -CH₂OH, or -CF₃;
R8 is -H or C1-C4 alkyl; and
R9 is -H or C1-C4 alkyl;
or
R8 and R9, together with the nitrogen atom they are bound to, form a saturated four- to six-membered heterocycle, the heterocycle optionally containing one more heteroatom which is N, O or S, S optionally being oxidized to a -SO-group or a -SO₂- group;
a salt of one of the compounds, a stereoisomer of one of the compounds, or a salt of a stereoisomer of one of the compounds.

In another preferred embodiment, the invention relates to compounds of formula (I), wherein
R1 is -H, -CH₂N(R3)R4, -CH₂SO₂CH₃, or -CH₂O-piperidyl, the piperidyl group optionally being N-substituted by C1-C4 alkyl, and wherein R1 is attached to either A₂ or to A₃;
R2 is phenyl optionally having 1 to 3 substituents independently selected from -F, -Cl, -Br, I, -CN, -SO₂CH₃, C1-C4 alkyl optionally being substituted with 1 to 3 F atoms, C1-C4 alkoxy optionally being substituted with 1 to 3 F atoms, C1-C4 alkylamino, and C1-C4 dialkylamino;
A₁ and A₄ are C, one of A₂ and A₃ is CR1 and the other of A₂ and A₃ is N;
Y is -OH or -NH₂;
R3 is -H, C2-C3 alkyl substituted with -OR8, C2-C3 alkyl substituted with -N(R8)R9, C3-C6 cycloalkyl, piperidine-4-yl optionally being N-substituted with C1-C4 alkyl, phenyl, pyridyl, or pyrimidyl, or piperidine-3-yl optionally being N-substituted with C1-C4 alkyl, phenyl, pyridyl, or pyrimidyl;
R4 is -H, C2-C3 alkyl substituted with -OR8, C2-C3 alkyl substituted with -N(R8)R9, C3-C6 cycloalkyl, piperidine-4-yl optionally being N-substituted with C1-C4 alkyl, phenyl, pyridyl, or pyrimidyl, or piperidine-3-yl optionally being N-substituted with C1-C4 alkyl, phenyl, pyridyl, or pyrimidyl;
or
R3 and R4, together with the nitrogen atom they are bound to, form a saturated four- to seven-membered heterocycle, the heterocycle optionally containing one more heteroatom being N, O or S, S optionally being oxidized to a -SO- group or a -SO₂- group,
and the heterocycle optionally being substituted by one or two substituents which independently are -OH, -F, amino, C1-C4 alkylamino, C1-C4 dialkylamino, C1-C4 alkyl optionally being substituted with 1 to 3 F atoms, C1-C4 acyl, benzoyl, phenoxy, 1-carboxamidyl optionally being substituted with C1-C4 alkyl or phenyl, 1-carboximidamidyl, sulfonyl optionally being substituted with C1-C4 alkyl or phenyl, 2-aminoethyl, 2-N-(C1-C4 alkyl)aminoethyl, 2-N,N-di(C1-C4 alkyl)aminoethyl, phenyl, pyridyl, pyrimidyl, benzyl, allyl, cyclopropyl, cyclobutyl, cyclopentyl, cyclohexyl, cyanomethyl, 2-cyanoethyl, 2-methansulfonylethyl, hydroxymethyl, 2-hydroxyethyl, 3-hydroxypropyl, (C1-C4 alkoxy)methyl, 2-(C1-C4 alkoxy)ethyl, 3-(C1-C4 alkoxy)propyl, N-methylpiperidyl, 1-phenylethyl, pyridylmethyl, N-methylpiperidylmethyl, 2-morpholinylethyl, morpholinocarbonylethyl, N,N-dimethylcarbonylmethyl, anilinocarbonylmethyl, N-methylanilinocarbonylmethyl, N-pyrrolidinocarbonylmethyl, N,N-dimethylsulfonyl, N-methylpiperazinyl, N-methylpiperazinylcarbonyl, or oxo,
or
R3 and R4, together with the nitrogen atom they are bound to, form a bridged system which is 2,5-diazabicyclo[2.2.1]hept-2-yl or 5-methyl-2,5-diazabicyclo [2.2.1]hept-2-yl;
R7 is -H, methyl, ethyl, -CH₂OH, or -CF₃;
R8 is -H or C1-C4 alkyl; and
R9 is -H or C1-C4 alkyl;
or
R8 and R9, together with the nitrogen atom they are bound to, form a saturated four- to six-membered heterocycle optionally containing one more heteroatom being N, O or S, S optionally being oxidized to a -SO- group or a -SO₂- group;
a salt of one of the compounds, a stereoisomer of one of the compounds, or a salt of a stereoisomer of one of the compounds.

In another preferred embodiment, the invention relates to compounds of formula (I), wherein
R1 is -CH₂N(R3)R4, -CH₂SO₂CH₃, or -CH₂O-piperidyl, the piperidyl group optionally being N-substituted by C1-C4 alkyl, and wherein R1 is attached to either A₂ or to A₃;
R2 is phenyl having a substituent in the 2-position and optionally up to two substituents in the remaining positions, the substituents being -F, -Cl, -Br, -I, -CN, -SO₂CH₃, C1-C4 alkyl optionally being substituted with 1 to 3 F atoms, C1-C4 alkoxy optionally being substituted with 1 to 3 F atoms, C1-C4 alkylamino, or C1-C4 dialkylamino;
A₁ and A₄ are C, one of A₂ and A₃ is CR1 and the other of A₂ and A₃ is N;
Y is -NH₂;
R3 is -H or piperidine-4-yl optionally being N-substituted with C1-C4 alkyl; and
R4 is -H or piperidine-4-yl optionally being N-substituted with C1-C4 alkyl;
or
R3 and R4, together with the nitrogen atom they are bound to, form a piperazine ring or 1,4-diazepane ring, the piperazine or 1,4-diazepane optionally being substituted by one or two substituents independently being -F, C1-C4 alkyl, C1-C4 alkyl substituted with 1 to 3 F atoms, C1-C4 acyl, benzoyl, 1-carboxamidyl optionally being substituted with C1-C4 alkyl or phenyl, 1-carboximidamidyl, sulfonyl optionally being substituted with C1-C4 alkyl or phenyl, 2-aminoethyl, 2-N-(C1-C4 alkyl)aminoethyl, 2-N,N-di(C1-C4 alkyl)aminoethyl, phenyl, pyridyl, pyrimidyl, benzyl, allyl, cyclopropyl, cyclobutyl, cyclopentyl, cyclohexyl, cyanomethyl, 2-cyanoethyl, 2-methansulfonylethyl, hydroxymethyl, 2-hydroxyethyl, 3-hydroxypropyl, (C1-C4 alkoxy)methyl, 2-(C1-C4 alkoxy)ethyl, 3-(C1-C4 alkoxy)propyl, N-methylpiperidyl, 1-phenylethyl, pyridylmethyl, N-methylpiperidylmethyl, 2-morpholinylethyl, morpholinocarbonylethyl, N,N-dimethylcarbonylmethyl, anilinocarbonylmethyl, N-methylanilinocarbonylmethyl, N-pyrrolidinocarbonylmethyl, N,N-dimethylsulfonyl, or oxo,
or
R3 and R4, together with the nitrogen atom they are bound to, form a bridged system which is 2,5-diazabicyclo[2.2.1]hept-2-yl or 5-methyl-2,5-diazabicyclo [2.2.1]hept-2-yl;
R7 is -H, methyl, -CH₂OH, or -CF₃;
a salt of one of the compounds, a stereoisomer of one of the compounds, or a salt of a stereoisomer of one of the compounds.

In another preferred embodiment, the invention relates to compounds of formula (I), wherein
R1 is -CH₂N(R3)R4 and is attached to either A₂ or to A₃;
R2 is phenyl having a -F, -Cl, -Br, -I, trifluormethyl, difluormethoxy or trifluormethoxy substituent in the 2-position and optionally up to two substituents in the remaining positions, the up to two substituents independently being -F, -Cl, -Br, I, -CN, -SO₂CH₃, C1-C4 alkyl optionally being substituted with 1 to 3 F atoms, C1-C4 alkoxy optionally being substituted with 1 to 3 F atoms, C1-C4 alkylamino, or C1-C4 dialkylamino;
A₁ and A₄ are CH, one of A₂ and A₃ is CR1 and the other of A₂ and A₃ is N;
Y is -NH₂;
R3 and R4, together with the nitrogen atom they are bound to, form a piperazine ring or 1,4-diazepane ring, the piperazine or 1,4-diazepane optionally being substituted by one or two substituents independently being -F, C1-C4 alkyl optionally being substituted with 1 to 3 F atoms, C1-C4 acyl, benzoyl, 1-carboxamidyl optionally being substituted with C1-C4 alkyl or phenyl, 1-carboximidamidyl, sulfonyl optionally being substituted with C1-C4 alkyl or phenyl, 2-aminoethyl, 2-N-(C1-C4 alkyl)aminoethyl, 2-N,N-di(C1-C4 alkyl)aminoethyl; phenyl, pyridyl, pyrimidyl, benzyl, allyl, cyclopropyl, cyclobutyl, cyclopentyl, cyclohexyl, cyanomethyl, 2-cyanoethyl, 2-methansulfonylethyl, hydroxymethyl, 2-hydroxyethyl, 3-hydroxypropyl, (C1-C4 alkoxy)methyl, 2-(C1-C4 alkoxy)ethyl, 3-(C1-C4 alkoxy)propyl, N-methylpiperidyl, 1-phenylethyl, pyridylmethyl, N-methylpiperidylmethyl, 2-morpholinylethyl, morpholinocarbonylethyl, N,N-dimethylcarbonylmethyl, anilinocarbonylmethyl, N-methylanilinocarbonylmethyl, N-pyrrolidinocarbonylmethyl, N,N-dimethylsulfonyl, or oxo;
R7 is -H, methyl, -CH₂OH, or -CF₃;
a salt of one of the compounds, a stereoisomer of one of the compounds, or a salt of a stereoisomer of one of the compounds.

In another preferred embodiment, the invention relates to compounds of formula (I), wherein
R1 is -CH₂N(R3)R4 and is attached to either A₂ or to A₃;
A₁ and A₄ are CH;
one of A₂ and A₃ is CR1 and the other is N;
R2 is phenyl having a -Cl, -Br, trifluormethyl, difluormethoxy or trifluormethoxy group in 2-position and optionally up to two substituents in the remaining positions, the substituents being -F, -Cl, -Br, -CN, -SO₂Me, C1-C4 alkyl, C1-C4 alkyl substituted with 1 to 3 F atoms, C1-C4 alkoxy, C1-C4 alkoxy substituted with 1 to 3 F atoms, C1-C4 alkylamino, or C1-C4 dialkylamino;
Y is -NH₂;
R3 and R4, together with the nitrogen atom they are bound to, form a piperazine ring, the piperazine optionally being substituted by one substituent being C1-C4 alkyl, C1-C4 alkyl substituted with 1 to 3 F atoms, or cyclopropyl;
R7 is methyl -CH₂OH, or CF₃.
a salt of one of the compounds, a stereoisomer of one of the compounds, or a salt of a stereoisomer of one of the compounds.

In another preferred embodiment, the invention relates to compounds of formula (I), wherein
R1 is -CH₂N(R3)R4 and is attached to A₃;
A₁ and A₄ are CH;
A₂ is N;
A₃ is CR1;
R2 is phenyl having a -Cl, -Br, trifluormethyl, difluormethoxy or trifluormethoxy group in 2-position;
Y is -NH₂;
R3 and R4, together with the nitrogen atom they are bound to, form a piperazine ring, the piperazine optionally being substituted by one substituent being C1-C4 alkyl, C1-C4 alkyl substituted with 1 to 3 F atoms, or cyclopropyl,
R7 is methyl, -CH₂OH, or CF₃;
a salt of one of the compounds, a stereoisomer of one of the compounds, or a salt of a stereoisomer of one of the compounds.

In another preferred embodiment, the invention relates to compounds of formula (I), wherein
R1 is -CH₂N(R3)R4 and is attached to A₃;
A₁ and A₄ are CH;
A₂ is N;
A₃ is CR1;
R2 is phenyl having trifluormethyl in 2-position;
Y is -NH₂;
R3 and R4, together with the nitrogen atom they are bound to, form a piperazine ring, the piperazine ring optionally being substituted by one or two substituents being C1-C4 alkyl, C1-C4 alkyl substituted with 1 to 3 F atoms, cyclopropyl, C1-C4 acyl, benzoyl, 1-carboxamidyl, 1-carboxamidyl substituted with C1-C4 alkyl, 1-carboxamidyl substituted with phenyl, 1-carboximidamidyl, sulfonyl, sulfonyl substituted with C1-C4 alkyl, sulfonyl substituted with phenyl, hydroxyethyl, 2-(C1-C4 alkoxy)ethyl, 2-aminoethyl, 2-N-(C1-C4 alkyl)aminoethyl, 2-N,N-di(C1-C4 alkyl)aminoethyl; phenyl, pyridyl, or oxo;
R7 is methyl;
a salt of one of the compounds, a stereoisomer of one of the compounds, or a salt of a stereoisomer of one of the compounds.

In another preferred embodiment, the invention relates to compounds of formula (I), wherein
R1 is -CH₂O-piperidyl, the piperidyl group optionally being N-substituted by C1-C4 alkyl, and is attached to either A₂ or to A₃;
A₁ and A₄ are CH;
one of A₂ and A₃ is CR1 and the other is N;
R2 is phenyl having a -Cl, -Br, trifluormethyl, difluormethoxy or trifluormethoxy group in 2-position and optionally up to two substituents in the remaining positions, the substituents being -F, -Cl, -Br, -CN, -SO₂Me, C1-C4 alkyl, C1-C4 alkyl substituted with 1 to 3 F atoms, C1-C4 alkoxy, C1-C4 alkoxy substituted with 1 to 3 F atoms, C1-C4 alkylamino, or C1-C4 dialkylamino;
Y is -NH₂;
R7 is methyl, -CH₂OH, or CF₃;
a salt of one of the compounds, a stereoisomer of one of the compounds, or a salt of a stereoisomer of one of the compounds.

In another preferred embodiment, the invention relates to compounds of formula (I), wherein
R1 is -CH₂O-piperidyl, the piperidyl group optionally being N-substituted by C1-C4 alkyl and is attached to A₃;
A₁ and A₄ are CH;
A₂ is N;
R2 is phenyl having a -Cl, -Br, trifluormethyl, difluormethoxy or trifluormethoxy group in 2-position;
Y is -NH₂;
R7 is methyl, -CH₂OH, or CF₃;
a salt of one of the compounds, a stereoisomer of one of the compounds, or a salt of a stereoisomer of one of the compounds.

In another preferred embodiment, the invention relates to compounds of formula (I), wherein
R1 is -CH₂SO₂CH₃,
and wherein R1 is attached to either A₂ or to A₃;
A₁ and A₄ are CH;
one of A₂ and A₃ is CR1 and the other is N;
R2 is phenyl having a -Cl, -Br, trifluormethyl, difluormethoxy or trifluormethoxy group in 2-position and optionally up to two substituents in the remaining positions, the substituents being -F, -Cl, -Br, -CN, -SO₂Me, C1-C4 alkyl, C1-C4 alkyl substituted with 1 to 3 F atoms, C1-C4 alkoxy, C1-C4 alkoxy substituted with 1 to 3 F atoms, C1-C4 alkylamino, or C1-C4 dialkylamino;
Y is -NH₂;
R7 is methyl, -CH₂OH, or CF₃;
a salt of one of the compounds, a stereoisomer of one of the compounds, or a salt of a stereoisomer of one of the compounds.

In another preferred embodiment, the invention relates to compounds of formula (I), wherein
R1 is -CH₂SO₂CH₃ and is attached to A₃;
A₁ and A₄ are CH;
A₂ is N;
R2 is phenyl having a -Cl, -Br, trifluormethyl, difluormethoxy or trifluormethoxy group in 2-position;
Y is -NH₂;
R7 is methyl, -CH₂OH, or CF₃;
a salt of one of the compounds, a stereoisomer of one of the compounds, or a salt of a stereoisomer of one of the compounds.

In another preferred embodiment, the invention relates to compounds of formula (I), wherein
R1 is -CH₂N(R3)R4 and wherein R1 is attached to either A₂ or to A₃;
A₁ and A₄ are CH;
one of A₂ and A₃ is CR1 and the other is N;
R3 is -H, piperidine-4-yl or piperidine-4-yl being N-substituted with C1-C4 alkyl;
R4 is -H, piperidine-4-yl or piperidine-4-yl being N-substituted with C1-C4 alkyl;
R2 is phenyl having a -Cl, -Br, trifluormethyl, difluormethoxy or trifluormethoxy group in 2-position and optionally up to two substituents in the remaining positions, the substituents being -F, -Cl, -Br, -CN, -SO₂Me, C1-C4 alkyl, C1-C4 alkyl substituted with 1 to 3 F atoms, C1-C4 alkoxy, C1-C4 alkoxy substituted with 1 to 3 F atoms, C1-C4 alkylamino, or C1-C4 dialkylamino;
Y is -NH₂;
R7 is methyl, -CH₂OH, or CF₃;
a salt of one of the compounds, a stereoisomer of one of the compounds, or a salt of a stereoisomer of one of the compounds.

In another preferred embodiment, the invention relates to compounds of formula (I), wherein
R1 is -CH₂N(R3)R4 and is attached to A₃;
A₁ and A₄ are CH;
A₂ is N;
A₃ is CR1;
R3 is -H, piperidine-4-yl or piperidine-4-yl being N-substituted with C1-C4 alkyl;
R4 is -H, piperidine-4-yl or piperidine-4-yl being N-substituted with C1-C4 alkyl;
R2 is phenyl having a -Cl, -Br, trifluormethyl, difluormethoxy or trifluormethoxy group in 2-position;
Y is -NH₂;
R7 is methyl, -CH₂OH, or CF₃;
a salt of one of the compounds, a stereoisomer of one of the compounds, or a salt of a stereoisomer of one of the compounds.

In another preferred embodiment, the invention relates to compounds of of formula (I), wherein R1 is N-methylpiperazine.

In another preferred embodiment, the invention relates to compounds of of formula (I), wherein R1 is -CH₂N(R3)R4.

In another preferred embodiment, the invention relates to compounds of of formula (I), wherein R1 is -CH₂N(R3)R4 and is attached to A₃.

In another preferred embodiment, the invention relates to compounds of of formula (I), wherein R1 is -CH₂O-piperidyl, the piperidyl group optionally being N-substituted by C1-C4 alkyl, and is attached to either A₂ or to A₃.

In another preferred embodiment, the invention relates to compounds of of formula (I), wherein R1 attached to A₂.

In another preferred embodiment, the invention relates to compounds of of formula (I), wherein R1 is attached to A₃.

In another preferred embodiment, the invention relates to compounds of of formula (I), wherein R1 is -CH₂SO₂CH₃.

In another preferred embodiment, the invention relates to compounds of of formula (I), wherein R2 is phenyl having a substituent in the 2-position and optionally up to two substituents in the remaining positions, the substituents being -F, - Cl, -Br, -l, -CN, -SO₂CH₃, C1-C4 alkyl optionally being substituted with 1 to 3 F atoms, C1-C4 alkoxy optionally being substituted with 1 to 3 F atoms, C1-C4 alkylamino, or C1-C4 dialkylamino.

In another preferred embodiment, the invention relates to compounds of of formula (I), wherein R2 is phenyl having a -Cl, -Br, trifluormethyl, difluormethoxy or trifluormethoxy group in 2-position.

In another preferred embodiment, the invention relates to compounds of of formula (I), wherein R2 is 2-chlorphenyl.

In another preferred embodiment, the invention relates to compounds of of formula (I), wherein R2 is 2-trifluormethylphenyl.

In another preferred embodiment, the invention relates to compounds of of formula (I), wherein Y is -NH₂.

In another preferred embodiment, the invention relates to compounds of of formula (I), wherein R3 is piperidine-4-yl being N-substituted with C1-C4 alkyl and R4 is H.

In another preferred embodiment, the invention relates to compounds of of formula (I), wherein R3 is piperidine-4-yl and R4 is H.

In another preferred embodiment, the invention relates to compounds of of formula (I), wherein A₂ and A₃ are CR1 or N.

In another preferred embodiment, the invention relates to compounds of of formula (I), wherein A₂ is CR1.

In another preferred embodiment, the invention relates to compounds of of formula (I), wherein A₃ is CR1.

In another preferred embodiment, the invention relates to compounds of of formula (I), wherein A₂ is N.

In another preferred embodiment, the invention relates to compounds of of formula (I), wherein A₃ is N.

In another preferred embodiment, the invention relates to compounds of of formula (I), wherein R3 is piperidine-4-yl being N-substituted with C1-C4 alkyl and R4 is H.

In another preferred embodiment, the invention relates to compounds of of formula (I), wherein R3 is piperidine-4-yl and R4 is H.

In another preferred embodiment, the invention relates to compounds of of formula (I), wherein R3 and R4, together with the nitrogen atom they are bound to, form a piperazine ring, the piperazine ring optionally being substituted by one or two substituents independently being -F, C1-C4 alkyl, C1-C4 alkyl substituted with 1 to 3 F atoms, C1-C4 acyl, benzoyl, 1-carboxamidyl optionally being substituted with C1-C4 alkyl or phenyl, 1-carboximidamidyl, sulfonyl optionally being substituted with C1-C4 alkyl or phenyl, 2-aminoethyl, 2-N-(C1-C4 alkyl)aminoethyl, 2-N,N-di(C1-C4 alkyl)aminoethyl, phenyl, pyridyl, pyrimidyl, benzyl, allyl, cyclopropyl, cyclobutyl, cyclopentyl, cyclohexyl, cyanomethyl, 2-cyanoethyl, 2-methansulfonylethyl, hydroxymethyl, 2-hydroxyethyl, 3-hydroxypropyl, (C1-C4 alkoxy)methyl, 2-(C1-C4 alkoxy)ethyl, 3-(C1-C4 alkoxy)propyl, N-methylpiperidyl, 1-phenylethyl, pyridylmethyl, N-methylpiperidylmethyl, 2-morpholinylethyl, morpholinocarbonylethyl, N,N-dimethylcarbonylmethyl, anilinocarbonylmethyl, N-methylanilinocarbonylmethyl, N-pyrrolidinocarbonylmethyl, N,N-dimethylsulfonyl, or oxo.

In another preferred embodiment, the invention relates to compounds of of formula (I), wherein R3 and R4, together with the nitrogen atom they are bound to, form a 1,4-diazepane ring, the 1,4-diazepane ring optionally being substituted by one or two substituents independently being -F, C1-C4 alkyl, C1-C4 alkyl substituted with 1 to 3 F atoms, C1-C4 acyl, benzoyl, 1-carboxamidyl optionally being substituted with C1-C4 alkyl or phenyl, 1-carboximidamidyl, sulfonyl optionally being substituted with C1-C4 alkyl or phenyl, 2-aminoethyl, 2-N-(C1-C4 alkyl)aminoethyl, 2-N,N-di(C1-C4 alkyl)aminoethyl, phenyl, pyridyl, pyrimidyl, benzyl, allyl, cyclopropyl, cyclobutyl, cyclopentyl, cyclohexyl, cyanomethyl, 2-cyanoethyl, 2-methansulfonylethyl, hydroxymethyl, 2-hydroxyethyl, 3-hydroxypropyl, (C1-C4 alkoxy)methyl, 2-(C1-C4 alkoxy)ethyl, 3-(C1-C4 alkoxy)propyl, N-methylpiperidyl, 1-phenylethyl, pyridylmethyl, N-methylpiperidylmethyl, 2-morpholinylethyl, morpholinocarbonylethyl, N,N-dimethylcarbonylmethyl, anilinocarbonylmethyl, N-methylanilinocarbonylmethyl, N-pyrrolidinocarbonylmethyl, N,N-dimethylsulfonyl, or oxo.

In another preferred embodiment, the invention relates to compounds of of formula (I), wherein R7 is -CH₂OH.

In another preferred embodiment, the invention relates to compounds of of formula (I), wherein R7 is CF₃.

In another preferred embodiment, the invention relates to compounds of of formula (I), wherein R7 is methyl.

CR1 represents a ring carbon atom to which substituent R1 is bound.

C1-C4 alkyl represents a straight-chain or branched alkyl group having 1 to 4 carbon atoms, including methyl, ethyl, propyl, isopropyl, butyl, isobutyl, sec-butyl, and tert-butyl.

C2-C3 alkyl presents a straight-chain or branched alkyl group having 2 or 3 carbon atoms, including ethyl, propyl, and isopropyl.

C1-C4 alkoxy represents groups which, in addition to the oxygen atom, contain a straight chain or branched C1-C4 alkyl group as outlined above. Accordingly, C1-C4 alkoxy includes methoxy, ethoxy, propoxy, isopropoxy, butoxy, isobutoxy, sec-butoxy, and tert-butoxy.

C1-C4 alkylamino represents groups in which the nitrogen atom is bound to a hydrogen atom and to straight chain or branched C1-C4 alkyl groups as outlined above. Accordingly, C1-C4 alkylamino includes methylamino, ethylamino, pro-pylamino, isopropylamino, butylamino, isobutylamino, sec-butylamino and tert-butylamino.

C1-C4 dialkylamino represents groups in which the nitrogen atom is bound to two identical or non-identical straight chain or branched C1-C4 alkyl groups which include methyl, ethyl, propyl, isopropyl, butyl, isobutyl, sec-butyl, or tert-butyl.

Di(C1-C4 alkyl)aminoethyl represents groups in which the nitrogen atom is bound to two straight chain or branched C1-C4 alkyl groups which include methyl, ethyl, propyl, isopropyl, butyl, isobutyl, sec-butyl, or tert-butyl.

C3-C6 cycloalkyl represents the cyclopropyl, cyclobutyl, cyclopentyl, and cyclohexyl groups.

Four- to six-membered heterocyclyl represents a saturated carbocycle group with four to six carbon ring atoms, one of which is replaced by an N atom and another of which optionally is replaced by an N, O or S atom. Examples for saturated four- to six-membered heterocyclyl comprise without limitation azetidinyl, 1,3-oxazolidinyl, 1,3-thiazolidinyl, isoxazolidinyl, isothiazolidinyl, 1,2,4-triazolidinyl, 1,2,4-oxadiazolidinyl, 1,2,4-thiadiazolidinyl, pyrrolidinyl, piperazinyl, piperidinyl, morpholinyl, 1,1-dioxothiomorpholinyl, hexahydropyrimidinyl, 1,3-oxazinanyl, 1,3-thiazinanyl, hexahydropyridazinyl, 1,2-oxazinanyl, 1,2-thiazinanyl, 1,2,4-triazinanyl, 1,2,5-oxadiazinanyl, and 1,2,5-thiadiazinanyl.

Four- to six-membered N-ethylheterocyclyl represents a four- to six-membered heterocyclyl which is N-substituted by an ethyl group.

Saturated four- to seven-membered heterocycle represents a saturated carbocycle with four to seven carbon ring atoms, one of which is replaced by an N atom and another of which optionally is replaced by an N, O or S atom. Examples for saturated four- to six-membered heterocycle comprise without limitation azetidine, 1,3-oxazolidine, 1,3-thiazolidine, isoxazolidine, isothiazolidine, 1,2,4-triazolidine, 1,2,4-oxadiazolidine, 1,2,4-thiadiazolidine, pyrrolidine, piperazine, piperidine, morpholine, 1,1-dioxothiomorpholine, hexahydropyrimidine, 1,3-oxazinane, 1,3-thiazinane, hexahydropyridazine, 1,2-oxazinane, 1,2-thiazinane, 1,2,4-triazinane, 1,2,5-oxadiazinane, 1,2,5-thiadiazinane, azepane, 1,4-diazepane, 1,4-oxazepane, and 1,4-thiazepane.

C1-C4 acyl represents groups comprising a carbonyl group which is part of a straight or branched C1-C4 chain. Examples for C1-C4 acyl are formyl, acetyl, propionyl, butyryl and isobutyryl.

Oxo means an oxygen atom which is bound by a double bond to a carbon atom, giving rise to a carbonyl group.

It is to be understood that the invention covers all combinations of substituent groups referred to hereinabove. In particular, the invention covers all combinations of preferred groups described herein.

Salts of the compounds according to the invention and the stereoisomers of the salts include all inorganic and organic acid addition salts and salts with bases, particularly all pharmaceutically acceptable inorganic and organic acid addition salts and salts with bases customarily used in pharmacy.

Examples of acid addition salts include, but are not limited to, hydrochlorides, hydrobromides, phosphates, nitrates, sulfates, acetates, trifluoroacetates, citrates, D-gluconates, benzoates, 2-(4-hydroxybenzoyl)benzoates, butyrates, sulfosalicylates, maleates, laurates, malates, lactates, fumarates, succinates, oxalates, tartrates, stearates, benzenesulfonates (besylates), toluenesulfonates (tosylates), methanesulfonates (mesylates), laurylsulfonates, 3-hydroxy-2-naphthoates, lactobionates, galactarates, embonates and ascorbates.

Examples of salts with bases include, but are not limited to, lithium, sodium, potassium, calcium, aluminum, magnesium, titanium, ammonium, meglumine and guanidinium salts.

The salts include water-insoluble (or practically insoluble) and, particularly, watersoluble salts.

The compounds according to the invention, the salts thereof and the stereoisomers of the compounds or the salts thereof may contain, e.g. when isolated in crystalline form, varying amounts of solvents. Included within the scope of the invention are, therefore, all solvates of the compounds of formula (I), the salts thereof, and the stereoisomers of the compounds and the salts thereof. Hydrates are a preferred example of said solvates.

The invention further relates to stereoisomers of formula (I). The invention also relates to mixtures of stereoisomers, including the racemates.

Some of the compounds, salts thereof, and stereoisomers of the compounds or the salts thereof may exist in different crystalline forms (polymorphs) as well as in amorphous forms, which are intended to be within the scope of the invention.

Furthermore, derivatives of the compounds of formula (I), the salts thereof, stereoisomers of the compounds or the salts thereof which are converted into compound (I), a salt thereof, or a stereoisomer of the compound or a salt thereof in a biological system (bioprecursors or prodrugs) are covered by the invention. Said biological system is e.g. a mammalian organism, particularly a human subject. The prodrug is, for example, converted into the compound of formula (I), a salt thereof, or a stereoisomer of the compound or a salt thereof by metabolic processes. For example, prodrugs may be esters of the compounds of formula (I) which can be readily hydrolyzed after uptake by a biological systems, e.g., by an esterase, to yield unmodified compound of formula (I).

The compounds according to the invention can be prepared as follows.

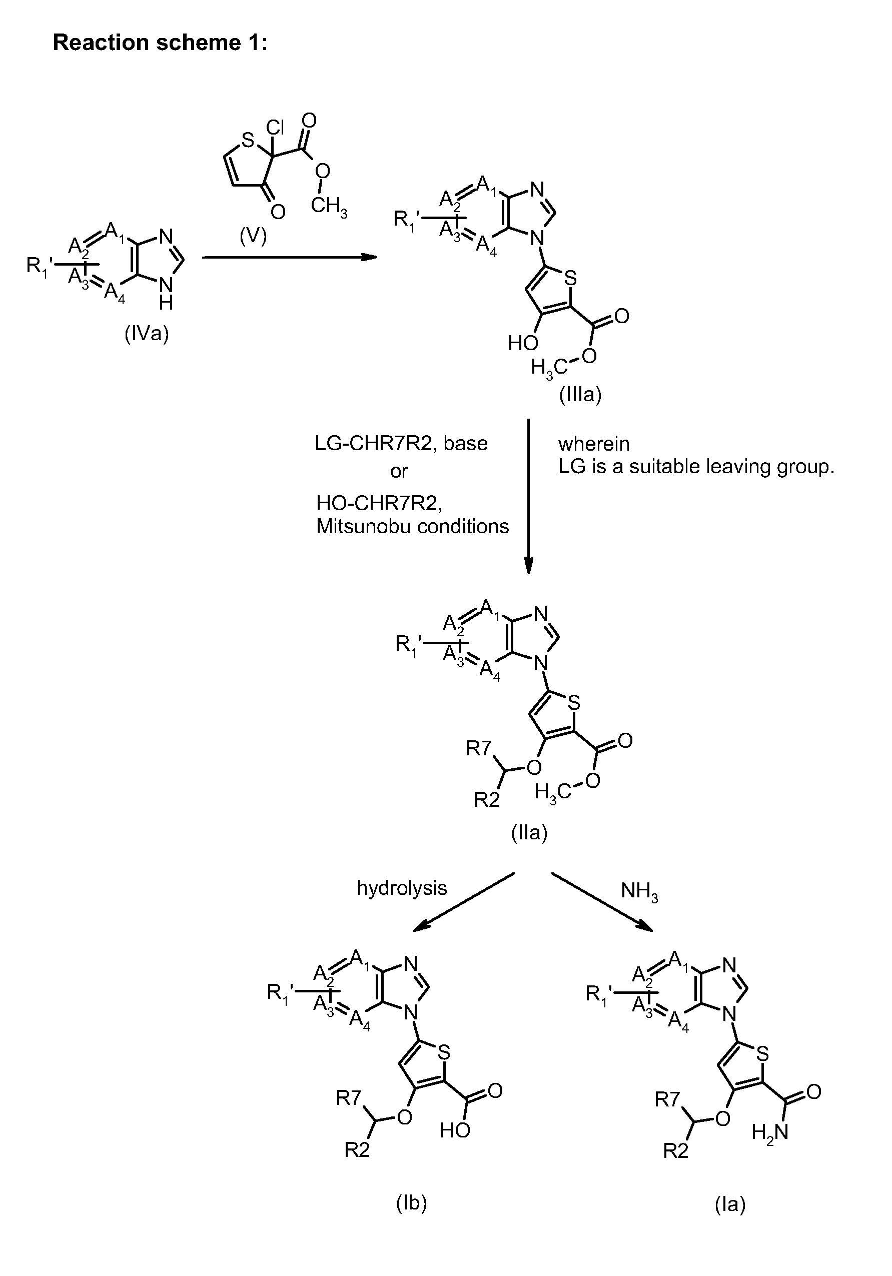

Compounds of formula (lVa), LG-CHR7R2 and HO-CHR7R2 (R2 and R7 having the same meaning as outlined above) are commercially available or can be obtained according to procedures known in the art or methods as described in reaction schemes 3-5. R1' includes, but is not limited to, -Cl, -OMe, -CH₂OPG, - C(O)N(R5)R6 (R5 and R6 having the same meaning as outlined above). Specific examples of a suitable protecting group (PG) include, but are not limited to, t-butyldimethylsilyl (TBDMS) and t-butyldiphenylsilyl (TBDPS). For the synthesis of compound (V), see Corral, C., Lissavetzky, J., Synthesis (1984), 847-850.

Specific examples of a suitable leaving group (LG) include, but are not limited to, bromide and chloride. An alternative leaving group might be -OS02CH3.

Due to tautomerism of the imidazole moiety, the reaction between non-symmetrical compounds (IVa) and (V) usually yields compounds (IIIa) as a mixture of regioisomers. In certain cases the formation of one of the two possible isomers is strongly preferred. The reaction is carried out in an appropriate solvent, e.g. CHCl₃, CH₂Cl₂ or toluene, preferably at room temperature. In some cases the addition of a suitable amine base (e.g. 2,2,6,6-tetramethylpiperidine or N-methylimidazole) may improve the yields. It is also possible to separate the mixture of regioisomers at the stage of compounds (IIIa), (IIa), (Ia) and (If), e.g., by flash column chromatography or by preparative HPLC. Preferentially the separation of the regioisomeric mixture is accomplished at the stage of compounds (IIa).

Typically, compounds of formula (IIIa) are used for the next reaction step towards compounds (IIa) as crude material without further purification. The synthesis of compounds (IIa) can either be carried out with a compound of formula LG-CHR7R2 in the presence of a base or with a compound of formula HO-CHR7R2 under standard Mitsunobu conditions. Under Mitsunobu conditions typically a compound of formula (IIIa), an alcohol of the formula HO-CHR7R2, a triarylphosphine, and a dialkyl azodicarboxylate are reacted in an inert solvent at room temperature. Examples of suitable triarylphosphines include but are not limited to triphenylphosphine, tri-p-toluylphosphine, and trimesitylphosphine. Examples of suitable dialkyl azodicarboxylates include, but are not limited to, diethyl azodicarboxylate, diisopropyl azodicarboxylate, and di-*tert*-butyl azodicarboxylate. Examples of suitable inert solvents for this reaction include, but are not limited to, tetrahydrofuran, dioxane, 1,2-dimethoxyethane, dichloromethane, and toluene. Further details can be found, e.g., in Hughes, D.L., Organic Reactions (1992), 42, 335-656; and in Mitsunobu, O., Synthesis (1981), 1-28.

In case of a chiral alcohol, under Mitsunobu conditions inversion of stereochemistry is observed.

Compounds of formula (Ia) can be obtained from (IIa). This reaction is typically performed at elevated temperatures in a sealed vessel (e.g., in the cavity of a microwave oven or in an autoclave) with an excess of ammonia. Typical temperatures, without being meant as a limitation, include the range from about 100 to 130 °C. Suitable solvents for this reaction include, but are not limited to, methanol and ethanol.

Compounds of formula (Ib) can be obtained from (IIa) by acidic or basic hydrolysis, basic hydrolysis being preferred.

The structural assignment of the regioisomers (attachment of the thiophene ring at N-1 or N-3 of the imidazole) can be established by two-dimensional ¹H NMR experiments (NOESY, COSY), which can be performed at the stage of compounds of the formula (la-f), (IIa) and (IIIa). Exemplarily, the analysis is shown for examples 16 and 17.

Optionally, the two dimensional 1 H NMR experiments are performed for both or for only one of the two regioisomers.

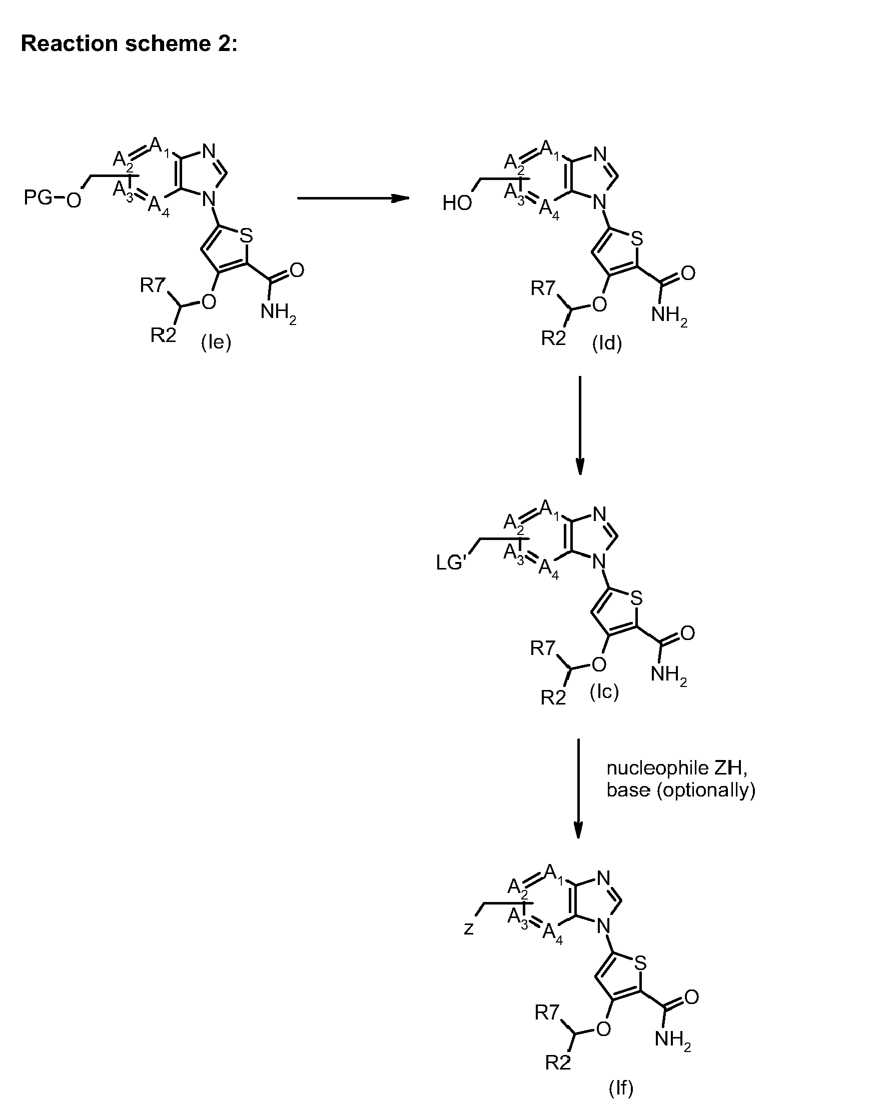

Compounds of formula (le) are a subtype of compounds of formula (la) and can be transformed to compounds of the formula (ld), (lc) and (lf) as outlined in scheme 2.

Specific examples of a suitable protecting group (PG) include, but are not limited to, t-butyldimethylsilyl (TBDMS) and t-butyldiphenylsilyl (TBDPS). Deprotection of a t-butyldimethylsilyl (TBDMS) or a t-butyldiphenylsilyl (TBDPS) group of compounds of formula (Ie) leading to compounds (Id) is carried out under standard conditions (e.g. tetra-*n*-butylammonium fluoride (TBAF) in THF). Details can be found in Kocienski, P.J., Protecting Groups, Georg Thieme Verlag, Stuttgart, 1994, p. 21-87, and in Greene, T.W., Wuts, P.G.M., Protecting Groups in Organic Synthesis (3rd Edition), J.Wiley & Sons, New York, 1999, p. 17-200.

Primary alcohols of formula (Id) can be converted to compounds (Ic) carrying a suitable leaving group (LG') by methods known in the art. A suitable leaving group, LG', includes, but is not limited to, -OSO₂CH₃. The introduction of this group can be accomplished, e.g., by reaction of compounds (Id) with (CH₃SO₂O)₂ or CH₃SO₂Cl in the presence of a base such as, e.g., triethylamine. Alternative leaving groups LG' might be chloride or bromide.

Reaction of compounds of formula (Ic) with certain nucleophiles (compounds of formula ZH), optionally in the presence of a base, yields compounds of formula (If).

Compounds of formula (ZH) include but are not limited to amines of the formula R3R4NH (R3 and R4 having the meaning as outlined above), N-substituted piperidin-4-ols, and magnesium or sodium methylsulfinate. Compounds of formula (ZH) are commercially available or can be obtained according to procedures known in the art.

Suitable bases include, but are not limited to, potassium carbonate, sodium hydride and triethylamine.

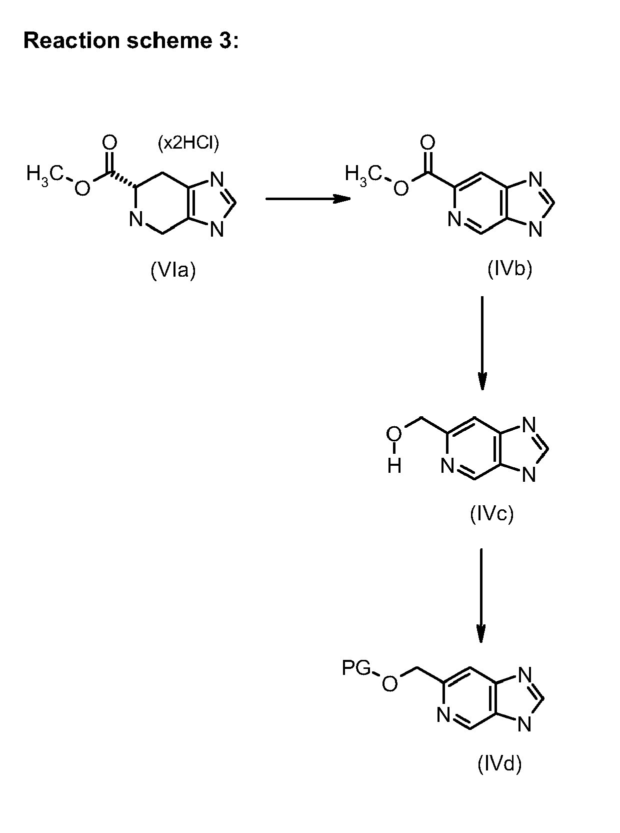

Compound (VIa) is prepared according to known procedures (see, e.g., WO 2004/039803 and WO 2005/103003).

Compound (IVb) can be prepared from (VIa) (optionally as dihydrochloride or as free base) using a suitable oxidizing agent (e.g. SeO₂ or MnO₂) in a suitable solvent (e.g. dioxane or toluene). Reduction of (IVb) with LiAlH₄ in a suitable solvent (e.g. THF) yields (IVc) which can be transformed to compound (IVd), carrying a suitable protecting group (PG). A suitable protecting group includes, but is not limited to, the t-butyldimethylsilyl (TBDMS) and t-butyldiphenylsilyl (TBDPS) group. The introduction of protecting groups can be carried out under conditions known in the art (see, e.g., Kocienski, P.J. Protecting Groups, Georg Thieme Verlag, Stuttgart, 1994, p. 21-87, and Greene, T.W., Wuts, P.G.M., Protecting Groups in Organic Synthesis (3rd Edition), J.Wiley & Sons, New York, 1999, p. 17-200).

Compounds of formula (IVd) are a subtype of compounds of formula (IVa) and are subjected to subsequent reactions as outlined in scheme 1.

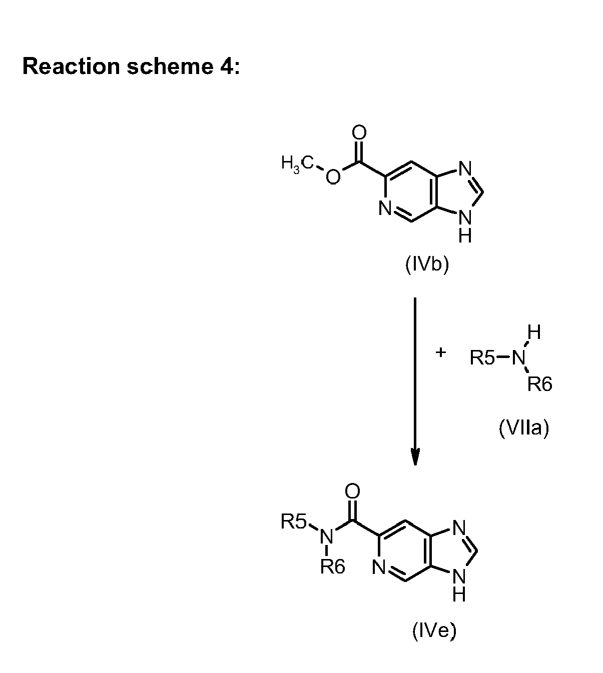

Compounds of formula (VIIa) are commercially available or can be obtained according to procedures known in the art.

Compounds of formula (IVe) are synthesized by reaction of compound (IVb) with an excess of the respective amines of the formula (VIIa) at elevated temperatures (e.g., about 140 °C) in a sealed vessel as described above. Suitable solvents for the reaction include, but are not limited to, methanol.

Compounds of formular (IVe) are a subtype of compounds of formula (IVa) and are subjected to subsequent reactions as outlined in scheme 1.

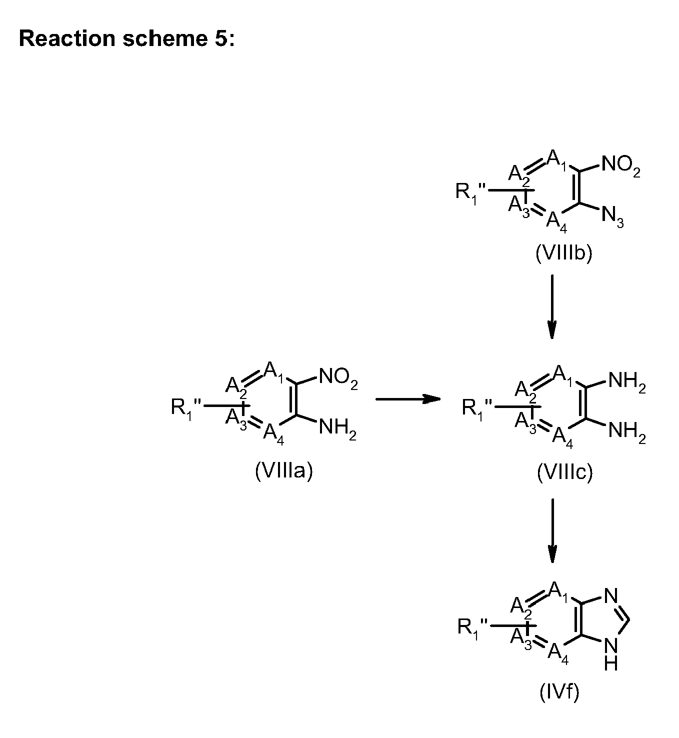

Compounds of formula (VIIIc) are commercially available or can be obtained according to procedures known in the art. Substituents R1" include, but are not limited to, -OCH₃ and -Cl.

The diamines (VIIIc) can be obtained, e.g., from compounds of formula (VIIIa) or (VIIIb) via reductive hydrogenation (e.g. with H₂/Pd), and may be used for the next step without purification. Compounds of formula (IVf) can be obtained by reaction of (VIIIc) with formic acid under reflux conditions. Compounds of formula (IVf) are a subtype of compounds of formula (IVa) and are subjected to subsequent reactions as outlined in scheme 1.

Compounds of formula (I) can be converted into different compounds of formula (I) by methods known in the art. For example,
- A compound of formula (I) wherein Y is -CONH₂ can be prepared from a compound of formula (I) wherein Y is -COOH by formation of the corresponding acid chloride (e.g., with SOCl₂) and subsequent reaction with NH₄OH.
- A compound of formula (I) wherein R1 is -CH₂OH can be converted into the corresponding aldehyde by oxidation, e.g., with the aid of a suitable oxidation reagent, and subsequently reacted with a suitable amine under reductive amination conditions (e.g., with NaBH(OAc)₃ as reducing agent), leading to the corresponding amine.
- A compound of formula (I) wherein R1 is -CH₂-piperazinyl can be converted into the corresponding N-alkylated, N-acylated or N-carbamoylated piperazine by reaction with an appropriate alkyl halogenide, carboxylic acid chloride, or isocyanate.

It is known to the person skilled in the art that, if there are a number of reactive centers on a starting or intermediate compound, it may be necessary to block one or more reactive centers temporarily by protective groups in order to allow a reaction to proceed specifically at the desired reaction center.

The compounds according to the invention are isolated and purified in a manner known *per se*, e.g. by distilling off the solvent in vacuo and recrystallizing the residue obtained from a suitable solvent, or by subjecting it to one of the customary purification methods, such as column chromatography employing a suitable support material.

Salts according to the invention of the compounds of formula (I) and of the stereoisomers thereof can be obtained by dissolving the free compound in a suitable solvent (for example a ketone such as acetone, methylethylketone or methylisobutylketone, an ether such as diethyl ether, tetrahydrofurane or dioxane, a chlorinated hydrocarbon such as methylene chloride or chloroform, a low molecular weight aliphatic alcohol such as methanol, ethanol or isopropanol, a low molecular weight aliphatic ester such as ethyl acetate or isopropyl acetate, an amide such as dimethylformamide, a nitril such as acetonitril, or water) which contains the desired acid or base, or to which the desired acid or base is then added. The acid or base can be employed in salt preparation, depending on whether a mono-or polybasic acid or base is concerned and depending on which salt is desired, in an equimolar quantitative ratio or a ratio differing therefrom. The salts can be obtained by filtering, reprecipitating, precipitating with a non-solvent for the salt or by evaporating the solvent. Salts obtained can be converted into the free compounds which, in turn, can be converted into salts. In this manner, pharmaceutically unacceptable salts, which can be obtained, for example, as process products in the manufacturing on an industrial scale, can be converted into pharmaceutically acceptable salts by processes known to the person skilled in the art.

In case R7 being methyl, ethyl, -CH₂OH or CF₃, the compounds according to the invention (including the salts thereof) are characterized by one stereogenic center at the carbon atom to which the substituents R7 and R2 are attached. This stereogenic center may have the absolute configuration R or the absolute configuration S (according to the rules of Cahn, Ingold and Prelog). In the formula below, the position of this asymmetrically substituted carbon atom is indicated by an asterisk:

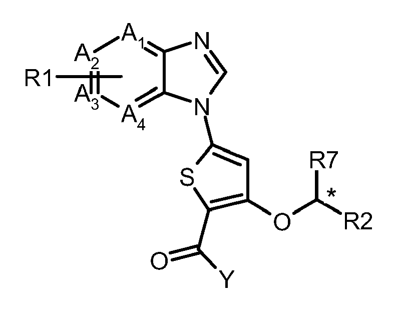

Accordingly, the invention further includes all mixtures of the stereoisomers mentioned above independent of the ratio of different stereoisomers to each other, including the racemates.

Accordingly, the invention further includes compounds of formula (I) wherein the carbon atom to which R2 and R7 are attached has the absolute configuration R.

Accordingly, the invention further includes compounds of formula (I) wherein the carbon atom to which R2 and R7 are attached has the absolute configuration S.

Pure diastereomers and pure enantiomers of the compounds of formula (I) and the salts thereof according to the invention can be obtained, e.g., by asymmetric synthesis, by using chiral starting compounds in synthesis and/or by splitting up enantiomeric and diastereomeric mixtures obtained in synthesis. Preferably, the pure diastereomeric and pure enantiomeric compounds of the invention are obtainable by asymmetric synthesis and/or by using chiral starting compounds in synthesis.

Enantiomeric and diastereomeric mixtures can be split up into the pure enantiomers and pure diastereomers by methods known to a person skilled in the art. Preferably, diastereomeric mixtures are separated by crystallization, in particular fractional crystallization, or chromatography. Enantiomeric mixtures can be separated, e.g., by forming diastereomers with a chiral auxiliary agent, resolving the diastereomers obtained and removing the chiral auxiliary agent. As chiral auxiliary agents, for example, chiral acids can be used to separate enantiomeric bases and chiral bases can be used to separate enantiomeric acids via formation of diastereomeric salts. Furthermore, diastereomeric derivatives such as diastereomeric esters can be formed from enantiomeric mixtures of alcohols or enantiomeric mixtures of acids, respectively, using chiral acids or chiral alcohols, respectively, as chiral auxiliary agents. Additionally, diastereomeric complexes or diastereomeric clathrates may be used for separating enantiomeric mixtures. Alternatively, enantiomeric mixtures can be split up using chiral separating columns in chromatography. Another suitable method for the isolation of enantiomers is the enzymatic separation.

As will be appreciated by persons skilled in the art, the invention is not limited to the particular embodiments described herein, but covers all modifications of said embodiments that are within the spirit and scope of the invention as defined by the appended claims.

All patents, patent applications, publications, test methods and other materials cited herein are incorporated by reference in their entireties.

The following examples illustrate the invention in greater detail, without restricting it. Further compounds according to the invention, of which the preparation is not explicitly described, can be prepared in an analogous way.

The compounds which are mentioned in the examples, the salts thereof, and stereoisomers of the compounds and the salts thereof represent preferred embodiments of the invention.

### Examples

¹H-NMR spectra were recorded using either Bruker DPX200 or Bruker AV400 or Bruker AVII300 or Bruker AV600 spectrometer in CDCl₃ or D₆-DMSO. Two dimensional ¹H NMR experiments (NOESY, COSY) were recorded using a Bruker DRX400. Chemical shifts are reported in parts per million (ppm) with tetramethylsilane (TMS) as an internal standard at zero ppm. Coupling constants (J) are given in hertz and the abbreviations s, d, t, q, m, and br refer to singlet, doublet, triplet, quartet, multiplet, and broad, respectively. The mass determinations were carried out by LCQ (Thermofinnigan). LC-MS determinations were carried out by Agilent 1100. HPLC runs were carried out by Varian ProStar using either phenomenex Gemini C18 or phenomenex Synergi RP-polar columns. Microwave reactions were carried out in an Emrys OptimizerEXP. Melting points were determinined using a Buechi Melting Point B-545. Flash chromatography was conducted with Macherey-Nagel silica gel 60M (230-400 mesh) or with Macherey-Nagel Alox N.

The following abbreviations are used: h: hour(s), °C: degrees centigrade, I: liter(s), ml: milliliter(s), DMSO: dimethyl sulfoxide, mp.: melting point, MS: mass spectrometry, ¹H NMR: ¹H nuclear magnetic resonance spectroscopy. ¹³C NMR: ¹³C nuclear magnetic resonance spectroscopy, HPLC: High Performance Liquid Chromatography, NOESY: Nuclear Overhauser Effect Spectroscopy, NOE: Nuclear Overhauser Effect, COSY: Correlated Spectroscopy.

### Final Compounds of type (Ia) and (Ib)

### 1. 5-(6-Chloro-1H-imidazo[4, 5-c]pyridin-1-yl)-3-{[2-(trifluoromethyl)benzyl]oxy}thiophene-2-carboxamide

A mixture of 140 mg of methyl 5-(6-chloro-1 H-imidazo[4,5-c]pyridin-1-yl)-3-{[2-(trifluoromethyl)benzyl]oxy}thiophene-2-carboxylate and 20 ml of a saturated solution of ammonia in methanol is stirred in a microwave vial at 120 °C for 4 h in the microwave cavity. The reaction mixture is concentrated to half of the volume. The solid that precipitated after standing is collected and recrystallized from acetonitrile to give the title compound.
¹H NMR (400 MHz, D₆-DMSO): δ = 5.57 (s, 2H), 6.82 (bs,1H), 7.75-7.69 (m, 1 H), 7.73 (s, 1 H), 7.76-7.87 (m, 4H), 7.90 (s, 1 H), 8.84 (s, 1 H), 8.93 (s, 1 H). MS (MH⁺ found) = 453.0
mp.: 248-249 °C (under decomposition)

### 2. 5-(1H-Imidazo[4,5-c]pyridin-1-yl)-3-{[2-(trifluoromethyl)benzyl]oxy}thiophene-2-carboxamide

In a similar manner as described for example 1, 195 mg of methyl 5-(1H-imidazo[4,5-c]pyridin-1-yl)-3-{[2-(trifluoromethyl)benzyl]oxy}thiophene-2-carboxylate (compound B1a) and 36 ml of a saturated solution of ammonia in methanol yield the title compound.
¹H NMR (200 MHz, D₆-DMSO): δ = 5.55 (s, 2H), 6.80 (bs, 1 H), 7.62-7.87 (m, 7H), 8.54 (d, J = 5.8 Hz, 1 H), 8.81 (s, 1 H), 9.10 (s, 1 H).
MS (MH⁺ found) = 419.1
mp.: 226-227 °C (under decomposition)

### 3. 5-(3H-Imidazo[4,5-c]pyridin-3-yl)-3-{[2-(trifluoromethyl)benzyl]oxy}thiophene-2-carboxamide

A mixture of 1.40 g of methyl 5-(3H-imidazo[4,5-c]pyridin-3-yl)-3-{[2-(trifluoromethyl)benzyl]oxy}thiophene-2-carboxylate (compound B1b) and 65 ml of a saturated solution of ammonia in methanol is stirred in an autoclave at 130 °C for 7 h. The mixture is allowed to cool down to room temperature and concentrated to about half of the volume. The precipitated solid is filtered, widely dissolved in 500 ml of methanol upon heating and filtered again. The filtrate is concentrated to about 25 ml upon which solid precipitated. It is filtered, dissolved in acetonitrile containing small amounts of water and finally the solution is lyophilized to give the title compound as a colorless powder.
The structural assignment of the regioisomer is unequivocally established by two-dimensional ¹H NMR experiments (NOESY, COSY).
¹H NMR (400 MHz, D₆-DMSO): δ = 5.57 (s, 2H), 6.81 (bs, 1 H), 7.64-7.68 (m, 1 H), 7.74 (bs, 1 H), 7.77-7.87 (m, 5H), 8.52 (d, J = 5.5 Hz, 1 H), 8.87 (s, 1 H), 9.18 (s, 1 H).
MS (MH⁺ found) = 419.1

### 4. 1-(5-Carbamoyl-4-{[2-(trifluoromethyl)benzyl]oxy}-2-thienyl)-N-(2-methoxyethyl)-1H-imidazo[4,5-c]pyridine-6-carboxamide

In a similar manner as described for example 1, 246 mg of methyl 5-{6-[(2-methoxyethyl)carbamoyl]-1H-imidazo[4,5-c]pyridin-1-yl}-3-{[2-(trifluoromethyl)benzyl]oxy}thiophene-2-carboxylate (compound B8) and 40 ml of a saturated solution of ammonia in methanol give the title compound.
The structural assignment of the regioisomer is unequivocally established by two-dimensional ¹H NMR experiments (NOESY, COSY).
¹H NMR (600 MHz, D₆-DMSO): δ = 3.30 (s, 3H), 3.52-3.54 (m, 4H), 5.56 (s, 2H), 6.83 (bs, 1 H), 7.65-7.68 (m, 1 H), 7.76 (s, 1 H), 7.80-7.90 (m, 4H), 8.39 (s, 1 H), 8.77-8.79 (m, 1 H), 8.95 (s, 1 H), 9.14 (s, 1 H).
MS (MH⁺ found) = 520.0
mp.: 227-228 °C (under decomposition)

### 5. 1-(5-Carbamoyl-4-{[2-(trifluoromethyl)benzyl]oxy}-2-thienyl)-N-(2-morpholin-4-ylethyl)-1H-imidazo[4,5-c]pyridine-6-carboxamide

A mixture of 150 mg of methyl 5-{6-[(2-morpholin-4-ylethyl)carbamoyl]-1H-imidazo[4,5-c]pyridin-1-yl}-3-{[2-(trifluoromethyl)benzyl]oxy}thiophene-2-carboxylate (compound B9) and 20 ml of a saturated solution of ammonia in methanol is stirred in a microwave vial at 125 °C for 4 h in the microwave cavity. The reaction mixture is concentrated to dryness, the resulting residue is dissolved in 5 ml of methanol and purified by preparative HPLC (water / acetonitrile, elution gradient 9/1 to 1/9 (v/v)). After lyophilization the title compound is obtained.
The structural assignment of the regioisomers is unequivocally established by two-dimensional ¹H NMR experiments (NOESY, COSY).
¹H NMR (400 MHz, D₆-DMSO): δ = 2.48 (m, 4H), 2.55-2.58 (m, 2H), 3.48-3.53 (m, 2H), 3.60 (t, J = 4.5 Hz, 4H), 5.55 (s, 2H), 6.83 (bs, 1 H), 7.64-7.68 (m, 1 H), 7.77-7.90 (m, 5H), 8.39 (s, 1 H), 8.83 (t, J = 5.8 Hz, 1 H), 8.95 (s, 1 H), 9.14 (s, 1 H).
MS (MH⁺ found) = 575.1

### 6. 5-{6-[(Diethylamino)methyl]-1H-imidazo[4,5-c]pyridin-1-yl}-3-{[2-(trifluoromethyl)benzyl]oxy}thiophene-2-carboxamide

To a solution of 58 mg of [1-(5-carbamoyl-4-{[2-(trifluoromethyl)benzyl]oxy}-2-thienyl)-1H-imidazo[4,5-c]pyridin-6-yl]methyl methanesulfonate (compound A1) in 4 ml of dichloromethane is added 146 mg of diethylamine, and the reaction mixture is stirred at room temperature for 12 h. The mixture is concentrated to dryness under vacuum and the residue is dissolved in acetonitrile/water and purified by preparative HPLC (water/acetonitrile, elution gradient 9/1 to 1/9 (v/v)). After lyophilization the title compound is obtained.
¹H NMR (400 MHz, D₆-DMSO): δ = 1.00 (t, J = 7.1 Hz, 6H), 2.55 (q, J = 7.1 Hz, 4H), 3.81 (s, 2H), 5.54 (s, 2H), 6.82 (bs, 1 H), 7.64-7.67 (m, 1 H), 7.72 (s, 1 H), 7.77-7.86 (m, 5H), 8.76 (s, 1 H), 8.97 (s, 1 H).
MS (MH⁺ found) = 504.0

### 7. 5-{6-[(Cyclopropylamino)methyl]-1H-imidazo[4,5-c]pyridin-1-yl}-3-{[2-(trifluoromethyl)benzyl]oxy}thiophene-2-carboxamide

In a similar manner as described for example 6, 58 mg of [1-(5-carbamoyl-4-{[2-(trifluoromethyl)benzyl]oxy}-2-thienyl)-1H-imidazo[4,5-c]pyridin-6-yl]methyl methanesulfonate (compound A1) and 228 mg of cyclopropylamine in 5 ml dichloromethane yield the title compound.
¹H NMR (400 MHz, D₆-DMSO): δ = 0.40-0.46 (m, 4H), 2.21-2.26 (m, 1H), 4.09 (s, 2H), 5.54 (s, 2H), 6.81 (bs, 1 H), 7.64-7.68 (m, 1 H), 7.72-7.87 (m, 7H), 8.77 (s, 1H), 9.03 (s, 1H).
MS (MH⁺ found) = 487.9

### 8. 5-{6-[(4-Methylpiperazin-1-yl)methyl]-1H-imidazo[4,5-c]pyridin-1-yl}-3-{[2-(trifluoromethyl)benzyl]oxy}thiophene-2-carboxamide

In a similar manner as described for example 6, 58 mg of [1-(5-carbamoyl-4-{[2-(trifluoromethyl)benzyl]oxy}-2-thienyl)-1H-imidazo[4,5-c]pyridin-6-yl]methyl methanesulfonate (compound A1) and 200 mg of 1-methylpiperazine in 5 ml dichloromethane yield the title compound.
¹H NMR (400 MHz, D₆-DMSO): δ = 2.14 (s, 3H), 2.33 (bs, 4H), 2.47 (bs, 4H), 3.73 (s, 2H), 5.54 (s, 2H), 6.81 (bs, 1 H), 7.64-7.58 (m, 1 H), 7.72-7.87 (m, 6H), 8.76 (s, 1H), 8.99 (s, 1 H).
MS (MH⁺ found) = 531.0

### 9. 5-[6-(Hydroxymethyl)-1H-imidazo[4,5-c]pyridin-1-yl]-3-{[2-(trifluoromethyl)benzyl]oxy}thiophene-2-carboxamide

A mixture of 3.15 g of methyl 5-[6-({[tert-butyl(dimethyl)silyl]oxy}methyl)-1H-imidazo[4,5-c]pyridin-1-yl]-3-{[2-(trifluoromethyl)benzyl]oxy}thiophene-2-carboxylate (compound B2b) and 218 ml of a saturated solution of ammonia in methanol is stirred in an autoclave at 120 °C for 3 h. The mixture is allowed to cool down to room temperature and concentrated to dryness to give the crude corresponding acid amide that is immediately used for the next step without further purification.
The residue is dissolved in 180 ml tetrahydrofuran and to this solution is added 2.0 ml of tetra-*n*-butylammonium fluoride solution (~75% in H₂O) at 0 °C. The reaction mixture is stirred at 0 °C for 12 h. The solvent is evaporated and the residue is treated with 250 ml dichloromethane and 60 ml saturated aqueous NaHCO₃ solution. The resulting precipitate is dissolved by addition of propan-2-ol, the organic layer is separated, washed with 60 ml saturated aqueous NaHCO₃ solution, dried with MgSO₄ and concentrated under vacuum. The residue is suspended in 40 ml dichloromethane and stirred for one hour. The suspension is filtered, the filter cake washed with 20 ml dichloromethane and dried under vacuum to yield the title compound.

The structural assignment of the regioisomers is unequivocally established by two-dimensional ¹H NMR experiments (NOESY, COSY).
¹H NMR (300 MHz, D₆-DMSO): δ = 4.72 (d, J = 5.5 Hz, 2H), 5.54-5.56 (m, 3H), 6.80 (bs, 1 H), 7.63-7.68 (m, 1 H), 7.74-7.88 (m, 6H), 8.78 (s, 1 H), 8.98 (d, J = 0.9 Hz, 1 H).
MS (MH⁺ found) = 449.0

### 10. 5-[6-(Hydroxymethyl)-3H-imidazo[4,5-c]pyridin-3-yl]-3-{[2-(trifluoromethyl)benzyl]oxy}thiophene-2-carboxamide

In a similar manner as described for example 9, 94 mg 5-[6-({[tert-butyl(dimethyl)silyl]oxy}methyl)-3H-imidazo[4,5-c]pyridin-3-yl]-3-{[2-(trifluoromethyl)benzyl]oxy}thiophene-2-carboxamide (compound A2) and 53 mg of tetra-*n*-butylammonium fluoride solution (~75% in H₂O) in 6 ml tetrahydrofuran yield the title compound.
The structural assignment of the regioisomers is unequivocally established by two-dimensional ¹H NMR experiments (NOESY, COSY).
¹H NMR (300 MHz, D₆-DMSO): δ = 4.71 (d, J = 5.8 Hz, 2H), 5.46 (t, J = 5.8Hz, 1 H), 5.57 (s, 2H), 6.80 (bs, 1 H), 7.63-7.87 (m, 7H), 8.84 (s, 1 H), 9.06 (d, J = 0.9Hz, 1 H).
MS (MH⁺ found) = 449.0

### 11. 1-(5-Carbamoyl-4-{[2-(trifluoromethyl)benzyl]oxy}-2-thienyl)-N-methyl-N-(2-morpholin-4-ylethyl)-1H-imidazo[4,5-c]pyridine-6-carboxamide

To a suspension of 25 mg sodium hydride (60% dispersion in mineral oil) in 2 ml anhydrous *N,N*-dimethylformamide is added 236 mg methyl 5-{6-[(2-morpholin-4-ylethyl)carbamoyl]-1H-imidazo[4,5-c]pyridin-1-yl}-3-{[2-(trifluoromethyl)benzyl]oxy}thiophene-2-carboxylate (compound B9) portionwise at 0 °C and the mixture is stirred for 30 minutes. 114 mg methyl iodide is added dropwise and after the addition the ice bath is removed and the reaction mixture is stirred at room temperature for 12 h. The solvent is evaporated and the residue is dissolved in 3 ml methanol and purified by preparative HPLC (water/acetonitrile, elution gradient 9/1 to 1/9 (v/v)) to give 5-[6-(methyl-(2-morpholin-4-yl-ethylcarbamoyl)-imidazo[4,5-c]pyridin-1-yl]-3-(2-trifluoromethyl benzyloxy)-thiophene-2-carboxylic acid methylester as crude material which is used for the next step without further purification.
In a similar manner as described for example 5, 29 mg of the above-synthesized crude 5-[6-(methyl-(2-morpholin-4-yl-ethylcarbamoyl)-imidazo[4,5-c]pyridin-1-yl)-3-(2-trifluoromethyl benzyloxy)-thiophene-2-carboxylic acid methylester and 20 ml of a saturated solution of ammonia in methanol yield the title compound.
The structural assignment of the regioisomer is unequivocally established by two-dimensional ¹H NMR experiments (NOESY, COSY).
¹H NMR (300 MHz, CDCl₃): δ = 2.37 (bs, 2H), 2.62-2.74 (m, 4H), 3.18 (s, 3H), 3.58 (bs, 2H), 3.67-3.74 (m, 4H), 5.47 (s, 2H), 5.73 (bs, 1 H), 6.93 (bs, 1 H), 7.03 (s, 1 H), 7.53-7.58 (m, 1 H), 7.64-7.70 (m, 2H), 7.78 (d, J = 7.7Hz, 1 H), 7.97-8.03 (m, 1 H), 8.18 (s, 1 H), 9.10 (d, J = 10.2 Hz, 1 H).
MS (MH⁺ found) = 589.0

### 12. 3-[1-(2-Chlorophenyl)ethoxy]-5-(3H-imidazo[4,5-c]pyridin-3-yl)thiophene-2-carboxamide

In a similar manner as described for example B3, 300 mg of an isomeric mixture of methyl 3-hydroxy-5-(1H-imidazo[4,5-c]pyridin-1-yl)thiophene-2-carboxylate and methyl 3-hydroxy-5-(3H-imidazo[4,5-c]pyridin-3-yl)thiophene-2-carboxylate (example C1) and 181 mg K₂CO₃ and 206 mg 1-chloro-2-(1-chloroethyl)benzene in 5 ml *N*,*N*-dimethylformamide yield methyl 3-[1-(2-chlorophenyl)ethoxy]-5-(1H-imidazo[4,5-c]pyridin-1-yl)thiophene-2-carboxylate and methyl 3-[1-(2-chlorophenyl)ethoxy]-5-(3H-imidazo[4,5-c]pyridin-3-yl)thiophene-2-carboxylate.
A mixture of 49 mg of the above-synthesized methyl 3-[1-(2-chlorophenyl)ethoxy]-5-(3H-imidazo[4,5-c]pyridin-3-yl)thiophene-2-carboxylate and 5 ml of a saturated solution of ammonia in methanol is stirred in a microwave vial at 125 °C for 4 h in the microwave cavity. The reaction mixture is allowed to cool down to room temperature, then another 5 ml of a saturated solution of ammonia in methanol is added and the procedure is repeated as described above. The solvent is removed under vacuum and the residue is purified by flash chromatography to yield the title compound.
The structural assignment of the regioisomer is unequivocally established by two-dimensional ¹H NMR experiments (NOESY, COSY).
¹H NMR (400 MHz, D₆-DMSO): δ = 1.75 (d, J = 6.3 Hz, 3H), 6.03 (q, J = 6.3 Hz, 1 H), 7.13 (bs, 1 H), 7.35 (s, 1 H), 7.37-7.46 (m, 2H), 7.50 (dd, J = 1.2 and 7.9 Hz, 1 H), 7.70 (dd, J = 1.5 and 7.7 Hz, 1 H), 7.80-7.83 (m, 2H), 8.49 (d, J = 5.5 Hz, 1 H), 8.79 (s, 1 H), 8.94 (s, 1 H).
MS (MH⁺ found) = 399.9

### 13. 5-(3H-Imidazo[4,5-c]pyridin-3-yl)-3-{1-[2-(trifluoromethyl)phenyl]ethoxy}thiophene-2-carboxamide

In a similar manner as described for example B3, 300 mg of an isomeric mixture of methyl 3-hydroxy-5-(1H-imidazo[4,5-c]pyridin-1-yl)thiophene-2-carboxylate and methyl 3-hydroxy-5-(3H-imidazo[4,5-c]pyridin-3-yl)thiophene-2-carboxylate (example C1) and 181 mg K₂CO₃ and 332 mg 1-(1-bromoethyl)-2-(trifluoromethyl)benzene in 5 ml *N*,*N*-dimethylformamide yield methyl 5-(1H-imidazo[4,5-c]pyridin-1-yl)-3-{1-[2-(trifluoromethyl)phenyl]ethoxy}thiophene-2-carboxylate and methyl 5-(3H-imidazo[4,5-c]pyridin-3-yl)-3-{1-[2-(trifluoromethyl)phenyl]ethoxy}thiophene-2-carboxylate.
A mixture of 92 mg of the above-synthesized methyl 5-(3H-imidazo[4,5-c]pyridin-3-yl)-3-{1-[2-(trifluoromethyl)phenyl]ethoxy}thiophene-2-carboxylate and 10 ml of a saturated solution of ammonia in methanol is stirred in a microwave vial at 125 °C for 4 h in the microwave cavity. The reaction mixture is allowed to come to room temperature, then another 10 ml of a saturated solution of ammonia in methanol is added and the procedure is repeated as described above. The solvent is removed under vacuum and the residue is purified by flash chromatography (*n*-hexane/ethyl acetate, elution gradient 10/0 to 3/7 (v/v)) to give the title compound.
The structural assignment of the regioisomer is unequivocally established by two-dimensional ¹H NMR experiments (NOESY, COSY).
¹H NMR (400 MHz, D₆-DMSO): δ = 1.76 (d, J = 6.2 Hz, 3H), 6.00 (q, J = 6.2 Hz, 1 H), 7.14 (bs, 1 H), 7.27 (s, 1 H), 7.57 (t, J = 7.7 Hz, 1 H), 7.76-7.85 (m, 4H), 7.96 (d, J = 7.7 Hz, 1 H), 8.49 (d, J = 5.5 Hz, 1 H), 8.76 (s, 1 H), 8.91 (s, 1 H).
MS (MH⁺ found) = 433.0

### 14. 5-(1H-Imidazo[4,5-c]pyridin-1-yl)-3-{1-[2-(trifluoromethyl)phenyl]ethoxy}thiophene-2-carboxamide

In a similar manner as described for example B3, 300 mg of an isomeric mixture of methyl 3-hydroxy-5-(1H-imidazo[4,5-c]pyridin-1-yl)thiophene-2-carboxylate and methyl 3-hydroxy-5-(3H-imidazo[4,5-c]pyridin-3-yl)thiophene-2-carboxylate (example C1) and 181 mg K₂CO₃ and 332 mg 1-(1-bromoethyl)-2-(trifluoromethyl)benzene in 5 ml *N*,*N*-dimethylformamide yield methyl 5-(1 H-imidazo[4,5-c]pyridin-1-yl)-3-{1-[2-(trifluoromethyl)phenyl]ethoxy}thiophene-2-carboxylate and methyl 5-(3H-imidazo[4,5-c]pyridin-3-yl)-3-{1-[2-(trifluoromethyl)phenyl]ethoxy}thiophene-2-carboxylate.
A mixture of 86 mg of the above-synthesized methyl 5-(1H-imidazo[4,5-c]pyridin-1-yl)-3-{1-[2-(trifluoromethyl)phenyl]ethoxy}thiophene-2-carboxylate and 10 ml of a saturated solution of ammonia in methanol is stirred in a microwave vial at 125 °C for 4 h in the microwave cavity. The reaction mixture is allowed to cool down to room temperature, then another 10 ml of a saturated solution of ammonia in methanol is added and the procedure is repeated as described above. The solvent is removed under vacuum and the residue is purified by flash chromatography to give the title compound.
The structural assignment of the regioisomer is unequivocally established by two-dimensional ¹H NMR experiments (NOESY, COSY).
¹H NMR (400 MHz, D₆-DMSO): δ = 1.77 (d, J = 6.2 Hz, 3H), 5.97 (q, J = 6.0 Hz, 1H), 7.14 (bs, 1H), 7.22 (s, 1H), 7.55-7.59 (m, 2H), 7.76-7.81 (m, 2H), 7.85 (bs, 1 H), 7.96 (d, J = 7.8 Hz, 1 H), 8.49 (d, J = 5.6 Hz, 1 H), 8.69 (s, 1 H), 9.07 (s, 1 H).
MS (MH⁺ found) = 432.9

### 15. 3-[1-(2-Chlorophenyl)ethoxy]-5-(1H-imidazo[4,5-c]pyridin-1-yl) thiophene-2-carboxamide

In a similar manner as described for example B3, 300 mg of an isomeric mixture of methyl 3-hydroxy-5-(1H-imidazo[4,5-c]pyridin-1-yl)thiophene-2-carboxylate and methyl 3-hydroxy-5-(3H-imidazo[4,5-c]pyridin-3-yl)thiophene-2-carboxylate (example C1) and 181 mg K₂CO₃ and 206 mg 1-chloro-2-(1-chloroethyl)benzene in 5 ml *N*,*N*-dimethylformamide yield methyl 3-[1-(2-chlorophenyl)ethoxy]-5-(1 H-imidazo[4,5-c]pyridin-1-yl)thiophene-2-carboxylate and methyl 3-[1-(2-chlorophenyl)ethoxy]-5-(3H-imidazo[4,5-c]pyridin-3-yl)thiophene-2-carboxylate.
A mixture of 87 mg of the above-synthesized methyl 3-[1-(2-chlorophenyl)ethoxy]-5-(1 H-imidazo[4,5-c]pyridin-1-yl)thiophene-2-carboxylate and 10 ml of a saturated solution of ammonia in methanol is stirred in a microwave vial at 125 °C for 4 h in the microwave cavity. The reaction mixture is allowed to cool down to room temperature, then another 10 ml of a saturated solution of ammonia in methanol is added and the procedure is repeated as described above. The solvent is removed under vacuum and the residue is purified by flash chromatography to yield the title compound.
The structural assignment of the regioisomer is unequivocally established by two-dimensional ¹H NMR experiments (NOESY, COSY).
¹H NMR (400 MHz, D₆-DMSO): δ = 1.75 (d, J = 6.4 Hz, 3H), 6.00 (q, J = 6.4 Hz, 1 H), 7.13 (bs, 1 H), 7.30 (s, 1 H), 7.30-7.45 (m, 2H), 7.51 (dd, J = 1.2 and 7.7 Hz, 1 H), 7.59-7.61 (m, 1 H), 7.70 (dd, J = 1.6 and 7.7 Hz, 1 H), 7.83 (bs, 1 H), 8.49 (d, J = 5.7 Hz, 1 H), 8.72 (s, 1 H), 9.07 (s, 1 H).
MS (MH⁺ found) = 398.9

### 16. 5-(6-Methoxy-1H-imidazo[4,5-c]pyridin-1-yl)-3-{[2-(trifluoromethyl)benzyl]oxy}thiophene-2-carboxamide

A suspension of 140 mg of an isomeric mixture of methyl 5-(6-methoxy-3H-imidazo[4,5-c]pyridin-3-yl)-3-{[2-(trifluoromethyl)benzyl]oxy}thiophene-2-carboxylate and methyl 5-(6-methoxy-1H-imidazo[4,5-c]pyridin-1-yl)-3-{[2-(trifluoromethyl)benzyl]oxy}thiophene-2-carboxylate (compounds B6a and B6b) in 20 ml of a saturated solution of ammonia in methanol is stirred in a microwave vial at 130 °C for 5 h in the microwave cavity. The reaction mixture is concentrated to dryness, the residue dissolved in 4 ml acetonitrile and 3 ml acidic buffer (KH₂PO₄, pH=2) and the isomers are separated and purified by preparative HPLC (acidic buffer/acetonitrile, 7/3 (v/v)). The acetonitrile is removed under vacuum and the aqueous solution is treated with NH₄OH until pH 8-9 is reached. The aqueous solution is extracted with dichloromethane three times and the combined organic layers are dried and concentrated to dryness under vacuum to yield the title compound.
The structural assignment of the regioisomer is unequivocally established by two-dimensional ¹H NMR experiments (NOESY, COSY).
NOE-crosspeaks are detected between H-8 and H-7, H-7 and H-4', H-4' and H-2: 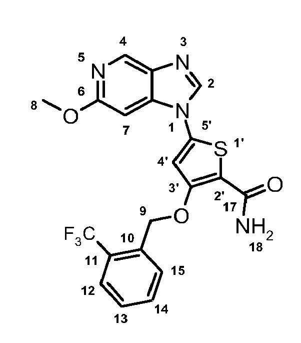 ¹H NMR (400 MHz, D₆-DMSO): δ = 3.94 (s, 3H, H-8), 5.56 (s, 2H, H-9), 6.79 (bs, 1 H, H-18), 7.11 (s, 1 H, H-7), 7.64-7.70 (m, 3H, including s, 1 H, H-4' at 7.68; bs, 1 H, H-18 and 1 H out of H-12, H-13, H-14, H-15), 7.77-7.86 (m, 3H, three H's out of H-12, H-13, H-14, H-15), 8.64 (s, 1 H, H-2), 8.69 (s, 1 H, H-4). MS (MH⁺ found) = 449.1

### 17. 5-(6-Methoxy-3H-imidazo[4,5-c]pyridin-3-yl)-3-{[2-(trifluoromethyl)benzyl]oxy}thiophene-2-carboxamide

The title compound is obtained as described in example 16.
The structural assignment of the regioisomer is unequivocally established by two-dimensional ¹H NMR experiments (NOESY, COSY).
NOE-crosspeaks are detected between H-4 and H-4', H-4' and H-2 as well as H-8 and H-7: 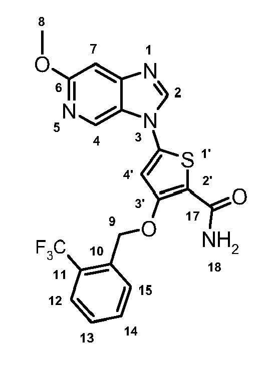 ¹H NMR (400 MHz, D₆-DMSO): δ = 3.92 (s, 3H, H-8), 5.56 (s, 2H, H-9), 6.77 (bs, 1 H, H-18), 7.15 (s, 1 H, H-7), 7.64-7.68 (m, 2H, H-18 and H-13 or H-14), 7.72 (s, 1 H, H-4'), 7.79 (t, J = 7.5 Hz, 1 H, H-13 or H-14), 7.84-7.86 (m, 2H, H-12 and H-15), 8.80 (s, 1 H, H-4), 8.81 (s, 1 H, H-2).
MS (MH⁺ found) = 449.0

### 18. 5-(6-Methoxy-1H-imidazo[4,5-c]pyridin-1-yl)-3-{[2-(trifluoromethyl)benzyl]oxy}thiophene-2-carboxylic acid

To a solution of 1.2 g of an isomeric mixture of methyl 5-(6-methoxy-3H-imidazo[4,5-c]pyridin-3-yl)-3-{[2-(trifluoromethyl)benzyl]oxy}thiophene-2-carboxylate and methyl 5-(6-methoxy-1H-imidazo[4,5-c]pyridin-1-yl)-3-{[2-(trifluoromethyl)benzyl]oxy}thiophene-2-carboxylate (compounds B6a and B6b) in 30 ml tetrahydrofuran is added dropwise 30 ml of a 1 N solution of LiOH. The reaction mixture is stirred for 12 h at room temperature and poured into a mixture of 200 ml diethylether and 0.1 N NaOH (1:1). The organic and aqueous layers are separated and the aqueous layer is acidified with HCl until pH 2 is reached. The aqueous layer is extracted with ethyl acetate (2 x 50 ml). The combined organic layers are dried with MgSO₄ and concentrated to dryness under vacuum. The residue is purified by preparative HPLC to yield the title compound. The structural assignment of the title compound is unequivocally established by two-dimensional ¹H NMR experiments (NOESY, COSY).
¹H NMR (400 MHz, D₆-DMSO): δ = 3.94 (s, 3H), 5.49 (s, 2H), 7.13 (s, 1 H), 7.61 (t, J = 7.7 Hz, 1 H), 7.66 (s, 1 H), 7.76-7.82 (m, 2H), 7.96 (d, J=7.7Hz, 1 H), 8.68 (s, 1 H), 8.69 (s, 1 H), 12.83 (bs, 1 H).
MS (MH⁺ found) = 450.1

### 19. 5-(3H-Imidazo[4,5-b]pyridin-3-yl)-3-{(2-(trifluoromethyl)benzyl]oxy}thiophene-2-carboxamide

In a similar manner as described for example 20, 4.41 g of methyl 5-(3H-imidazo[4,5-b]pyridin-3-yl)-3-{[2-(trifluoromethyl)benzyl]oxy}thiophene-2-carboxylate (compound B5b) and 400 ml of a saturated solution of ammonia in methanol yield the title compound.
¹H NMR (200 MHz, D₆-DMSO): δ = 5.52 (s, 2H), 6.74 (bs, 1 H), 7.46 (dd, J = 4.9 and 8.1 Hz, 1 H), 7.61-7.89 (m, 6H), 8.26 (dd, J = 1.4 and 8.1 Hz, 1 H), 8.53 (dd, J = 1.4 and 4.8 Hz, 1 H), 9.10 (s, 1 H).
MS (MH⁺ found) = 419.0

### 20. 5-(1H-Imidazo[4,5-b]pyridin-1-yl)-3-{[2-(trifluoromethyl)benzyl]oxy}thiophene-2-carboxamide

A mixture of 2.50 g of methyl 5-(1H-imidazo[4,5-b]pyridin-1-yl)-3-{[2-(trifluoromethyl)benzyl]oxy}thiophene-2-carboxylate (compound B5a) and 250 ml of a saturated solution of ammonia in methanol is stirred in an autoclave at 130 °C for 12 h. The reaction mixture is allowed to cool down to room temperature, concentrated, and the resulting residue is purified by flash chromatography (neutral alumina oxide, eluents: ethyl acetate / methanol). After crystallization from acetonitrile the title compound is obtained.
¹H NMR (400 MHz, D₆-DMSO): δ = 5.55 (s, 2H), 6.78 (bs, 1 H), 7.47 (dd, J = 4.7 and 8.2 Hz, 1 H), 7.66 (t, J = 7.6 Hz, 1 H), 7.72-7.74 (m, 2H), 7.79 (t, J = 7.6 Hz, 1 H), 7.84-7.86 (m, 2H), 8.27 (dd, J = 1.3 and 8.2 Hz, 1 H), 8.57 (dd, J = 1.3 and 4.7 Hz, 1 H), 8.94 (s, 1 H).
MS (MH⁺ found) = 419.1

### 21. 5-(5,6-Dimethoxy-1H-imidazo[4,5-b]pyridin-1-yl)-3-{[2-(trifluoromethyl)benzyl]oxy}thiophene-2-carboxamide (compound 21A) and 5-(5,6-Dimethoxy-3H-imidazo[4,5-b]pyridin-3-yl)-3-{[2-(trifluoromethyl)benzyl]oxy}thiophene-2-carboxamide (compound 21 B)

In a similar manner as described for example 16, 700 mg of an isomeric mixture of methyl 5-(5,6-dimethoxy-1H-imidazo[4,5-b]pyridin-1-yl)-3-{[2-(trifluoromethyl)benzyl]oxy}thiophene-2-carboxylate (compound B4a) and methyl 5-(5,6-dimethoxy-3H-imidazo[4,5-b]pyridin-3-yl)-3-{[2-(trifluoromethyl)benzyl]oxy}thiophene-2-carboxylate (compound B4b) and 80 ml of a saturated solution of ammonia in methanol give the title compounds 21A and 21 B.

### Compound 21A:

¹H NMR (400 MHz, D₆-DMSO): δ = 3.88 (s, 3H), 3.95 (s, 3H), 5.56 (s, 2H), 6.81 (bs, 1 H), 7.61 (s, 1 H), 7.63-7.67 (m, 2H), 7.72 (bs, 1 H), 7.78 (t, J = 7.6 Hz, 1 H), 7.84-7.86 (m, 2H), 8.55 (s, 1 H).
MS (MH⁺ found) = 479.0

### Compound 21B:

¹H NMR (400 MHz, D₆-DMSO): δ = 3.86 (s, 3H), 3.97 (s, 3H), 5.53 (s, 2H), 6.77 (bs, 1 H), 7.63-7.67 (m, 2H), 7.76-7.86 (m, 5H), 8.78 (s, 1 H)
MS (MH⁺ found) = 479.1

### 22. 5-(5-methoxy-3H-imidazo[4,5-b]pyridin-3-yl)-3-{[2-(trifluoromethyl)benzyl]oxy}thiophene-2-carboxamide

A mixture of 0.74 g of methyl 5-(5-methoxy-3H-imidazo[4,5-b]pyridin-3-yl)-3-{[2-(trifluoromethyl)benzyl]oxy}thiophene-2-carboxylate (compound B7b) and 200 ml of a saturated solution of ammonia in methanol is stirred in an autoclave at 130 °C for 10 h. The reaction mixture is allowed to cool down to room temperature and concentrated to about half of the volume. The precipitated solid is filtered, dissolved in 4 ml methanol / DMSO (1:1) and purified by preparative HPLC (water / acetonitrile, elution gradient 9/1 to 1/9 (v/v)). After lyophilization the obtained solid is crystallized with dichloromethane / *n-*hexane to give the title compound.
The structural assignment of the regioisomer is unequivocally established by two-dimensional ¹H NMR experiments (NOESY, COSY).
¹H NMR (400 MHz, D₆-DMSO): δ = 3.96 (s, 3H), 5.54 (s, 2H), 6.78 (bs, 1H), 6.85 (d, J = 8.8 Hz, 1 H), 7.62-7.67 (m, 2H), 7.79 (t, J = 7.5 Hz, 1 H), 7.85-7.87 (m, 3H), 8.13 (d, J = 8.5 Hz, 1 H), 8.88 (s, 1 H).
MS (MH⁺ found) = 448.9

### 23. 5-(5-methoxy-1H-imidazo[4,5-b]pyridin-1-yl)-3-{[2-(trifluoromethyl)benzyl]oxy}thiophene-2-carboxamide

In a similar manner as described for example 22, 1.50 g of methyl 5-(5-methoxy-1H-imidazo[4,5-b]pyridin-1-yl)-3-{[2-(trifluoromethyl)benzyl]oxy}thiophene-2-carboxylate (compound B7a) and 200 ml of a saturated solution of ammonia in methanol yield the title compound.
The structural assignment of the regioisomer is unequivocally established by two-dimensional ¹H NMR experiments (NOESY, COSY).
¹H NMR (400 MHz, D₆-DMSO): δ = 3.93 (s, 3H), 5.55 (s, 2H), 6.76 (bs, 1 H), 6.89 (d, J = 8.8 Hz, 1 H), 7.64-7.69 (m, 3H), 7.79 (t, J = 7.6 Hz, 1 H), 7.84-7.86 (m, 2H), 8.18 (d, J = 8.8 Hz, 1 H), 8.76 (s, 1 H).
MS (MH⁺ found) = 449.0

### Intermediate Compounds of type (le) and (Ic)

### A1. [1-(5-Carbamoyl-4-{[2-(trifluoromethyl)benzyl]oxy}-2-thienyl)-1H-imidazo[4,5-c]pyridin-6-yl]methyl methanesulfonate

To a suspension of 336 mg of 5-(6-hydroxymethyl-imidazo[4,5-c]pyridin-1-yl)-3-(2-trifluoromethyl-benzyloxy)-thiophene-2-carboxylic acid amide (compound 9) in 15 ml anhydrous dichloromethane is added 265 mg triethylamine and then 209 mg methanesulfonic anhydride at 0°C under nitrogen atmosphere. The reaction mixture is allowed to warm to room temperature and is stirred for one hour. 100 ml dichloromethane is added and the organic layer is washed twice with 30 ml of saturated NaHCO₃ solution. The organic layers are combined, dried with MgSO₄ and concentrated in vacuum. The residue is dissolved in ethyl acetate and filtered through a short plug of silica gel. After evaporation of the solvent the title compound is obtained.
¹H NMR (300 MHz, D₆-DMSO): δ = 3.29 (s, 3H), 5.46 (s, 2H), 5.54 (s, 2H), 6.81 (bs, 1 H), 7.63-7.68 (m, 1 H), 7.75-7.87 (m, 5H), 8.00 (d, J = 0.9 Hz, 1 H), 8.86 (s, 1 H), 9.12 (d, J = 0.9 Hz, 1 H).
MS (MH⁺ found) = 526.9

### A2. 5-[6-({[Tert-butyl(dimethyl)silyl]oxy}methyl)-3H-imidazo[4,5-c]pyridin-3-yl]-3-{[2-(trifluoromethyl)benzyl]oxy}thiophene-2-carboxamide

A mixture of 1.05 g of methyl 5-[6-({[tert-butyl(dimethyl)silyl]oxy}methyl)-3H-imidazo[4,5-c]pyridin-3-yl]-3-{[2-(trifluoromethyl)benzyl]oxy}thiophene-2-carboxylate (compound B2a) and 100 ml of a saturated solution of ammonia in methanol is stirred in an autoclave at 120 °C for 3.5 h. The reaction mixture is allowed to cool down to room temperature, concentrated to dryness, dissolved in dichloromethane and filtered through a plug of silica gel (eluents: dichloromethane / ethyl acetate) to yield the title product.
¹H NMR (300 MHz, D₆-DMSO): δ = 0.13 (s, 6H), 0.95 (s, 9H), 4.90 (s, 2H), 5.57 (s, 2H), 6.8 (bs, 1 H), 7.63-7.87 (m, 7H), 8.86 (s, 1 H), 9.08 (s, 1 H).
MS (MH⁺ found) = 562.9

### Intermediate Compounds of type (IIa)

### B1. Methyl 5-(1H-imidazo[4,5-c]pyridin-1-yl)-3-{[2-(trifluoromethyl)benzyl]oxy}thiophene-2-carboxylate (compound B1a) and Methyl 5-(3H-imidazo[4,5-c]pyridin-3-yl)-3-{[2-(trifluoromethyl)benzyl]oxy}thiophene-2-carboxylate (compound B1b)

In a similar manner as described for example B3, 7.92 g of an isomeric mixture of methyl 3-hydroxy-5-(1H-imidazo[4,5-c]pyridin-1-yl)thiophene-2-carboxylate and methyl 3-hydroxy-5-(3H-imidazo[4,5-c]pyridin-3-yl)thiophene-2-carboxylate (example C1) and 4.77 g of K₂CO₃ and 8.23 g of 1-(bromomethyl)-2-(trifluoromethyl)benzene in 60 ml *N,N-*dimethylformamide yield compound B1a and compound B1b.

### Compound B1a:

¹H NMR (200 MHz, D₆-DMSO): δ = 3.80 (s, 3H), 5.52 (s, 2H), 7.59-7.67 (m, 1H), 7.77-8.00 (m, 5H), 8.55 (d, J = 5.7 Hz, 1 H), 8.88 (s, 1 H), 9.11 (s, 1 H).

### Compound B1b:

¹H NMR (200 MHz, D₆-DMSO): δ = 3.80 (s, 3H), 5.54 (s, 2H), 7.59-7.66 (m, 1 H), 7.77-7.86 (m, 4H), 7.99 (d, J = 7.6 Hz, 1 H), 8.54 (d, J = 5.5 Hz, 1 H), 8.94 (s, 1 H), 9.23 (d, J = 0.7 Hz, 1 H).

### B2. Methyl 5-[6-({[tert-butyl(dimethyl)silyl]oxy}methyl)-3H-imidazo[4,5-c]pyridin-3-yl]-3-{[2-(trifluoromethyl)benzyl]oxy}thiophene-2-carboxylate (compound B2a) and Methyl 5-[6-({[tert-butyl(dimethyl)silyl]oxy}methyl)-1H-imidazo[4,5-c]pyridin-1-yl]-3-{[2-(trifluoromethyl)benzyl]oxy}thiophene-2-carboxylate (compound B2b)

In a similar manner as described for example B3, 2.08 g of an isomeric mixture of methyl 5-[6-({[tert-butyl(dimethyl)silyl]oxy}methyl)-3H-imidazo[4,5-c]pyridin-3-yl]-3-hydroxythiophene-2-carboxylate and methyl 5-[6-({[tert-butyl(dimethyl)silyl]oxy}methyl)-1H-imidazo[4,5-c]pyridin-1-yl]-3-hydroxythiophene-2-carboxylate (example C3) and 0.82 g of K₂CO₃ and 1.42 g of 1-(bromomethyl)-2-(trifluoromethyl)benzene in 15 ml *N,N-*dimethylformamide yield compound B2a and compound B2b.

### Compound B2a:

¹H NMR (300 MHz, D₆-DMSO): δ = 0.13 (s, 6H), 0.95 (s, 9H), 3.79 (s, 3H), 4.90 (s, 2H), 5.53 (s, 2H), 7.62 (t, J = 7.7 Hz, 1 H), 7.77-7.83 (m, 4H), 7.98 (d, J = 7.5 Hz, 1 H), 8.92 (s, 1 H), 9.12 (d, J = 0.9 Hz, 1 H).
MS (MH⁺ found) = 577.9

### Compound B2b:

¹H NMR (300 MHz, D₆-DMSO): δ = 0.13 (s, 6H), 0.96 (s, 9H), 3.79 (s, 3H), 4.91 (s, 2H), 5.48 (s, 2H), 7.63 (d, J = 7.7 Hz, 1 H), 7.78-7.83 (m, 3H), 7.90 (d, J = 0.7 Hz, 1 H), 7.98 (d, J = 7.7 Hz, 1 H), 8.91 (s, 1 H), 9.01 (d, J = 0.7 Hz, 1 H).
MS (MH⁺ found) = 578.0

### B3. Methyl 5-(6-chloro-1H-imidazo[4,5-c]pyridin-1-yl)-3-{[2-(trifluoromethyl)benzyl]oxy}thiophene-2-carboxylate (compound B3a) and Methyl 5-(6-chloro-3H-imidazo[4,5-c]pyridin-3-yl)-3-{[2-(trifluoromethyl)benzyl]oxy}thiophene-2-carboxylate (compound B3b)

To a solution of 557 mg of an isomeric mixture of methyl 5-(6-chloro-1H-imidazo[4,5-c]pyridin-1-yl)-3-hydroxythiophene-2-carboxylate and methyl 5-(6-chloro-3H-imidazo[4,5-c]pyridin-3-yl)-3-hydroxythiophene-2-carboxylate (example C2) in 25 ml anhydrous *N*,*N*-dimethylformamide is added 248 mg K₂CO₃ and 428 mg 1-(bromomethyl)-2-(trifluoromethyl)benzene under a nitrogen atmosphere. The reaction mixture is stirred for 12 h at room temperature, poured into 500 ml of ice water, then 40 ml of a saturated solution of KCI is added and the mixture is allowed to stand for 12 h at 0 °C. The resulting solid is filtered and washed with water. The filter cake is dissolved in dichloromethane, the organic layer is washed with water, dried with MgSO₄ and concentrated under vacuum. The isomeric mixture is separated and purified by flash chromatography [(Silicagel, hexane / ethylacatate (7/3 v/v)]. The separated isomers are crystallized from dichloromethane / hexane to yield compound B3a and compound B3b. The structural assignment of the regioisomers is unequivocally established by two-dimensional ¹H NMR experiments (NOESY, COSY).

### Compound B3a:

¹H NMR (400 MHz, D₆-DMSO): δ = 3.80 (s, 3H), 5.53 (s, 2H), 7.63 (t, J = 7.7 Hz, 1 H), 7.78-7.83 (m, 3H), 7.91-7.98 (m, 2H), 8.88 (s, 1 H), 8.92 (s, 1 H).

### Compound B3b:

¹H NMR (400 MHz, D₆-DMSO): δ = 3.80 (s, 3H), 5.53 (s, 2H), 7.63 (t, J = 7.6 Hz, 1 H), 7.78-7.82 (m, 3H), 7.98-7.99 (m, 2H), 9.01 (s, 1 H), 9.02 (s, 1 H).

### B4. Methyl 5-(5,6-dimethoxy-1H-imidazo[4,5-b]pyridin-1-yl)-3-{[2-(trifluoromethyl)benzyl]oxy}thiophene-2-carboxylate (compound B4a) and Methyl 5-(5,6-dimethoxy-3H-imidazo[4,5-b]pyridin-3-yl)-3-{[2-(trifluoromethyl)benzyl]oxy}thiophene-2-carboxylate (compound B4b)

To a solution of an isomeric mixture of 1.76 g of methyl 5-(5,6-dimethoxy-1H-imidazo[4,5-b]pyridin-1-yl)-3-hydroxythiophene-2-carboxylate and methyl 5-(5,6-dimethoxy-3H-imidazo[4,5-b]pyridin-3-yl)-3-hydroxythiophene-2-carboxylate (example C7) in 20 ml anhydrous *N*,*N*-dimethylformamide is added 0.86 g K₂CO₃ and 1.51 g of 1-(bromomethyl)-2-(trifluoromethyl)benzene under a nitrogen atmosphere. The reaction mixture is stirred for 12 h at room temperature, poured into 200 ml of ice water and stirred for 1 h. The solid is filtered, dissolved in acetonitrile and the isomeric mixture is separated and purified by preparative HPLC to yield compound B4a and compound B4b. The structural assignment of compound B4a is unequivocally established by two-dimensional ¹H NMR experiments (NOESY, COSY).

### Compound B4a:

¹H NMR (400 MHz, D₆-DMSO): δ = 3.79 (s, 3H), 3.90 (s, 3H), 3.96 (s, 3H), 5.53 (s, 2H), 7.60-7.65 (m, 2H), 7.70 (s, 1 H), 7.78-7.83 (m, 2H), 7.97 (d, J = 7.7 Hz, 1H), 8.80 (s, 1 H).
MS (MH⁺ found) = 494.1
compound B4b:
MS (MH⁺ found) = 494.1

### B5. Methyl 5-(1H-imidazo[4,5-b]pyridin-1-yl)-3-{[2-(trifluoromethyl)benzyl]oxy}thiophene-2-carboxylate (compound B5a) and Methyl 5-(3H-imidazo[4,5-b]pyridin-3-yl)-3-{[2-(trifluoromethyl)benzyl]oxy}thiophene-2-carboxylate (compound B5b)

In a similar manner as described for example B3, 12.49 g of an isomeric mixture of methyl 3-hydroxy-5-(1H-imidazo[4,5-b]pyridin-1-yl)thiophene-2-carboxylate and methyl 3-hydroxy-5-(3H-imidazo[4,5-b]pyridin-3-yl)thiophene-2-carboxylate (example C8) and 7.45 g of K₂CO₃ and 12.91 g of 1-(bromomethyl)-2-(trifluoromethyl)benzene in 200 ml *N,N-*dimethylformamide yield compound B5a and compound B5b.

### Compound B5a:

¹H NMR (400 MHz, D₆-DMSO): δ = 3.79 (s, 3H), 5.52 (s, 2H), 7.48 (dd, J = 4.7 and 8.2 Hz, 1 H), 7.63 (t, J = 7.7 Hz, 1 H), 7.80-7.84 (m, 3H), 7.98 (d, J = 7.7 Hz, 1 H), 8.33 (dd, J = 1.4 and 8.2 Hz, 1 H), 8.58 (dd, J = 1.4 and 4.7 Hz, 1 H), 9.25 (s, 1 H).
MS (MH⁺ found) = 434.1

### Compound B5b:

¹H NMR (400 MHz, D₆-DMSO): δ = 3.79 (s, 3H), 5.49 (s, 2H), 7.49 (dd, J = 4.8 and 8.1 Hz, 1 H), 7.63 (t, J = 7.7 Hz, 1 H), 7.79-7.84 (m, 2H), 7.94 (s, 1 H), 8.01 (d, J = 7.1 Hz, 1 H), 8.28 (dd, J = 0.8 and 8.0 Hz, 1 H), 8.56 (d, J = 4.8 Hz, 1H), 9.25 (s, 1 H).
MS (MH⁺ found) = 434.1

### B6. Methyl 5-(6-methoxy-3H-imidazo[4,5-c]pyridin-3-yl)-3-{[2-(trifluoromethyl)benzyl]oxy}thiophene-2-carboxylate (compound B6a) and Methyl 5-(6-methoxy-1H-imidazo[4,5-c]pyridin-1-yl)-3-{[2-(trifluoromethyl)benzyl]oxy}thiophene-2-carboxylate (compound B6b)

To a solution of 1.20 g of an isomeric mixture of methyl 3-hydroxy-5-(6-methoxy-3H-imidazo[4,5-c]pyridin-3-yl)thiophene-2-carboxylate and methyl 3-hydroxy-5-(6-methoxy-1H-imidazo[4,5-c]pyridin-1-yl)thiophene-2-carboxylate (example C6) in 15 ml anhydrous *N*,*N*-dimethylformamide is added 0.65 g of K₂CO₃ and 1.12 g of 1-(bromomethyl)-2-(trifluoromethyl)benzene under a nitrogen atmosphere. The reaction mixture is stirred for 12 h at room temperature and poured into 400 ml of ice water. The resulting solid is filtered, washed with water and dried under vacuum to give the crude title compounds as an isomeric mixture. The isomeric mixture is used for the next steps (examples 16, 17 and 18) without further purification.
MS (MH⁺ found) = 464.1

### B7. Methyl 5-(5-methoxy-1H-imidazo[4,5-b]pyridin-1-yl)-3-{[2-(trifluoromethyl)benzyl]oxytthiophene-2-carboxylate (Compound B7a) and Methyl 5-(5-methoxy-3H-imidazo[4,5-b]pyridin-3-yl)-3-{[2-(trifluoromethyl)benzyl]oxy}thiophene-2-carboxylate (Compound B7b)

In a similar manner as described for example B3, 1.4 g of an isomeric mixture of methyl 3-hydroxy-5-(5-methoxy-1H-imidazo[4,5-b]pyridin-1-yl)thiophene-2-carboxylate and methyl 3-hydroxy-5-(5-methoxy-3H-imidazo[4,5-b]pyridin-3-yl)thiophene-2-carboxylate (example C9) and 0.76 g K₂CO₃ and 1.31 g 1-(bromomethyl)-2-(trifluoromethyl)benzene in 20 ml *N*,*N-*dimethylformamide yield compound B7a and compound B7b as crude material. These compounds are used for the next step (examples 22 and 23) without further purification.
LC-MS (MH⁺ found) = 464.1 (compound B7a and B7b)

### B8. Methyl 5-{6-[(2-methoxyethyl)carbamoyl]-1H-imidazo[4,5-c]pyridin-1-yl}-3-{[2-(trifluoromethyl)benzyl]oxy}thiophene-2-carboxylate

To a solution of 1.5 g of methyl 3-hydroxy-5-{6-[(2-methoxyethyl)carbamoyl]-1H-imidazo[4,5-c]pyridin-1-yl}thiophene-2-carboxylate (compound C5) in 10 ml anhydrous *N*,*N*-dimethylformamide is added 610 mg K₂CO₃ and 1.14 g 1-(bromomethyl)-2-(trifluoromethyl)benzene under a nitrogen atmosphere. The reaction mixture is stirred for 12 h at room temperature, poured into 400 ml of ice water and the resulting precipitate is filtered. The filter cake is washed with water, dissolved in 300 ml ethyl acetate and washed with 50 ml saturated aqueous KCl solution. The organic layer is separated, dried with MgSO₄ and concentrated under vacuum. The residue is crystallized from methanol to yield the title compound.
¹H NMR (200 MHz, D₆-DMSO): δ = 3.31 (s, 3H), 3.52 (m, 4H), 3.81 (s, 3H), 5.51 (s, 2H), 7.59-7.67 (m, 1 H), 7.78-7.85 (m, 2H), 7.89 (s, 1 H), 8.0 (d, J = 7.9 Hz, 1 H), 8.42 (s, 1 H), 8.80 (bs, 1 H), 9.02 (s, 1 H), 9.15 (s, 1 H).
LC-MS (MH⁺ found) = 535.0

### B9. Methyl 5-{6-[(2-morpholin-4-ylethyl)carbamoyl]-1H-imidazo[4,5-c]pyridin-1-yl}-3-{[2-(trifluoromethyl)benzyl]oxy}thiophene-2-carboxylate

In a similar manner as described for example B8, 1.03 g of methyl 3-hydroxy-5-{6-[(2-morpholin-4-ylethyl)carbamoyl]-1H-imidazo[4,5-c]pyridin-1-yl}thiophene-2-carboxylate (compound C4), 326 mg K₂CO₃ and 683 mg 1-(bromomethyl)-2-(trifluoromethyl)benzene in 11 ml of N,N-dimethylformamide yield the title compound as crude material. The compound is used for the next step (examples 5 and 11) without further purification.
MS (MH⁺ found) = 590.1

### Intermediate Compounds of type (IIIa)

### C1. Methyl 3-hydroxy-5-(1H-imidazo[4,5-c]pyridin-1-yl)thiophene-2-carboxylate (Compound C1a) and Methyl 3-hydroxy-5-(3H-imidazo[4,5-c]pyridin-3-yl)thiophene-2-carboxylate (compound C1b)

A suspension of 17.27 g of 1*H*-imidazo[4,5-c]pyridine in 4 I chloroform is sonicated at 40 °C until the solid has been dissolved. To this solution a solution of 13.29 g methyl 2-chloro-3-oxo-2,3-dihydrothiophene-2-carboxylate in 120 ml chloroform is added dropwise and the mixture is stirred for 18 h at room temperature. The precipitated solid (1*H*-imidazo[4,5-c]pyridine hydrochloride) is filtered off. The filtrate is concentrated under reduced pressure to a volume of 400 ml and the precipitated solid (1*H*-imidazo[4,5-c]pyridine hydrochloride) is filtered off. The filtrate is washed with water (2x40 ml). The organic layer is separated and concentrated under reduced pressure to a volume of about 200 ml. This solution is triturated with 400 ml of n-hexane and stored at 0 °C. The precipitated solid (predominantly title compounds) is collected by filtration and washed with n-hexane. The filtrate is evaporated to dryness and the residue triturated with 600 ml ethyl acetate at 60 °C. The mixture is filtered and the organic layer concentrated under reduced pressure. The resulting solid (predominantly title compounds) is filtered and washed with ethyl acetate. The combined solids are dried under vacuum to yield the crude title compounds. The isomeric mixture is used for the next step (example B1) without further purification.
LC-MS (MH⁺ found) = 276.0 (isomeric mixture)

### C2. Methyl 5-(6-chloro-1H-imidazo[4,5-c]pyridin-1-yl)-3-hydroxythiophene-2-carboxylate (Compound C2a) and Methyl 5-(6-chloro-3H-imidazo[4,5-c]pyridin-3-yl)-3-hydroxythiophene-2-carboxylate (compound C2b)

To a mixture of 0.68 g of 6-chloro-1*H*-imidazo[4,5-c]pyridine and 0.65 g of 2,2,6,6-tetramethylpiperidine in 100 ml chloroform is slowly added a solution of 0.87 g of methyl 2-chloro-3-oxo-2,3-dihydrothiophene-2-carboxylate in 40 ml chloroform. The reaction mixture is stirred for 12 h at room temperature. After washing with water (3 x 50 ml) the organic layer is separated, dried with MgSO₄ and concentrated to a volume of about 100 ml. The residue is treated with n-hexane until precipitation takes place. The solid is filtered and the procedure is repeated once again. The combined solids are dried under vacuum to yield the title compounds as an isomeric mixture, which is used for the next step (example B3) without further purification.
LC-MS (MH⁺ found) = 310.0 (isomeric mixture)

### C3. Methyl 5-[6-({[tert-butyl(dimethyl)silyl]oxy}methyl)-3H-imidazo[4,5-c]pyridin-3-yl]-3-hydroxythiophene-2-carboxylate (compound C3a) and Methyl 5-[6-({[tert-butyl(dimethyl)silyl]oxy}methyl)-1H-imidazo[4,5-c]pyridin-1-yl]-3-hydroxythiophene-2-carboxylate (compound C3b)

To a mixture of 21.2 g of 6-({[tert-butyl(dimethyl)silyl]oxy}methyl)-1H-imidazo[4,5-c]pyridine and 11.36 g of 2,2,6,6-tetramethylpiperidine in 2 I dichloromethane is slowly added a solution of 8.77 g of methyl 2-chloro-3-oxo-2,3-dihydrothiophene-2-carboxylate in 300 ml chloroform at 30-40 °C. The reaction mixture is stirred for 12 h, washed with water (2 x 200 ml) and concentrated under vacuum. The residue is purified by flash chromatography (eluents: ethyl acetate / methanol / triethylamine) two times and after precipitation from ethyl acetate / n-hexane the title compounds are obtained as a mixture of regioisomers.
¹H NMR (300 MHz, D₆-DMSO) (isomeric mixture): δ = 0.13 (s, 6H), 0.14 (s, 6H), 0.95 (s, 9H), 0.96 (s, 9H), 3.79 (s, 3H), 3.80 (s, 3H), 4.89 (s, 2H), 4.91 (s, 2H), 7.16 (s, 1 H), 7.22 (s, 1 H), 7.76 (s, 1 H), 7.88 (s, 1 H), 8.84 (s, 1 H), 8.88 (s, 1 H), 9.00 (s, 1 H), 9.04 (s, 1 H).
LC-MS (MH⁺ found) = 420.0 (isomeric mixture)

### C4. Methyl 3-hydroxy-5-{6-[(2-morpholin-4-ylethyl)carbamoyl]-1H-imidazo[4,5-c]pyridin-1-yl}thiophene-2-carboxylate

To a solution of 2.27 g of 1*H*-imidazo[4,5-c]pyridine-6-carboxylic acid (2-morpholin-4-yl-ethyl)-amide in 150 ml chloroform is slowly added a solution of 1.27 g of methyl 2-chloro-3-oxo-2,3-dihydrothiophene-2-carboxylate in 100 ml chloroform and the reaction mixture is stirred for 12 h at room temperature. The reaction mixture is washed with water (3 x 50 ml) and the organic layer is dried with MgSO₄ and concentrated under vacuum. The residue is dissolved in dichloromethane and purified by flash chromatography (dichloromethane/methanol, elution gradient 10/0 to 9/1 (v/v))) to give the title compound.
MS (MH⁺ found) = 432.0

### C5. Methyl 3-hydroxy-5-{6-[(2-methoxyethyl)carbamoyl]-1H-imidazo[4,5-c]pyridin-1-yl}thiophene-2-carboxylate

In a similar manner as described for example C3, 1.81 g of 1 *H*-imidazo[4,5-c]pyridine-6-carboxylic acid (2-methoxy-ethyl)-amide and 1.27 g of methyl 2-chloro-3-oxo-2,3-dihydrothiophene-2-carboxylate in 300 ml chloroform yield the title compound which is used for the next step without further purification (example B8).
LC-MS (MH⁺ found) = 377.0

### C6. Methyl 3-hydroxy-5-(6-methoxy-1H-imidazo[4,5-c]pyridin-1-yl)thiophene-2-carboxylate (compound C6a) and Methyl 3-hydroxy-5-(6-methoxy-3H-imidazo[4,5-c]pyridin-3-yl)thiophene-2-carboxylate (compound C6b)

To a solution of 2.00 g of 6-methoxy-1*H*-imidazo[4,5-c]pyridine in 50 ml of chloroform is added 1.25 g of methyl 2-chloro-3-oxo-2,3-dihydrothiophene-2-carboxylate in portions and the reaction mixture is stirred for 120 h at room temperature. The reaction mixture is washed with water (3 x 50 ml) and the organic layer is dried and concentrated under vacuum. The residue is recrystallized from methanol to yield the title compounds as an isomeric mixture.
¹H NMR (400 MHz, CDCl₃) of isomeric mixture: δ = 3.95 (s, 6H), 4.02 (s, 6H), 6.84 (s, 1 H), 6.88 (s, 1 H), 6.98 (s, 1 H), 7.16 (s, 1 H), 8.01 (s, 1 H), 8.16 (s, 1 H), 8.68 (s, 1 H), 8.74 (s, 1 H), 9.79 (bs, 2H).

### C7. Methyl 5-(5,6-dimethoxy-1H-imidazo[4,5-b]pyridin-1-yl)-3-hydroxythiophene-2-carboxylate (compound C7a) and Methyl 5-(5,6-dimethoxy-3H-imidazo[4,5-b]pyridin-3-yl)-3-hydroxythiophene-2-carboxylate (compound C7b)

A suspension of 11.6 g of 2-azido-5,6-dimethoxy-3-nitropyridine (example E5) in 600 ml methanol is treated with 1.7 g Pd/C (10% Pd) and hydrogenated for 16 h under atmospheric pressure. The reaction mixture is rapidly filtered through a plug of celite, the filtrate is concentrated under vacuum and the residue is refluxed in 100 ml of formic acid for 18 h. The formic acid is removed under vacuum and the resulting residue is dried under vacuum at 100-110 °C to yield crude 5,6-dimethoxy-1H-imidazo[4,5-b]pyridine (as formic acid salt and / or as free base) that is used for the following step without further purification (MS (MH⁺ found) = 180.2).
In a similar manner as described for example C6, 1.50 g of the above-synthesized 5,6-dimethoxy-1*H*-imidazo[4,5-b]pyridine and 0.81 g of methyl 2-chloro-3-oxo-2,3-dihydrothiophene-2-carboxylate in 40 ml chloroform yield the title compounds as an isomeric mixture which is used for the next step without further purification (example B4).
¹H NMR (400 MHz, CDCl₃) of isomeric mixture: δ = 3.94 (s, 3H), 3.95 (s, 3H), 3.97 (s, 6H), 4.16 (s, 3H), 4.21 (s, 3H), 6.84 (s, 1 H), 7.16 (s, 1 H), 7.36 (s, 1 H), 7.53 (s, 1 H), 8.07 (s, 1 H), 8.16 (s, 1 H), 9.83 (bs, 2H).

### C8. Methyl 3-hydroxy-5-(1H-imidazo[4,5-b]pyridin-1-yl)thiophene-2-carboxylate (compound C8a) and Methyl 3-hydroxy-5-(3H-imidazo[4,5-b]pyridin-3-yl) thiophene-2-carboxylate (compound C8b)

In a similar manner as described for example C6, 30.0 g of 1*H*-imidazo[4,5-b]pyridine and 19.26 g of methyl 2-chloro-3-oxo-2,3-dihydrothiophene-2-carboxylate in 2 I chloroform give 3-hydroxy-5-imidazo[4,5-b]pyridin-3-yl-thiophene-2-carboxylic acid methyl ester (compound C8b) and the crude title compounds as an isomeric mixture which is used for the next step without further purification (example B5). The structural assignment of compound C8b is unequivocally established by two-dimensional ¹H NMR experiment (NOESY, COSY).

### Compound C8b:

¹H NMR (400 MHz, D₆-DMSO): δ = 3.81 (s, 3H), 7.40 (s, 1 H), 7.46 (dd, J = 4.8 and 8.0 Hz, 1 H), 8.25 (dd, J = 1.3 and 8.0 Hz, 1 H), 8.53 (dd, J = 1.3 and 4.8 Hz, 1 H), 9.11 (s, 1 H), 10.61 (bs, 1 H).
MS (MH⁺ found) = 276.0

### C9. Methyl 3-hydroxy-5-(5-methoxy-1H-imidazo[4,5-b]pyridin-1-yl)thiophene-2-carboxylate (compound C9a) and Methyl 3-hydroxy-5-(5-methoxy-3H-imidazo[4, 5-b]pyridin-3-yl)thiophene-2-carboxylate (compound C9b)

10.19 g of 6-methoxypyridine-2,3-diamine dihydrochloride is dissolved in 100 ml formic acid and stirred under reflux for 72 h. The formic acid is evaporated under vacuum and the residue is dried under vacuum at 95 °C to give crude 5-methoxy-1H-imidazo[4,5-b]pyridine (as hydrochloride and / or as formic acid salt and / or as free base) that is used for the following step without further purification.
In a similar manner as described for example C2, 8.54 g of the above-synthesized 5-methoxy-1*H*-imidazo[4,5-b]pyridine and 4.46 g of methyl 2-chloro-3-oxo-2,3-dihydrothiophene-2-carboxylate and 13.07 g of 2,2,6,6-tetramethylpiperidine in 620 ml chloroform yield the title compounds as an isomeric mixture which is used for the next step (example B7) without further purification.
LC-MS (MH⁺ found) = 306.0

### Intermediate Compounds of type (IVa)

### D1. 6-({[Tert-butyl(dimethyl)silyl]oxy}methyl)-1H-imidazo[4,5-c]pyridine

17.7 g of methyl 1H-imidazo[4,5-c]pyridine-6-carboxylate (example D2) is added in portions to a suspension of 19.0 g lithiumaluminium hydride in 0.6 I anhydrous tetrahydrofuran under nitrogen atmosphere at 0 °C. After the addition is completed the ice / water bath is removed and the mixture is stirred at room temperature for 12 h. 30 ml ethyl acetate followed by 100 ml methanol are slowly added at 0 °C (until gas evolution has stopped). Water (50 ml) is slowly added and the resulting precipitate is filtered. The precipitate is suspended in 1 I methanol and filtered through a plug of celite. The combined filtrates are evaporated under vacuum to give crude 1H-imidazo[4,5-c]pyridin-6-ylmethanol as a brown solid that is used for the next step without further purification. To a *N*,*N*-dimethylformamide solution (140 mL) of 1 H-imidazo[4,5-c]pyridin-6-ylmethanol is added 28.0 g *tert*-butyldimethylsilyl chloride and 19.4 g imidazole. The mixture is stirred at room temperature for 12 h and then poured into ice water (2.5 l). The resulting solid is filtered, washed with 100 ml water and dissolved in 0.5 l ethyl acetate. The organic layer is dried with MgSO₄ and then concentrated under vacuum to a volume of about 100 ml. 50 ml n-hexane is added and the precipitating solid is filtered and washed with 50 ml n-hexane. The filtrate is concentrated and the resulting precipitate is washed with n-hexane. Both batches of solid are combined and dried under vacuum to yield the title compound as a yellow solid.
¹H NMR (300 MHz, D₆-DMSO): δ = 0.11 (s, 9H), 0.94 (s, 6H), 4.84 (s, 2H), 7.56 (s, 1 H), 8.32 (s, 1 H), 8.83 (s, 1 H), 12.70 (br s, 1 H).
MS (MH⁺ found) = 264.1

### D2. Methyl 1H-imidazo[4,5-c]pyridine-6-carboxylate

68.5 g methyl (6S)-4,5,6,7-tetrahydro-1 H-imidazo[4,5-c]pyridine-6-carboxylate dihydrochloride, 81.8 g triethylamine and 63.3 g selenium dioxide are suspended in 1.1 I anhydrous 1,4-dioxane. The mixture is refluxed for 2 h. 26.3 g triethylamine is added and the mixture is allowed to cool down to room temperature and stirred overnight. The solvent is removed under reduced pressure and the residue is suspended in 300 ml methanol. 46.8 g triethylamine is added to adjust to pH 8-9. The resulting precipitate is filtered and washed with 70 ml methanol. The precipitate is treated with 500 ml *N,N-*dimethylformamide, stirred at 140 °C for 30 minutes and filtered hot. Upon cooling to room temperature, precipitation takes place. The precipitate is filtered, washed with 80 ml cold methanol and dried under vacuum to yield the title compound.
¹H NMR (200 MHz, D₆-DMSO): 3.89 (s, 3H), 8.31 (s, 1 H), 8.58 (s, 1 H), 9.02 (s, 1 H), 13.2 (br s, 1 H).
MS (MH⁺ found) = 178.0

### D3. 6-Chloro-1H-imidazo[4,5-c]pyridine

A suspension of 1.04 g of 2-chloro-5-nitropyridin-4-amine (example E6) in 100 ml ethanol is treated with 50 mg Pd/C (10% Pd) and hydrogenated for 12 h under atmospheric pressure. The reaction mixture is filtered through a plug of celite and the filtrate is concentrated under vacuum. The resulting oil is treated with 4 ml diethoxymethyl acetate and stirred for 2 h at room temperature and for one hour at 90 °C. The reaction mixture is allowed to cool down to room temperature, 20 ml dichloromethane is added and the organic layer is extracted with water (4 x 20 ml). The combined aqueous layers are concentrated to a volume of 10 ml and purified by preparative HPLC to yield the title compound.
¹H NMR (200 MHz, D₆-DMSO): δ = 7.69 (d, J = 0.8 Hz, 1 H), 8.46 (s, 1 H), 8.75 (d, J = 0.8 Hz, 1 H), 13.0 (bs, 1 H).
MS (MH⁺ found) = 154.1

### D4. 6-Methoxy-1H-imidazo[4,5-c]pyridine

A solution of 15.51 g of 2-methoxy-5-nitropyridin-4-amine (example E7) in 1.55 l methanol is treated with 4.65 g Pd/C (10% Pd) and hydrogenated for 12 h under atmospheric pressure. The reaction mixture is filtered through a plug of celite and the filtrate is concentrated under vacuum. The resulting residue is treated with 181 ml formic acid and the mixture is refluxed for 50 h. The formic acid is distilled off and the residue is repeatedly purified by flash chromatography (neutral alumina oxide, ethyl acetate / methanol) to yield the title compound.
¹H NMR (200 MHz, D₆-DMSO): δ = 3.87 (s, 3H), 6.85 (d, J = 0.9 Hz, 1H), 8.24 (s, 1 H), 8.54 (d, J = 0.9Hz, 1 H), 12.5 (bs, 1 H).
MS (MH⁺ found) = 150.1

### D5. N-(2-Morpholin-4-ylethyl)-1H-imidazo[4,5-c]pyridine-6-carboxamide

2.0 g of methyl-1H-imidazo[4,5-c]pyridine-6-carboxylate (example D2), 22.1 g of 2-morpholin-4-ylethanamine and 70 ml methanol are placed in an autoclave for 2 h at 140 °C. The methanol and 2-morpholin-4-ylethanamine is distilled off the reaction mixture under reduced pressure and the residue is purified by flash chromatography. After crystallization from ethyl acetate / n-hexane the title compound is obtained.
¹H NMR (200 MHz, D₆-DMSO): δ = 2.41-2.54 (m, 6H), 3.42-3.51 (m, 2H), 3.56-3.61 (m, 4H), 8.25 (d, J = 0.95 Hz, 1 H), 8.53 (s, 1 H), 8.70 (t, J = 5.8 Hz, 1H), 8.97 (d, J = 0.95 Hz, 1 H).
MS (MH⁺ found) = 276.1

### D6. N-(2-Methoxyethyl)-1H-imidazo[4,5-c]pyridine-6-carboxamide

In a similar manner as described for example D5, 2.0 g of methyl-1H-imidazo[4,5-c]pyridine-6-carboxylate (example D2) and 8.5 g of 2-methoxyethanamine in 50 ml methanol yield the title compound.
LC-MS (MH⁺ found) = 221.0

### Further Intermediates

### E1. 2, 6-Dibromopyridin-3-ol

To 1 I 10 % (w/v) aqueous NaOH solution are added 49 ml Br₂ at 0 °C. To this mixture an ice cold solution of 30.0 g pyridin-3-ol in 30 ml 10 % (w/v) aqueous NaOH solution is slowly added and the reaction mixture is stirred for 2 h at 0 °C and then for 2 h at room temperature. The resulting precipitate is filtered and the filtrate is acidified with aqueous HCl solution (pH 1). The resulting precipitate is filtered and dried under vacuum to yield the title compound.
¹H NMR (400 MHz, D₆-DMSO): δ = 7.25 (d, J = 8.0 Hz, 1 H), 7.47 (d, J = 8.0 Hz, 1 H), 11.10 (s, 1 H).

### E2. 2,6-Dibromo-3-methoxypyridine

To a solution of 52.5 g 2,6-dibromopyridin-3-ol (example E1) in 90 ml DMSO is added 26.2 g K₂CO₃ and 43.8 ml methyl iodide and the reaction mixture is stirred under reflux for one hour. The mixture is allowed to cool down to room temperature and poured into 1 I of water and stirred for 3 h at 80 °C. After cooling to room temperature the resulting solid is filtered, the filter cake is washed with ice cold water and dried under vacuum to yield the title compound.
¹H NMR (400 MHz, CDCl₃): δ = 3.91 (s, 3H), 7.04 (d, J = 8.0 Hz, 1 H), 7.38 (d, J = 8.0 Hz, 1 H).

### E3. 2,6-Dibromo-3-methoxy-5-nitropyridine

To 150 ml of concentrated sulfuric acid is added at 0 °C 150 ml fuming nitric acid. 40.0 g of 2,6-dibromo-3-methoxypyridine (example E2) is added portionwise to this mixture at 0 °C. The reaction mixture is stirred for 45 minutes at 0 °C and then heated to 65 °C for 2 h. The mixture is poured into 2 l of crushed ice and stored at 0 °C overnight. The resulting precipitate is filtered, washed with water and dried under vacuum to yield the title compound.
¹H NMR (400 MHz, CDCl₃): δ = 4.01 (s, 3H), 7.63 (s, 1 H).

### E4. 2-Bromo-5,6-dimethoxy-3-nitropyridine

20 g of 2,6-dibromo-3-methoxy-5-nitropyridine (example E3) are dissolved in 550 ml of anhydrous methanol at 30-40 °C. 4.6 g sodium methoxide dissolved in 30 ml anhydrous methanol is added to this solution. The reaction mixture is stirred for one hour at room temperature, poured into 700 ml of water and stored in the refrigerator overnight. The precipitate is filtered, washed with ice cold water and dried under vacuum to yield the title compound.
¹H NMR (400 MHz, CDCl₃): δ = 3.95 (s, 3H), 4.12 (s, 3H), 7.69 (s, 1 H). ¹³C NMR (100 MHz, CDCl₃): δ = 55.68, 56.73, 115.33, 121.89, 143.18, 155.10.

### E5. 2-Azido-5,6-dimethoxy-3-nitropyridine

To a suspension of 22.0 g of 2-bromo-5,6-dimethoxy-3-nitropyridine (example E4) in 50 ml DMSO are added 16.0 g sodium azide. The reaction mixture is stirred for 24 h at 50 °C. Additional 3.20 g sodium azide are added and after stirring for additional 44 h the reaction is completed. The solvent is removed under vacuum and the resulting residue is extracted with dichloromethane (3 x 100 ml). The solvent is removed and the residue is purified by flash chromatography (petroleum ether / ethyl acetate) to yield the title compound.
¹H NMR (400 MHz, CDCl₃): δ = 3.96 (s, 3H), 4.17 (s, 3H), 6.65 (s, 1 H).

### E6. 2-Chloro-5-nitropyridin-4-amine

357.5 g of 2-chloropyridin-4-amine is added in portions to 2.28 I of cold concentrated sulfuric acid. 0.86 I of nitric acid (90%) are slowly added so that the temperature of the reaction mixture always stays below 10 °C. After the addition the mixture is stirred for one hour at room temperature and poured into 4 l of water containing 10 kg of ice. 6.2 I 25% (w/v) aqueous NH₃ solution is slowly added. The resulting precipitate is filtered, washed with ice water and dried under vacuum to give 316.6 g of the 2-chloro-N-nitropyridin-4-amine. 316.2 g of this intermediate compound are treated with 2.35 I concentrated sulfuric acid and stirred for 30 minutes at 88-94 °C. The mixture is cooled to room temperature and poured into 2 I of water containing 5 kg ice. 6.5 125% (w/v) aqueous NH₃ solution is slowly added. The resulting precipitate is filtered and pre-dried under reduced pressure. Finally the residue is dried by azeotropic distillation with benzene. After crystallization from benzene the title compound is obtained.
¹H NMR (200 MHz, D₆-DMSO): δ = 6.96 (s, 1 H), 8.07 (bs, 2H), 8.84 (s, 1 H).

### E7. 2-Methoxy-5-nitropyridin-4-amine

To a suspension of 36.9 g of 2-chloro-5-nitropyridin-4-amine (example E6) in 500 ml methanol is added 250 ml of a freshly prepared solution of sodium methoxide in methanol (5.7 g sodium) and the reaction mixture is refluxed for 12 h. About 500 ml methanol are distilled off and 500 ml of water are added. The resulting precipitate is filtered, washed with ice water and dried under vacuum to the title compound.
¹H NMR (400 MHz, D₆-DMSO): δ = 8.82 (s, 1 H), 7.65 (bs, 2H), 6.15 (s, 1 H), 3.85 (s, 3H).

### Commercial utility

The compounds, salts thereof, and the stereoisomers of the compounds and the salts thereof according to the invention are hereinafter referred to as the compounds of the invention. In particular, the compounds of the invention are pharmaceutically acceptable.

The compounds of the invention have valuable pharmaceutical properties which make them commercially utilizable. Thus, one aspect of the invention relates to compounds according to formula I for use in the treatment or prophylaxis of diseases. In particular, as Plk1 inhibitors, they are able to interfere with the cell cycle of various cells, particularly neoplastic cells. In particular, the Plk1 inhibiting compounds of the invention can disrupt mitosis and drive cancer cells into apoptosis. The compounds of the invention are distinguished by valuable and desirable properties, such as, for example, high selectivity and low toxicity. On cellular level the compounds exhibit a cytotoxic effect only or preferentially on proliferating cells. The compounds are able to induce mitotic arrest in proliferating cells. Other valuable properties of the compounds of the invention include superior bioavailability in general (e.g. good enteral absorption), superior therapeutic window, superior pharmacokinetics (e.g. half-life), improved tolerability as compared to other anti-neoplastic agents, and further beneficial effects related with their therapeutic and pharmaceutical suitability.

Accordingly, the invention further relates to the compounds of the invention for use in the treatment or prophylaxis of diseases. Furtheron, the invention relates to the compounds of the invention for use in the treatment or prophylaxis of diseases alleviated by inhibition of kinases involved in cell division, preferably Polo-like kinases, even more preferably Plk1. In particular, the invention relates to the compounds of the invention for use in the treatment or prophylaxis of diseases characterized by increased Plk1 activity. Increased activity means that the activity of Plk1 in a given cell, group of cells, tissue, or region within a tissue is higher by a certain factor than it is in the healthy state. The factor by which Plk1 activity is increased can be, e.g., at least 1.5, at least 3, at least 10, or even at least 100. Without being meant as a limitation, the reason for said increased Plk1 activity may be increased expression ("overexpression") of the Plk1 gene or genes, which can be due to, among others, altered regulation of expression, mutations in the PIk1 promotor, gene duplication, gene amplification, genomic rearrangements, and so forth. Other processes leading to increased PIk1 activity might comprise reduced inhibition of PIk1 enzyme, enhanced PIk1 stability, reduced PIk1 inactivation or degradation, and altered availability of substrate(s) and/or cofactors.

Preferably, the invention further relates to the compounds of the invention for use in the treatment or prophylaxis of diseases comprising abnormal cell growth. In one embodiment, said abnormal cell growth is a cancer. In particular, the disease may be one of the following: Cancers of the colon including colorectal carcinoma, ovaries, breast, prostate, bladder, lung, gastrointestinal carcinomas, gastrointestinal stromal tumors, small cell lung cancer, head and neck cancer, cancers of cervix, pancreas, esophagus, kidney, larynx and hypopharynx, liver, endocrine glands, soft tissue, testis, retinoblastoma and Wilms tumor, endometrial tumors, malignant melanoma, non-Hodgekins lymphoma, Hodgekins lymphoma or other lymphomas, chronic and acute myeloic leukemia, acute lymphoblastic leukemia, T-cell lymphoma, plasma cell neoplasia, and leukemias, hematological malignancies including multiple myeloma, soft tissue sarcoma, osteosarcoma, fibrosarcoma, and other tumors of mesothelial origin, glioma, astrocytoma, cancers of unknown primary site, glioblastoma and other brain tumors and tumors of neuronal origin, germ cell cancers, myelodysplastic syndromes, myeloproliferative syndromes, disorders such as polycythemia vera, essential thrombocytopenia, myelofibrosis, hypereosinophilic syndrome, or other paraneoplastic syndromes. In another embodiment, said abnormal cell growth is a benign proliferative disease such as, e.g., benign prostatic hyperplasia, restenosis, fibrosis, or psoriasis, or any other kind of unwanted cell or tissue proliferation.

The invention also relates to the use of a compound of the invention in the manufacture of a pharmaceutical composition inhibiting Plk1, in particular a pharmaceutical composition for the treatment or prophylaxis of diseases alleviated by inhibition of Plk1, preferably, a pharmaceutical composition for the treatment or prophylaxis of the diseases exemplified above.

The invention further relates to a method of treating or preventing a disease comprising administering to a patient in need thereof a therapeutically effective amount of at least one of the compounds of the invention.

In particular, the invention relates to a method of treating or preventing one of the above mentioned diseases comprising administering to a patient in need thereof a therapeutically effective amount of at least one of the compounds of the invention.

Especially, the invention relates to a method of treating or preventing a disease which is alleviated by inhibition of Plk1 comprising administering to a patient in need thereof a therapeutically effective amount of at least one of the compounds of the invention.

Furthermore, the invention preferably relates to a method of treating or preventing cancers of the colon including colorectal carcinoma, ovaries, breast, prostate, bladder, lung, gastrointestinal carcinomas, gastrointestinal stromal tumors, small cell lung cancer, head and neck cancer, cancers of cervix, pancreas, esophagus, kidney, larynx and hypopharynx, liver, endocrine glands, soft tissue, testis, retinoblastoma and Wilms tumor, endometrial tumors, malignant melanoma, non-Hodgekins lymphoma, Hodgekins lymphoma or other lymphomas, chronic and acute myeloic leukemia, acute lymphoblastic leukemia, T-cell lymphoma, plasma cell neoplasia, and leukemias, hematological malignancies including multiple myeloma, soft tissue sarcoma, osteosarcoma, fibrosarcoma, and other tumors of mesothelial origin, glioma, astrocytoma, cancers of unknown primary site, glioblastoma and other brain tumors and tumors of neuronal origin, germ cell cancers, myelodysplastic syndromes, myeloproliferative syndromes, disorders such as polycythemia vera, essential thrombocytopenia, myelofibrosis, hypereosinophilic syndrome, or other paraneoplastic syndromes, or benign proliferative disease such as, e.g., benign prostatic hyperplasia, restenosis, fibrosis, or psoriasis, or any other kind of unwanted cell or tissue proliferation, comprising administering to a patient in need thereof a therapeutically effective amount of at least one of the compounds of the invention.

In the above methods, the patient is preferably a mammal, more preferably a human. Furthermore, in the above methods, at least one of the compounds of the invention can be used. Preferably, one or two of the compounds of the invention are used, more preferably, one of the compounds of the invention is used.

In a particularly preferred embodiment of the invention, the above methods of treating or preventing one of the above mentioned diseases comprise administering to a patient in need thereof a therapeutically effective amount of one compound of the examples according to the present invention.

The invention furthermore relates to a pharmaceutical composition which comprises at least one of the compounds of the invention together with at least one pharmaceutically acceptable carrier, diluent or excipient.

Preferably, the pharmaceutical composition comprises one or two of the compounds of the invention. More preferably, the pharmaceutical composition comprises one of the compounds of the invention.

In a particularly preferred embodiment of the invention, the pharmaceutical composition comprises a compound of the examples according to the present invention together with at least one pharmaceutically acceptable carrier, diluent or excipient.

The invention additionally relates to a pharmaceutical composition comprising at least one of the compounds of the invention, at least one pharmaceutically acceptable carrier, diluent or excipient, and at least one additional therapeutic agent with the proviso that the compound or compounds of the invention and the at least one addtional therapeutic agent are therapeutically compatible. By therapeutically compatible it is meant that (a) the toxicity of the compound or compounds of the invention and the toxicity of the at least one addtional therapeutic agent do not add up to an unacceptable toxicity, and (b) that the therapeutic effect of the compound or compounds of the invention is not modified or reduced in an unacceptable or undesired way by the at least one additional therapeutic agent, and vice versa. In a preferred embodiment, the therapeutic effect of the compound or compounds of the invention and the therapeutic effect of the at least one addtional therapeutic agent are additive. In another preferred embodiment, the therapeutic effect of the compound or compounds of the invention and the therapeutic effect of the at least one addtional therapeutic agent are synergistic, i.e., their combined effect is different from, preferably more beneficial than, the pure sum of the therapeutic effects when the compound or compounds and the agent(s) are administered alone, without any interaction of the effects the compound or compounds and the effects the agent(s) exert to the treated patient.

The invention also relates to a kit of parts, comprising at least one pharmaceutical composition comprising at least one of the compounds of the invention, and at least one pharmaceutical composition comprising at least one additional therapeutic agent.

Typical additional therapeutic agents useful in the present invention include, but are not limited to, anti-neoplastic agents in general, cytostatic agents, cytotoxic agents, antimetabolites, microtubule interfering agents, platinum derivatives, alkylating agents, topoisomerase I inhibitors, topoisomerase II inhibitors, signal transduction inhibitors, cell cycle signalling inhibitors, angiogenesis inhibitors including antibodies and receptor tyrosine kinase inhibitors, immunotherapeutic agents, monoclonal antibodies (including, but not limited to, murine, chimeric, and humanized monoclonal antibodies), proapoptotic agents, hormones, hormone analogues, antibiotic agents, anti-emetic agents, and analgetic agents.

In this respect, the therapeutic agent includes anti-neoplastic agents, cytostatic agents, cytotoxic agents, antimetabolites, microtubule interfering agents, platinum complexes, DNA alkylating agents, DNA intercalating agents, topoisomerase I inhibitors, topoisomerase II inhibitors, signal transduction inhibitors including kinase inhibitors, cell cycle signalling inhibitors, angiogenesis inhibitors including antibodies and receptor tyrosine kinase inhibitors, proapoptotic agents, hormones, hormone analogues, antibiotic agents, immunotherapeutic agents, monoclonal antibodies (including, but not limited to, murine, chimeric, and humanized monoclonal antibodies), anti-emetic agents, or analgetic agents in form of the free compounds, the pharmaceutically acceptable salts thereof, the pharmaceutically acceptable derivatives thereof (e.g., but not limited to, ester derivatives), the solvates thereof and the stereoisomers of the compounds, salts, derivatives and solvates.

Antimetabolites interfere with DNA synthesis. Purine analogues and pyrimidine analogues are incorporated, during the S phase of the cell cycle, into growing DNA strands and thereby suppress incorporation of the proper DNA building blocks, purines and pyrimidines. Examples for purine analogues and pyrimidine analogues include, but are not limited to, 5-fluorouracil, floxuridine, cytosine arabinose, mercaptopurine, thioguanine, fludarapine, pentostatin, and cladribine. Another class of antimetabolites prevents the synthesis of tetrahydrofolate, which is essential for purine and pyrimidine biosynthesis. Examples for antimetabolites suppressing synthesis of tetrahydrofolate include, but are not limited to, methotrexate and pemetrexed.

Microtubule interfering agents are chemotherapeutics that are directed against the formation of microtubules by tumor cells during M phase of the cell cycle. The group of anti-microtubule agents comprises vinca alkaloids and diterpenoids. Examples for vinca alkaloids include vinblastine, navelbine, vindesine, vinorelbine, and vincristine. Examples for diterpenoids include, but are not limited to, paclitaxel and docetaxel.

Platinum complexes are active against tumor cells by interacting with the cells' genomic DNA. More specifically, crosslinks are introduced into the DNA which introduces serious damage to the affected tumor cells. Examples for platinum complexes include, but are not limited to, cisplatin, carboplatin, and oxaliplatin.

Alkylating agents act by alkylating the genomic DNA of tumor cells, which interrupts normal function of the DNA and causes aptoptosis. Examples for alkylating agents include, but are not limited to, alkyl sulfonates (e.g., busulfan), ethyleneimines and methylmelamines (e.g., hexamethylmelamine, thiotepa), nitrogen mustards (e.g., melphalan, cyclophosphamide, mechlorethamine, uramustine, and chlorambucil), nitrosoureas (e.g., carmustine, streptozocin), and triazenes (e.g., dacarbazine, temozolomide).

Inhibitors of topoisomerase I or topoisomerase II inhibit these enzymes' essential function in DNA supercoiling during cell division. Examples for inhibitors of topoisomerase I include, but are not limited to, camptothecins (e.g., irinotecan, topotecan). Examples for inhibitors of topoisomerase II include, but are not limited to, amsacrine, etoposide, etoposide phosphate, and teniposide.

Signal transduction inhibitors are compounds that interfere with certain cellular processes such as, e.g., cell division. Compounds useful for treatment of neoplasms include, but are not limited to, inhibitors of receptor and non-receptor tyrosine kinases, inhibitors of serine/threonine kinases, inhibitors of phosphoinositide 3-kinases, inhibitors of Ras oncogenes, myoinositol signaling inhibitors, and SH2/SH3 domain blockers. Examples for inhibitors of receptor tyrosine kinases include, but are not limited to, erlotinib (Tarceva), gefitinib (Iressa), dasatinib (Sprycel), sorafenib (Nexavar), SU11248 (Sutent), and lapatinib (Tycerb).

Cell cycle signalling inhibitors are intended to interfere with cell cycle progression by inhibiting the biological effect of crucial signalling molecules such as, e.g., cyclins/cyclin-dependent kinases and other essential cell cycle regulators.

Angiogenesis inhibitors such as, e.g., antibodies or receptor tyrosine kinase inhibitors inhibit the neovascularization of tumors. Examples include, but are not limited to, anti-VEGF antibodies such as, e.g., bevacizumab.

Monoclonal antibodies (including, but not limited to, murine, chimeric, and humanized monoclonal antibodies) are capable of binding to surface antigens specific for tumor cells, thus rendering susceptible the cancer cells to attack by the immune system. Examples for monoclonal antibodies as antitumor agents include, but are not limited to, trastuzumab (Herceptin), rituximab (Rituxan), gemtuzumab ozogamicin (Mylotarg), alemtuzumab (Campath), ibritumomab tiuxetan (Zevalin), tositumomab (Bexxar), cetuximab (Erbitux), bevacizumab (Avastin), and panitumumab (Vectibix).

Proapoptotic agents are intended to unblock apoptosis in cancer cells by reducing Bcl-2 activity in malignant cells characterized by upregulation of Bcl-2 activity. A proapoptotic effect is mediated by, for example, without being limited thereto, bcl-2 antisense oligonucleotides.

Hormones and hormone analogues are useful to treat neoplasms in which cell growth is affected by the level at which a certain hormone or hormones (or analogues thereof) are present. Examples for classes of hormones and hormone analogues include, but are not limited to, adrenocorticosteroids, androgens, antiandrogens, aromatase inhibitors, estrogens, anti-estrogens, gonadotropin-releasing hormone and its analogues, and progestrins. Examples for individual hormones and hormone analogues include, but are not limited to, aminoglutethimide, anastrozole, bicalutamide, cyproterone acetate, dexamethasone, droloxifene, dutasteride, exemestane, finasteride, flutamide, gosereline, iodoxyfene, letrazole, luprolide, megrestrol acetate, nilutamide, prednisolone, prednisone, raloxifene, tamoxifen, toremifene, and vorazole.

Antibiotic chemotherapeutic agents (antineoplastics) bind to or intercalate with DNA, which leads to cell death. Examples of antibiotic agents effective against cancer cells include, but are not limited to, actinomycin D, bleomycin, plicamycin, mitomycin, and anthracyclins such as, e.g., doxorubicin, daunorubicin, epirubicin, and others.

Immunotherapeutic agents are activating the immune system in order to attack cancer cells. Examples for immunotherapeutic agents include, but are not limited to, monoclonal antibodies, radiolabeled murine antibodies, and agents used for topic immunotherapy such as, e.g., imiquimod.

Anti-emetic agents are agents effective against nausea and vomiting, which can arise as side effects of chemotherapy. Examples for anti-emetic agents include, but are not limited to, 5HT₃ receptor antagonists (e.g., dolasetron, granisetron, ondansetron, tropisetron, palonosetron, and others), dopamine antagonists (e.g., domperidone, metoclopramide, droperidol, haloperidol, chlorpromazine, promethazine, prochlorperazine, and others), antihistamines (H₁R antagonists; cyclizine, diphenhydramine, dimenhydrinate, meclizine, hydroxyzine, and others), benzodiazepines such as, e.g., midazolam and others, cannabinoids such as, e.g, cannabis, marinol and others, and other anti-emetic agents not falling into one of the above groups, such as, e.g., emetrol, propofol, trimethobenzamide, and ginger extracts, among others.

Analgetic agents are compounds effective in relieving pain. Examples for analgetics include, but are not limited to, NSAIDs (non-steroidal anti-inflammatory drugs such as, e.g., Aspirin, rofecoxib, celecoxib, and others), paracetamol, carbamazepine, gabapentin, pregabalin, opiates, morphinomimetics (e.g., morphine, codeine, oxycodone, hydrocodone, diamorphine, pethidine, tramadol, buprenorphine, and others), and tricyclic antidepressants such as, e.g., amitriptyline.

In a preferred embodiment, the pharmaceutical composition comprises a compound of the invention in combination with an alkylating agent. In a particularly preferred embodiment, the pharmaceutical composition comprises:
a compound of the invention and cyclophosphamid,
a compound of the invention and chlorambucil,
a compound of the invention and melphalan,
a compound of the invention and BCNU (carmustin),
a compound of the invention and CCNU (lomustin),
a compound of the invention and thiotepa,
a compound of the invention and busulfan,
a compound of the invention and dacarbazine, or
a compound of the invention and temozolomide.

In a further preferred embodiment, the pharmaceutical composition comprises a compound of the invention in combination with an antimetabolite. In a particularly preferred embodiment, the pharmaceutical composition comprises:
a compound of the invention and aminopterin,
a compound of the invention and 5-fluorouracil,
a compound of the invention and methotrexate,
a compound of the invention and cytarabin,
a compound of the invention and mercaptopurin,
a compound of the invention and azathioprin,
a compound of the invention and azacytidine,
a compound of the invention and gemcitabine, or
a compound of the invention and capecitabine.

In a further preferred embodiment, the pharmaceutical composition comprises a compound of the invention in combination with an alkaloid. In a particularly preferred embodiment, the pharmaceutical composition comprises:
a compound of the invention and vinblastin,
a compound of the invention and vincristin,
a compound of the invention and daunomycin,
a compound of the invention and adriamycin,
a compound of the invention and bleomycin,
a compound of the invention and bleomycin, or
a compound of the invention and procarbazin.

In a further preferred embodiment, the pharmaceutical composition comprises a compound of the invention in combination with a platinum derivative. In a particularly preferred embodiment, the pharmaceutical composition comprises:
a compound of the invention and cisplatin,
a compound of the invention and carboplatin,
a compound of the invention and oxaliplatin,
a compound of the invention and satraplatin.

In a further preferred embodiment, the pharmaceutical composition comprises a compound of the invention in combination with a tubulin inhibitor. In a particularly preferred embodiment, the pharmaceutical composition comprises:
a compound of the invention and paclitaxel, or
a compound of the invention and docetaxel.

In a further preferred embodiment, the pharmaceutical composition comprises a compound of the invention in combination with a topoisomerase inhibitor. In a particularly preferred embodiment, the pharmaceutical composition comprises:
a compound of the invention and irinotecan, or
a compound of the invention and topotecan.

In a further preferred embodiment, the pharmaceutical composition comprises a compound of the invention in combination with an inhibitor of the EGFR pathway. In a particularly preferred embodiment, the pharmaceutical composition comprises:
a compound of the invention and cetuximab,
a compound of the invention and trastuzumab,
a compound of the invention and gefitinib,
a compound of the invention and erlotinib,
a compound of the invention and lapatinib,
a compound of the invention and imatinib,
a compound of the invention and nilotinib, or
a compound of the invention and dasatinib.

In a further preferred embodiment, the pharmaceutical composition comprises a compound of the invention in combination with an inhibitor of farnesyltransferase. In a particularly preferred embodiment, the pharmaceutical composition comprises a compound of the invention and tipifarnib.

In a further preferred embodiment, the pharmaceutical composition comprises a compound of the invention in combination with an inhibitor of the VEGF-/ VEGFR signaling pathway. In a particularly preferred embodiment, the pharmaceutical composition comprises:
a compound of the invention and a VEGFR2 Mab,
a compound of the invention and bevacizumab,
a compound of the invention and sorafenib,
a compound of the invention and sunitinib, or
a compound of the invention and valatinib.

In a further preferred embodiment, the pharmaceutical composition comprises a compound of the invention in combination with a specific monoclonal antibody. In a particularly preferred embodiment, the pharmaceutical composition comprises:
a compound of the invention and trastuzumab,
a compound of the invention and rituximab,
a compound of the invention and gemtuzumab,
a compound of the invention and alemtuzumab,
a compound of the invention and ibritumomab,
a compound of the invention and tositumomab,
a compound of the invention and cetuximab,
a compound of the invention and bevacizumab,
a compound of the invention and panitumumab,
a compound of the invention and Anti-CD20 Mab, or
a compound of the invention and Anti-CD52 Mab.

In a further preferred embodiment, the pharmaceutical composition comprises a compound of the invention in combination with an Eg5 inhibitor. In a particularly preferred embodiment, the pharmaceutical composition comprises
a compound of the invention and ispinesib.

In a further preferred embodiment, the pharmaceutical composition comprises a compound of the invention in combination with a proteasome inhibitor. In a particularly preferred embodiment, the pharmaceutical composition comprises a compound of the invention and bortezomib.

In a further preferred embodiment, the pharmaceutical composition comprises a compound of the invention in combination with a cyclin-dependent kinase inhibitor. In a particularly preferred embodiment, the pharmaceutical composition comprises:
a compound of the invention and flavopyridol, or
a compound of the invention and R-roscovitine.

In a further preferred embodiment, the pharmaceutical composition comprises a compound of the invention in combination with a multitarget enzyme inhbitor. In a particularly preferred embodiment, the pharmaceutical composition comprises a compound of the invention and pemetrexed.

In a further preferred embodiment, the pharmaceutical composition comprises a compound of the invention in combination with a histon deacetylase inhbitor. In a particularly preferred embodiment, the pharmaceutical composition comprises:
a compound of the invention and vorinostat,
a compound of the invention and belinostat,
a compound of the invention and LBH589,
a compound of the invention and depsipeptide,
a compound of the invention and 2-propylpentanoic acid,
a compound of the invention and BYK 397957,
a compound of the invention and BYK408740.

In a further preferred embodiment, the pharmaceutical composition comprises a compound of the invention in combination with a hypo- or demethylating agent. In a particularly preferred embodiment, the pharmaceutical composition comprises:
a compound of the invention and procaine,
a compound of the invention and azacitidine, or
a compound of the invention and decitabine.

The invention additionally relates to a pharmaceutical composition comprising at least one of the compounds of the invention, at least one pharmaceutically acceptable carrier, diluent or excipient, and two additional therapeutic agents with the proviso that the compound or compounds of the invention and the two addtional therapeutic agents are therapeutically compatible. By therapeutically compatible it is meant that (a) the toxicity of the compound or compounds of the invention and the toxicity of the two addtional therapeutic agents do not add up to an unacceptable toxicity, and (b) that the therapeutic effect of the compound or compounds of the invention is not modified or reduced in an unacceptable or undesired way by the two additional therapeutic agents, and vice versa. In a preferred embodiment, the therapeutic effect of the compound or compounds of the invention and the therapeutic effect of the two addtional therapeutic agents are additive. In another preferred embodiment, the therapeutic effect of the compound or compounds of the invention and the therapeutic effect of the two addtional therapeutic agents are synergistic, i.e., their combined effect is different from, preferably more beneficial than, the pure sum of all three therapeutic effects when the compound or compounds and the agents are administered alone, without any interaction of the effects the compound or compounds and the effects the agents exert to the treated patient.

In a further preferred embodiment, the first and the second additional therapeutic agents are independently selected from anti-neoplastic agents, cytostatic agents, cytotoxic agents, antimetabolites, microtubule interfering agents, platinum derivatives, DNA alkylating agents, DNA intercalating agents, topoisomerase I inhibitors, topoisomerase II inhibitors, signal transduction inhibitors including kinase inhibitors, cell cycle signalling inhibitors, angiogenesis inhibitors including antibodies and receptor tyrosine kinase inhibitors, proapoptotic agents, hormones, hormone analogues, antibiotic agents, immunotherapeutic agents, monoclonal antibodies (including, but not limited to, murine, chimeric, and humanized monoclonal antibodies), anti-emetic agents, and analgetic agents, in form of the free compounds, the pharmaceutically acceptable salts thereof, the pharmaceutically acceptable derivatives thereof (e.g., but not limited to, ester derivatives), the solvates thereof and the stereoisomers of the compounds, salts, derivatives and solvates.

In a particularly preferred embodiment, one of the two additional therapeutic agents is a taxane.

In a further particularly preferred embodiment, one of the two additional therapeutic agents is a platinum compound.

In a further particularly preferred embodiment, one of the two additional therapeutic agents is a hormone or hormone analogue.

In a further particularly preferred embodiment, one of the two additional therapeutic agents is a monoclonal antibody.

The invention also relates to a kit of parts, comprising at least one pharmaceutical composition comprising at least one of the compounds of the invention, and at least one pharmaceutical composition comprising two additional therapeutic agents.

The invention also relates to a kit of parts, comprising at least one pharmaceutical composition comprising at least one of the compounds of the invention and a first additional therapeutic agent, and at least one pharmaceutical composition comprising a second additional therapeutic agent.

The invention also relates to a kit of parts, comprising at least one pharmaceutical composition comprising at least one of the compounds of the invention, at least one pharmaceutical composition comprising a first additional therapeutic agent, and at least one pharmaceutical composition comprising a second additional therapeutic agent.

The above mentioned compound of the invention is preferably a compound according to the examples.

The invention furthermore relates to pharmaceutical compositions according to the invention, as defined above, inhibiting Plk1, especially for the treatment or prophylaxis of diseases alleviated by inhibition of Plk1, in particular for the treatment or prophylaxis of the diseases exemplified above.

The invention also encompasses pharmaceutical compositions according to the invention, as defined above, for the treatment or prophylaxis of the following diseases involving abnormal cell growth: Cancers of the colon including colorectal carcinoma, ovaries, breast, prostate, bladder, lung, gastrointestinal carcinomas, gastrointestinal stromal tumors, small cell lung cancer, head and neck cancer, cancers of cervix, pancreas, esophagus, kidney, larynx and hypopharynx, liver, endocrine glands, soft tissue, testis, retinoblastoma and Wilms tumor, endometrial tumors, malignant melanoma, non-Hodgekins lymphoma, Hodgekins lymphoma or other lymphomas, chronic and acute myeloic leukemia, acute lymphoblastic leukemia, T-cell lymphoma, plasma cell neoplasia, and leukemias, hematological malignancies including multiple myeloma, soft tissue sarcoma, osteosarcoma, fibrosarcoma, and other tumors of mesothelial origin, glioma, astrocytoma, cancers of unknown primary site, glioblastoma and other brain tumors and tumors of neuronal origin, germ cell cancers, myelodysplastic syndromes, myeloproliferative syndromes, disorders such as polycythemia vera, essential thrombocytopenia, myelofibrosis, hypereosinophilic syndrome, or other paraneoplastic syndromes. In another embodiment, said abnormal cell growth is a benign proliferative disease such as, e.g., benign prostatic hyperplasia, restenosis, fibrosis, or psoriasis, or any other kind of unwanted cell or tissue proliferation.

The pharmaceutical compositions according to the invention preferably contain the compound or compounds of the invention in a total amount of from 0.1 to 99.9 wt%, more preferably 5 to 95 wt%, in particular 20 to 80 wt%. In case at least one therapeutic agent selected from anti-neoplastic agents in general, cytostatic agents, cytotoxic agents, antimetabolites, microtubule interfering agents, platinum derivatives, alkylating agents, topoisomerase I inhibitors, topoisomerase II inhibitors, signal transduction inhibitors, cell cycle signalling inhibitors, angiogenesis inhibitors including antibodies and receptor tyrosine kinase inhibitors, immunotherapeutic agents, monoclonal antibodies (including, but not limited to, murine, chimeric, and humanized monoclonal antibodies), proapoptotic agents, hormones, hormone analogues, antibiotic agents, anti-emetic agents, and analgetic agents is present in the pharmaceutical compositions of the invention, the total amount of said therapeutic agent or therapeutic agents in the pharmaceutical compositions is preferably in the range of from 0.1 to 99.9 wt%, more preferably 5 to 95 wt%, in particular 20 to 80 wt%, with the proviso that the total amount of the compound or compounds of the invention and the therapeutic agent or therapeutic agents is less than 100 wt%. Preferably, the at least one compound of the invention and the at least one therapeutic agent are present in the pharmaceutical composition in a weight ratio of from 1000:1 to 1:1000.

As pharmaceutically acceptable auxiliaries, any auxiliaries known to be suitable for preparing pharmaceutical compositions can be used. Examples thereof include, but are not limited to, solvents, diluents, excipients, dispersants, emulsifiers, solubilizers, gel formers, ointment bases, antioxidants, preservatives, stabilizers, carriers, fillers, binders, thickeners, complexing agents, disintegrating agents, buffers, permeation promoters, polymers, lubricants, coating agents, propellants, tonicity adjusting agents, surfactants, colorants, flavorings, sweeteners and dyes. In particular, auxiliaries of a type appropriate to the desired formulation and the desired mode of administration are used.

The pharmaceutical compositions can be formulated, for example, into tablets, coated tablets (dragees), pills, cachets, capsules (caplets), granules, powders, suppositories, solutions (e.g., but not limited to, sterile solutions), emulsions, suspensions, ointments, creams, lotions, pastes, oils, gels, sprays and patches (for example, without being limited thereto, transdermal therapeutic systems). Additionally, the pharmaceutical compositions can be prepared as, e.g., liposome delivery systems, systems in which the compound of the invention is coupled to monoclonal antibodies, and systems in which the compound of the invention is coupled to polymers (for example, without being limited thereto, soluble or biodegradable polymers).

In case of pharmaceutical compositions comprising at least one of the compounds of the invention and at least one therapeutic agent selected from anti-neoplastic agents in general, cytostatic agents, cytotoxic agents, antimetabolites, anti-microtubule agents, platinum derivatives, alkylating agents, topoisomerase I inhibitors, topoisomerase II inhibitors, signal transduction inhibitors, cell cycle signalling inhibitors, angiogenesis inhibitors including antibodies and receptor tyrosine kinase inhibitors, immunotherapeutic agents, monoclonal antibodies (including, but not limited to, murine, chimeric, and humanized monoclonal antibodies), proapoptotic agents, hormones, hormone analogues, antibiotic agents, anti-emetic agents, and analgetic agents, the compound of the invention and the therapeutic agent may be formulated together into the same dosage form (for example, without being limited thereto, tablets or solutions for injection), separately into the same dosage form (for example, without being limited thereto, tablets or solutions for injection), or into different dosage forms (for example, without being limited thereto, the compound of the invention may be formulated as tablet and the therapeutic agent may be formulated as powder, solution or suspension).

The pharmaceutical compositions can be manufactured in a manner known to a person skilled in the art, e.g. by dissolving, mixing, granulating, dragee-making, levigating, emulsifying, encapsulating, entrapping or lyophilizing processes.

The selected formulation depends *inter alia* on the route of administering the pharmaceutical composition. The pharmaceutical compositions of the invention can be administered by any suitable route, for example, by the oral, sublingual, buccal, intravenous, intraarterial, intramuscular, subcutaneous, intracutaneous, topical, transdermal, intranasal, intraocular, intraperitoneal, intrasternal, intracoronary, transurethral, rectal or vaginal route, by inhalation or by insufflation. Oral administration is preferred.

In case of pharmaceutical compositions comprising at least one of the compounds of the invention and at least one therapeutic agent selected from anti-neoplastic agents in general, cytostatic agents, cytotoxic agents, antimetabolites, anti-microtubule agents, platinum derivatives, alkylating agents, topoisomerase I inhibitors, topoisomerase II inhibitors, signal transduction inhibitors, cell cycle signalling inhibitors, angiogenesis inhibitors including antibodies and receptor tyrosine kinase inhibitors, immunotherapeutic agents, monoclonal antibodies (including, but not limited to, murine, chimeric, and humanized monoclonal antibodies), proapoptotic agents, hormones, hormone analogues, antibiotic agents, anti-emetic agents, and analgetic agents, the compound of the invention and the therapeutic agent may be administered by the same route, e.g., without limitation, orally, or by different routes, e.g., without limitation, the compound of the invention can be administered orally and the therapeutic agent can be administered topically or by injection.

Tablets, coated tablets (dragees), pills, cachets, capsules (caplets), granules, solutions, emulsions and suspensions are, e.g., suitable for oral administration. In particular, said formulations can be adapted so as to represent, for example, an enteric form, an immediate release form, a delayed release form, a repeated dose release form, a prolonged release form or a sustained release form. Said forms can be obtained, for example, by coating tablets, by dividing tablets into several compartments separated by layers disintegrating under different conditions (e.g., under different pH conditions) or by coupling the compound of the invention to a biodegradable polymer.

Administration by inhalation is preferably made by using an aerosol. The aerosol is a liquid-gaseous dispersion, a solid-gaseous dispersion or a mixed liquid/solid-gaseous dispersion.

The aerosol may be generated by means of aerosol-producing devices such as dry powder inhalers (DPls), pressurized metered dose inhalers (PMDIs) and nebulizers. Depending on the kind of the compound of the invention, and optionally the therapeutic agent, to be administered, the aerosol-producing device can contain the compound and, optionally, the therapeutic agent in form of a powder, a solution or a dispersion. The powder may contain, for example, one or more of the following auxiliaries: carriers, stabilizers and fillers. The solution may contain in addition to the solvent, for example, one or more of the following auxiliaries: propellants, solubilizers (co-solvents), surfactants, stabilizers, buffers, tonicity adjusting agents, preservatives and flavorings. The dispersion may contain in addition to the dispersant, for example, one or more of the following auxiliaries: propellants, surfactants, stabilizers, buffers, preservatives and flavorings. Examples of carriers include, but are not limited to, saccharides, e.g. lactose and glucose. Examples of propellants include, but are not limited to, fluorohydrocarbons, e.g. 1,1,1,2-tetrafluoroethane and 1,1,1,2,3,3,3-heptafluoropropane.

The particle size of the aerosol particles (solid, liquid or solid/liquid particles) is preferably less than 100 µm, more preferably it is in the range of from 0.5 to 10 µm, in particular in the range of from 2 to 6 µm (D50 value, measured by laser diffraction).

Specific aerosol-producing devices which may be used for inhaled administration include, but are not limited to, Cyclohaler®, Diskhaler®, Rotadisk®, Turbohaler®, Autohaler®, Turbohaler®, Novolizer®, Easyhaler®, Aerolizer®, Jethaler®, Diskus®, Ultrahaler® and Mystic® inhalers. The aerosol-producing devices may be combined with spacers or expanders, e.g. Aerochamber®, Nebulator®, Volumatic® and Rondo®, for improving inhalation efficiency.

In case of topical administration, suitable pharmaceutical formulations are, for example, ointments, creams, lotions, pastes, gels, powders, solutions, emulsions, suspensions, oils, sprays, and patches (for example, without being limited thereto, transdermal therapeutic systems).

For parenteral modes of administration such as, for example, intravenous, intraarterial, intramuscular, subcutaneous, intracutaneous, intraperitoneal and intrasternal administration, preferably solutions (for example, without being limited thereto, sterile solutions or isotonic solutions) are used. They are preferably administered by injection or infusion techniques.

In case of intranasal administration, for example, sprays and solutions to be applied in drop form are preferred formulations.

For intraocular administration, solutions to be applied in drop form, gels and ointments are exemplified formulations.

Generally, the pharmaceutical compositions according to the invention can be administered such that the dose of the compound of the invention is in the range customary for Plk1 inhibitors. In particular, a dose in the range of from 0.01 to 5000 mg of the compound of the invention per day is preferred for an average adult patient having a body weight of 70 kg. In this respect, it is to be noted that the dose is dependent, for example, on the specific compound used, the species treated, age, body weight, general health, sex and diet of the subject treated, mode and time of administration, rate of excretion, severity of the disease to be treated and drug combination. In case the pharmaceutical composition of the invention comprises at least one of the compounds of the invention and at least one therapeutic agent selected from anti-neoplastic agents in general, cytostatic agents, cytotoxic agents, antimetabolites, anti-microtubule agents, platinum derivatives, alkylating agents, topoisomerase I inhibitors, topoisomerase II inhibitors, signal transduction inhibitors, cell cycle signalling inhibitors, angiogenesis inhibitors including antibodies and receptor tyrosine kinase inhibitors, immunotherapeutic agents, monoclonal antibodies, proapoptotic agents, hormones, hormone analogues, antibiotic agents, anti-emetic agents, and analgetic agents, the same dose ranges apply to the therapeutic agent.

The pharmaceutical compositions according to the invention can be administered in a single dose per day or in multiple subdoses, for example, 2 to 4 doses per day. A single dose unit of the pharmaceutical composition can contain e.g. from 0.01 mg to 5000 mg, preferably 0.1 mg to 2000 mg, more preferably 0.5 to 1000 mg, most preferably 1 to 500 mg, of the compound of the invention. In case the pharmaceutical composition of the invention comprises at least one of the compounds of the invention and at least one therapeutic agent selected from anti-neoplastic agents in general, cytostatic agents, cytotoxic agents, antimetabolites, anti-microtubule agents, platinum derivatives, alkylating agents, topoisomerase I inhibitors, topoisomerase II inhibitors, signal transduction inhibitors, cell cycle signalling inhibitors, angiogenesis inhibitors including antibodies and receptor tyrosine kinase inhibitors, immunotherapeutic agents, monoclonal antibodies, proapoptotic agents, hormones, hormone analogues, antibiotic agents, anti-emetic agents, and analgetic agents, a single dose unit of the pharmaceutical composition can contain e.g. from 0.01 mg to 5000 mg, preferably 0.1 mg to 2000 mg, more preferably 0.5 to 1000 mg, most preferably 1 to 500 mg, of the therapeutic agent. Furthermore, the pharmaceutical composition can be adapted to weekly, monthly or even more infrequent administration, for example by using an implant, e.g. a subcutaneous or intramuscular implant, by using the compound of the invention in form of a sparingly soluble salt, or by using the compound of the invention coupled to a polymer. Administration of the pharmaceutical composition in a single dose per day is preferred.

In case the pharmaceutical composition of the invention comprises at least one of the compounds of the invention and at least one therapeutic agent selected from anti-neoplastic agents in general, cytostatic agents, cytotoxic agents, antimetabolites, anti-microtubule agents, platinum derivatives, alkylating agents, topoisomerase I inhibitors, topoisomerase II inhibitors, signal transduction inhibitors, cell cycle signalling inhibitors, angiogenesis inhibitors including antibodies and receptor tyrosine kinase inhibitors, immunotherapeutic agents, monoclonal antibodies, proapoptotic agents, hormones, hormone analogues, antibiotic agents, anti-emetic agents, and analgetic agents, administration of the compound of the invention and administration of the therapeutic agent can be made simultaneously or sequentially. In case of sequential administration, the compound of the invention can be administered before or after administration of the therapeutic agent.

### Biological investigations

The inhibition of Plk1 kinase activity can be measured using recombinant Plk1 protein expressed as an N-terminally histidine-tagged protein in SF9 insect cells and alpha casein as substrate. The PIk1 assay is run in 96 well plates by incubating 50 to 100 ng per well recombinant PIk1, 500 ng per well alpha casein (Sigma, # C-6780) as substrate, 10 µl of compound of invention (test compounds were dissolved as 20 mM solutions in dimethylsulfoxide (DMSO) and subsequently diluted), and 100 nM Li-ATP (including ³³P-ATP) in 100 µl of reaction buffer (50 nM HEPES, pH 7.5; 3 mM MgCl₂; 3 mM MnCl₂; 3 µM Na-Orthovanadat; 1 mM DTT; 1 µg/ml PEG 8000) for 80 minutes at 30°C. The reactions are terminated by adding 100 µl stopping buffer (2% H₃PO₄ for 5 minutes) and are washed 3 times with washing solution (0,9% NaCl). Determining ³³P incorporated into the protein substrate alpha casein, the detection is based on the adhesion of the phosphorylated protein to the surface of scintillator-coated flash plates (Perkin Elmer, USA; # SMP-200). Phosphorylation of alpha casein is measured by counting the radioactivity bound to the plate for 60 sec. using a plate reader (Perkin Elmer, USA; Wallac Microbeta). By this method, the IC₅₀ value of the PIk1 inhibition is determined as described above. Preferred compounds are characterized by an IC₅₀ value of Plk1 inhibition of below 1 µM.

**Table 1**

| compound (example #) | Plk1 inhibition |
|---|---|
| 1 | +++ |
| 2 | +++ |
| 3 | ++ |
| 4 | ++ |
| 5 | ++ |
| 6 | ++ |
| 7 | +++ |
| 8 | ++++ |
| 9 | +++ |
| 10 | ++ |
| 11 | + |
| 12 | ++++ |
| 13 | +++ |
| 14 | +++ |
| 15 | +++ |
| 16 | +++ |
| 17 | +++ |
| 18 | +++ |
| 19 | +++ |
| 20 | ++ |
| 21B | +++ |
| 21A | ++ |
| 22 | ++++ |
| 23 | +++ |

Plk1 inhibition as determined in enzymatic assays; cytotoxicity in cellular assays:
- ++++: pIC₅₀ >8
- +++: pIC₅₀ >7
- ++: pIC₅₀ >6
- +: pIC₅₀ >5
- -: pIC₅₀ <5

## Claims

1. Compound of formula (I) 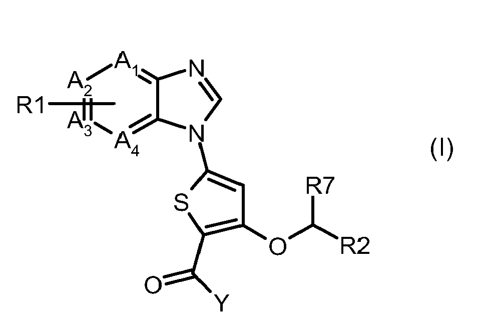 wherein
R1 is -H, -CH₂OH, -CH₂N(R3)R4, -C(O)N(R5)R6, -OCH₃, -F, -Cl, -Br, -CH₂SO₂CH₃, or -CH₂O-piperidyl, the piperidyl group optionally being N-substituted by C1-C4 alkyl,
and wherein R1 is attached to either A₂ or to A₃;
R2 is phenyl optionally having 1 to 3 substituents independently selected from -F, -Cl, -Br, -I, -CN, -OH, -NO₂, -SO₂CH₃, C1-C4 alkyl optionally being substituted with 1 to 3 F atoms, C1-C4 alkoxy optionally being substituted with 1 to 3 F atoms, C1-C4 alkylamino, and C1-C4 dialkylamino;
A₁, A₂, A₃ and A₄ independently are CR1, CH or N,
wherein one of A₁, A₂, A₃ and A₄ is N, one of A₂ and A₃ is CR1, and all others are CH;
Y is -OH or -NH₂;
R3 is -H, -SO₂CH₃, C1-C4 alkyl, C2-C3 alkyl substituted with -OR8, C2-C3 alkyl substituted with -N(R8)R9, C3-C6 cycloalkyl, or saturated four- to six-membered heterocyclyl with at least one ring atom being N, the heterocyclyl group optionally being N-substituted with C1-C4 alkyl, phenyl, pyridyl, or pyrimidyl;
R4 is -H, -SO₂CH₃, C1-C4 alkyl, C2-C3 alkyl substituted with -OR8, C2-C3 alkyl substituted with -N(R8)R9, C3-C6 cycloalkyl, or saturated four- to six-membered heterocyclyl with at least one ring atom being N, the heterocyclyl group optionally being N-substituted with C1-C4 alkyl, phenyl, pyridyl, or pyrimidyl;
or
R3 and R4, together with the nitrogen atom they are bound to, form a saturated four- to seven-membered heterocycle, the heterocycle optionally containing one more heteroatom which is N, O or S, S optionally being oxidized to a -SO- group or a -SO₂- group,
and the heterocycle optionally being substituted by one or two substituents which independently are -OH, -F, amino, C1-C4 alkylamino, C1-C4 dialkylamino, C1-C4 alkyl optionally being substituted with 1 to 3 F atoms, C1-C4 acyl, benzoyl, phenoxy, 1-carboxamidyl optionally being substituted with C1-C4 alkyl or phenyl, 1-carboximidamidyl, sulfonyl optionally being substituted with C1-C4 alkyl or phenyl, 2-aminoethyl, 2-N-(C1-C4 alkyl)aminoethyl, 2-N,N-di(C1-C4 alkyl)aminoethyl, phenyl, pyridyl, pyrimidyl, benzyl, allyl, cyclopropyl, cyclobutyl, cyclopentyl, cyclohexyl, cyanomethyl, 2-cyanoethyl, 2-methansulfonylethyl, hydroxymethyl, 2-hydroxyethyl, 3-hydroxypropyl, (C1-C4 alkoxy)methyl, 2-(C1-C4 alkoxy)ethyl, 3-(C1-C4 alkoxy)propyl, N-methylpiperidyl, 1-phenylethyl, pyridylmethyl, N-methylpiperidylmethyl, 2-morpholinylethyl, morpholinocarbonylethyl, N,N-dimethylcarbonylmethyl, anilinocarbonylmethyl, N-methylanilinocarbonylmethyl, N-pyrrolidinocarbonylmethyl, N,N-dimethylsulfonyl, N-methylpiperazinyl, N-methylpiperazinylcarbonyl, or oxo,
or
R3 and R4, together with the nitrogen atom they are bound to, form a bridged system which is 2,5-diazabicyclo[2.2.1]hept-2-yl or 5-methyl-2,5-diazabicyclo [2.2.1]hept-2-yl;
R5 is -H, methyl, C1-C4 alkoxyethyl, or four- to six-membered N-ethylheterocyclyl;
R6 is -H, methyl, C1-C4 alkoxyethyl, or four- to six-membered N-ethylheterocyclyl;
R7 is -H, methyl, ethyl, -CH₂OH or -CF₃;
R8 is -H or C1-C4 alkyl; and
R9 is -H or C1-C4 alkyl;
or
R8 and R9, together with the nitrogen atom they are bound to, form a saturated four- to six-membered heterocycle, the heterocycle optionally containing one more heteroatom which is N, O or S, S optionally being oxidized to a -SO-group or a -SO₂- group;
a salt of one of the compounds, a stereoisomer of one of the compounds, or a salt of a stereoisomer of one of the compounds.

2. Compound according to claim 1, wherein
R1 is -H, -CH₂OH, -CH₂N(R3)R4, -C(O)N(R5)R6, -OCH₃, -F, -Cl, -Br, -CH₂SO₂CH₃, or -CH₂O-piperidyl, the piperidyl group optionally being N-substituted by C1-C4 alkyl,
and wherein R1 is attached to either A₂ or to A₃;
R2 is phenyl optionally having 1 to 3 substituents independently selected from -F, -Cl, -Br, I, -CN, -SO₂CH₃, C1-C4 alkyl optionally being substituted with 1 to 3 F atoms, C1-C4 alkoxy optionally being substituted with 1 to 3 F atoms, C1-C4 alkylamino, and C1-C4 dialkylamino;
A₁ and A₄ are C, one of A₂ and A₃ is CR1 and the other of A₂ and A₃ is N;
Y is -OH or -NH₂;
R3 is -H, -SO₂CH₃, C1-C4 alkyl, C2-C3 alkyl substituted with -OR8, C2-C3 alkyl substituted with -N(R8)R9, C3-C6 cycloalkyl, piperidine-4-yl optionally being N-substituted with C1-C4 alkyl, phenyl, pyridyl, or pyrimidyl, or piperidine-3-yl optionally being N-substituted with C1-C4 alkyl, phenyl, pyridyl, or pyrimidyl;
R4 is -H, -SO₂CH₃, C1-C4 alkyl, C2-C3 alkyl substituted with -OR8, C2-C3 alkyl substituted with -N(R8)R9, C3-C6 cycloalkyl, piperidine-4-yl optionally being N-substituted with C1-C4 alkyl, phenyl, pyridyl, or pyrimidyl, or piperidine-3-yl optionally being N-substituted with C1-C4 alkyl, phenyl, pyridyl, or pyrimidyl;
or
R3 and R4, together with the nitrogen atom they are bound to, form a saturated four- to seven-membered heterocycle, the heterocycle optionally containing one more heteroatom which is N, O or S, S optionally being oxidized to a -SO- group or a -SO₂- group,
and the heterocycle optionally being substituted by one or two substituents which independently are -OH, -F, amino, C1-C4 alkylamino, C1-C4 dialkylamino, C1-C4 alkyl optionally being substituted with 1 to 3 F atoms, C1-C4 acyl, benzoyl, phenoxy, 1-carboxamidyl optionally being substituted with C1-C4 alkyl or phenyl, 1-carboximidamidyl, sulfonyl optionally being substituted with C1-C4 alkyl or phenyl, 2-aminoethyl, 2-N-(C1-C4 alkyl)aminoethyl, 2-N,N-di(C1-C4 alkyl)aminoethyl, phenyl, pyridyl, pyrimidyl, benzyl, allyl, cyclopropyl, cyclobutyl, cyclopentyl, cyclohexyl, cyanomethyl, 2-cyanoethyl, 2-methansulfonylethyl, hydroxymethyl, 2-hydroxyethyl, 3-hydroxypropyl, (C1-C4 alkoxy)methyl, 2-(C1-C4 alkoxy)ethyl, 3-(C1-C4 alkoxy)propyl, N-methylpiperidyl, 1-phenylethyl, pyridylmethyl, N-methylpiperidylmethyl, 2-morpholinylethyl, morpholinocarbonylethyl, N,N-dimethylcarbonylmethyl, anilinocarbonylmethyl, N-methylanilinocarbonylmethyl, N-pyrrolidinocarbonylmethyl, N,N-dimethylsulfonyl, N-methylpiperazinyl, N-methylpiperazinylcarbonyl, or oxo,
or
R3 and R4, together with the nitrogen atom they are bound to, form a bridged system which is 2,5-diazabicyclo[2.2.1]hept-2-yl or 5-methyl-2,5-diazabicyclo [2.2.1]hept-2-yl;
R5 is -H, methyl, C1-C4 alkoxyethyl, or four- to six-membered N-ethylheterocyclyl;
R6 is -H, methyl, C1-C4 alkoxyethyl, or four- to six-membered N-ethylheterocyclyl;
R7 is -H, methyl, ethyl, -CH₂OH, or -CF₃;
R8 is -H or C1-C4 alkyl; and
R9 is -H or C1-C4 alkyl;
or
R8 and R9, together with the nitrogen atom they are bound to, form a saturated four- to six-membered heterocycle, the heterocycle optionally containing one more heteroatom which is N, O or S, S optionally being oxidized to a -SO-group or a -SO₂- group;
a salt of one of the compounds, a stereoisomer of one of the compounds, or a salt of a stereoisomer of one of the compounds.

3. Compound according to claim 1, wherein
R1 is -H, -CH₂N(R3)R4, -CH₂SO₂CH₃, or -CH₂O-piperidyl, the piperidyl group optionally being N-substituted by C1-C4 alkyl,
and wherein R1 is attached to either A₂ or to A₃;
R2 is phenyl optionally having 1 to 3 substituents independently selected from -F, -Cl, -Br, I, -CN, -SO₂CH₃, C1-C4 alkyl optionally being substituted with 1 to 3 F atoms, C1-C4 alkoxy optionally being substituted with 1 to 3 F atoms, C1-C4 alkylamino, and C1-C4 dialkylamino;
A₁ and A₄ are C, one of A₂ and A₃ is CR1 and the other of A₂ and A₃ is N;
Y is -OH or -NH₂;
R3 is -H, C2-C3 alkyl substituted with -OR8, C2-C3 alkyl substituted with -N(R8)R9, C3-C6 cycloalkyl, piperidine-4-yl optionally being N-substituted with C1-C4 alkyl, phenyl, pyridyl, or pyrimidyl, or piperidine-3-yl optionally being N-substituted with C1-C4 alkyl, phenyl, pyridyl, or pyrimidyl;
R4 is -H, C2-C3 alkyl substituted with -OR8, C2-C3 alkyl substituted with -N(R8)R9, C3-C6 cycloalkyl, piperidine-4-yl optionally being N-substituted with C1-C4 alkyl, phenyl, pyridyl, or pyrimidyl, or piperidine-3-yl optionally being N-substituted with C1-C4 alkyl, phenyl, pyridyl, or pyrimidyl;
or
R3 and R4, together with the nitrogen atom they are bound to, form a saturated four- to seven-membered heterocycle, the heterocycle optionally containing one more heteroatom being N, O or S, S optionally being oxidized to a -SO- group or a -SO₂- group,
and the heterocycle optionally being substituted by one or two substituents which independently are -OH, -F, amino, C1-C4 alkylamino, C1-C4 dialkylamino, C1-C4 alkyl optionally being substituted with 1 to 3 F atoms, C1-C4 acyl, benzoyl, phenoxy, 1-carboxamidyl optionally being substituted with C1-C4 alkyl or phenyl, 1-carboximidamidyl, sulfonyl optionally being substituted with C1-C4 alkyl or phenyl, 2-aminoethyl, 2-N-(C1-C4 alkyl)aminoethyl, 2-N,N-di(C1-C4 alkyl)aminoethyl, phenyl, pyridyl, pyrimidyl, benzyl, allyl, cyclopropyl, cyclobutyl, cyclopentyl, cyclohexyl, cyanomethyl, 2-cyanoethyl, 2-methansulfonylethyl, hydroxymethyl, 2-hydroxyethyl, 3-hydroxypropyl, (C1-C4 alkoxy)methyl, 2-(C1-C4 alkoxy)ethyl, 3-(C1-C4 alkoxy)propyl, N-methylpiperidyl, 1-phenylethyl, pyridylmethyl, N-methylpiperidylmethyl, 2-morpholinylethyl, morpholinocarbonylethyl, N,N-dimethylcarbonylmethyl, anilinocarbonylmethyl, N-methylanilinocarbonylmethyl, N-pyrrolidinocarbonylmethyl, N,N-dimethylsulfonyl, N-methylpiperazinyl, N-methylpiperazinylcarbonyl, or oxo,
or
R3 and R4, together with the nitrogen atom they are bound to, form a bridged system which is 2,5-diazabicyclo[2.2.1]hept-2-yl or 5-methyl-2,5-diazabicyclo [2.2.1]hept-2-yl;
R7 is -H, methyl, ethyl, -CH₂OH, or -CF₃;
R8 is -H or C1-C4 alkyl; and
R9 is -H or C1-C4 alkyl;
or
R8 and R9, together with the nitrogen atom they are bound to, form a saturated four- to six-membered heterocycle optionally containing one more heteroatom being N, O or S, S optionally being oxidized to a -SO- group or a -SO₂- group;
a salt of one of the compounds, a stereoisomer of one of the compounds, or a salt of a stereoisomer of one of the compounds.

4. Compound according to claim 1, wherein
R1 is -CH₂N(R3)R4, -CH₂SO₂CH₃, or -CH₂O-piperidyl, the piperidyl group optionally being N-substituted by C1-C4 alkyl,
and wherein R1 is attached to either A₂ or to A₃;
R2 is phenyl having a substituent in the 2-position and optionally up to two substituents in the remaining positions, the substituents being -F, -Cl, -Br, -I, -CN, -SO₂CH₃, C1-C4 alkyl optionally being substituted with 1 to 3 F atoms, C1-C4 alkoxy optionally being substituted with 1 to 3 F atoms, C1-C4 alkylamino, or C1-C4 dialkylamino;
A₁ and A₄ are C, one of A₂ and A₃ is CR1 and the other of A₂ and A₃ is N;
Y is -NH₂;
R3 is -H or piperidine-4-yl optionally being N-substituted with C1-C4 alkyl; and
R4 is -H or piperidine-4-yl optionally being N-substituted with C1-C4 alkyl;
or
R3 and R4, together with the nitrogen atom they are bound to, form a piperazine ring or 1,4-diazepane ring, the piperazine or 1,4-diazepane optionally being substituted by one or two substituents independently being -F, C1-C4 alkyl, C1-C4 alkyl substituted with 1 to 3 F atoms, C1-C4 acyl, benzoyl, 1-carboxamidyl optionally being substituted with C1-C4 alkyl or phenyl, 1-carboximidamidyl, sulfonyl optionally being substituted with C1-C4 alkyl or phenyl, 2-aminoethyl, 2-N-(C1-C4 alkyl)aminoethyl, 2-N,N-di(C1-C4 alkyl)aminoethyl, phenyl, pyridyl, pyrimidyl, benzyl, allyl, cyclopropyl, cyclobutyl, cyclopentyl, cyclohexyl, cyanomethyl, 2-cyanoethyl, 2-methansulfonylethyl, hydroxymethyl, 2-hydroxyethyl, 3-hydroxypropyl, (C1-C4 alkoxy)methyl, 2-(C1-C4 alkoxy)ethyl, 3-(C1-C4 alkoxy)propyl, N-methylpiperidyl, 1-phenylethyl, pyridylmethyl, N-methylpiperidylmethyl, 2-morpholinylethyl, morpholinocarbonylethyl, N,N-dimethylcarbonylmethyl, anilinocarbonylmethyl, N-methylanilinocarbonylmethyl, N-pyrrolidinocarbonylmethyl, N,N-dimethylsulfonyl, or oxo,
or
R3 and R4, together with the nitrogen atom they are bound to, form a bridged system which is 2,5-diazabicyclo[2.2.1]hept-2-yl or 5-methyl-2,5-diazabicyclo [2.2.1]hept-2-yl;
R7 is -H, methyl, -CH₂OH, or -CF₃;
a salt of one of the compounds, a stereoisomer of one of the compounds, or a salt of a stereoisomer of one of the compounds.

5. Compound according to claim 1, wherein
R1 is -CH₂N(R3)R4 and is attached to either A₂ or to A₃;
R2 is phenyl having a -F, -Cl, -Br, -I, trifluormethyl, difluormethoxy or trifluormethoxy substituent in the 2-position and optionally up to two substituents in the remaining positions, the up to two substituents independently being -F, -Cl, -Br, I, -CN, -SO₂CH₃, C1-C4 alkyl optionally being substituted with 1 to 3 F atoms, C1-C4 alkoxy optionally being substituted with 1 to 3 F atoms, C1-C4 alkylamino, or C1-C4 dialkylamino;
A₁ and A₄ are C, one of A₂ and A₃ is CR1 and the other of A₂ and A₃ is N;
Y is -NH₂;
R3 and R4, together with the nitrogen atom they are bound to, form a piperazine ring or 1,4-diazepane ring, the piperazine or 1,4-diazepane optionally being substituted by one or two substituents independently being -F, C1-C4 alkyl optionally being substituted with 1 to 3 F atoms, C1-C4 acyl, benzoyl, 1-carboxamidyl optionally being substituted with C1-C4 alkyl or phenyl, 1-carboximidamidyl, sulfonyl optionally being substituted with C1-C4 alkyl or phenyl, 2-aminoethyl, 2-N-(C1-C4 alkyl)aminoethyl, 2-N,N-di(C1-C4 alkyl)aminoethyl; phenyl, pyridyl, pyrimidyl, benzyl, allyl, cyclopropyl, cyclobutyl, cyclopentyl, cyclohexyl, cyanomethyl, 2-cyanoethyl, 2-methansulfonylethyl, hydroxymethyl, 2-hydroxyethyl, 3-hydroxypropyl, (C1-C4 alkoxy)methyl, 2-(C1-C4 alkoxy)ethyl, 3-(C1-C4 alkoxy)propyl, N-methylpiperidyl, 1-phenylethyl, pyridylmethyl, N-methylpiperidylmethyl, 2-morpholinylethyl, morpholinocarbonylethyl, N,N-dimethylcarbonylmethyl, anilinocarbonylmethyl, N-methylanilinocarbonylmethyl, N-pyrrolidinocarbonylmethyl, N,N-dimethylsulfonyl, or oxo;
R7 is -H, methyl, -CH₂OH, or -CF₃;
a salt of one of the compounds, a stereoisomer of one of the compounds, or a salt of a stereoisomer of one of the compounds.

6. Compound according to any preceeding claim, wherein R1 is N-methylpiperazine.

7. Compound according to any of the preceeding claims, wherein R2 is 2-chlorphenyl or 2-trifluormethylphenyl.

8. Compound according to claim 1, the compound being
5-(6-Chloro-1 H-imidazo[4,5-c]pyridin-1-yl)-3-{[2-(trifluoromethyl)benzyl]oxy}thiophene-2-carboxamide,
5-(1H-Imidazo[4,5-c]pyridin-1-yl)-3-{[2-(trifluoromethyl)benzyl]oxy}thiophene-2-carboxamide,
5-(3H-Imidazo[4,5-c]pyridin-3-yl)-3-{[2-(trifluoromethyl)benzyl]oxy}thiophene-2-carboxamide,
1-(5-Carbamoyl-4-{[2-(trifluoromethyl)benzyl]oxy}-2-thienyl)-N-(2-methoxyethyl)-1 H-imidazo[4,5-c]pyridine-6-carboxamide,
1-(5-Carbamoyl-4-{[2-(trifluoromethyl)benzyl]oxy}-2-thienyl)-N-(2-morpholin-4-ylethyl)-1 H-imidazo[4,5-c]pyridine-6-carboxamide,
5-{6-[(Diethylamino)methyl]-1 H-imidazo[4,5-c]pyridin-1-yl}-3-{[2-(trifluoromethyl)benzyl]oxy}thiophene-2-carboxamide,
5-{6-[(Cyclopropylamino)methyl]-1H-imidazo[4,5-c]pyridin-1-yl}-3-{[2-(trifluoromethyl)benzyl]oxy}thiophene-2-carboxamide,
5-{6-[(4-Methylpiperazin-1-yl)methyl]-1H-imidazo[4,5-c]pyridin-1-yl}-3-{[2-(trifluoromethyl)benzyl]oxy}thiophene-2-carboxamide,
5-[6-(Hydroxymethyl)-1H-imidazo[4,5-c]pyridin-1-yl]-3-{[2-(trifluoromethyl)benzyl]oxy}thiophene-2-carboxamide,
5-[6-(Hydroxymethyl)-3 H-imidazo[4,5-c]pyridin-3-yl]-3-{[2-(trifluoromethyl)benzyl]oxy}thiophene-2-carboxamide,
1-(5-Carbamoyl-4-{[2-(trifluoromethyl)benzyl]oxy}-2-thienyl)-N-methyl-N-(2-morpholin-4-ylethyl)-1 H-imidazo[4,5-c]pyridine-6-carboxamide,
3-[1-(2-Chlorophenyl)ethoxy]-5-(3H-imidazo[4,5-c]pyridin-3-yl)thiophene-2-carboxamide,
5-(3H-Imidazo[4,5-c]pyridin-3-yl)-3-{1-[2-(trifluoromethyl)phenyl]ethoxy}thiophene-2-carboxamide,
5-(1 H-Imidazo[4,5-c]pyridin-1-yl)-3-{1-[2-(trifluoromethyl)phenyl]ethoxy}thiophene-2-carboxamide,
3-[1-(2-Chlorophenyl)ethoxy]-5-(1H-imidazo[4,5-c]pyridin-1-yl)thiophene-2-carboxamide,
5-(6-Methoxy-1H-imidazo[4,5-c]pyridin-1-yl)-3-{[2-(trifluoromethyl)benzyl]oxy}thiophene-2-carboxamide,
5-(6-Methoxy-3H-imidazo[4,5-c]pyridin-3-yl)-3-{[2-(trifluoromethyl)benzyl]oxy}thiophene-2-carboxamide,
5-(6-Methoxy-1H-imidazo[4,5-c]pyridin-1-yl)-3-{[2-(trifluoromethyl)benzyl]oxy}thiophene-2-carboxylic acid,
5-(3H-Imidazo[4,5-b]pyridin-3-yl)-3-{[2-(trifluoromethyl)benzyl]oxy}thiophene-2-carboxamide,
5-(1H-Imidazo[4,5-b]pyridin-1-yl)-3-{[2-(trifluoromethyl)benzyl]oxy}thiophene-2-carboxamide,
5-(5,6-Dimethoxy-1 H-imidazo[4,5-b]pyridin-1-yl)-3-{[2-(trifluoromethyl)benzyl]oxy}thiophene-2-carboxamide,
5-(5,6-Dimethoxy-3H-imidazo[4,5-b]pyridin-3-yl)-3-{[2-(trifluoromethyl)benzyl]oxy}thiophene-2-carboxamide,
5-(5-methoxy-3H-imidazo[4,5-b]pyridin-3-yl)-3-{[2-(trifluoromethyl)benzyl]oxy}thiophene-2-carboxamide,
5-(5-methoxy-1H-imidazo[4,5-b]pyridin-1-yl)-3-{[2-(trifluoromethyl)benzyl]oxy}thiophene-2-carboxamide,
a salt of one of the compounds, a stereoisomer of one of the compounds, or a salt of a stereoisomer of one of the compounds.

9. Compound, pharmaceutically acceptable salt thereof, stereoisomer of the compound or of a pharmaceutically acceptable salt thereof according to any of claims 1 to 8 for use in the treatment or prophylaxis of diseases.

10. Pharmaceutical composition comprising at least one of the compounds, pharmaceutically acceptable salts thereof, stereoisomers of the compound or of a pharmaceutically acceptable salt thereof according to any of claims 1 to 8 together with at least one pharmaceutically acceptable auxiliary.

11. Pharmaceutical composition according to claim 10, further comprising at least one therapeutic agent being an anti-neoplastic agent, a cytostatic agent, a cytotoxic agent, an antimetabolite, a microtubule interfering agent, a platinum derivative, an alkylating agent, a topoisomerase I inhibitor, a topoisomerase II inhibitor, a signal transduction inhibitor, a cell cycle signalling inhibitor, an angiogenesis inhibitor including an antibody or a receptor tyrosine kinase inhibitor, an immunotherapeutic agent, a monoclonal antibody, a proapoptotic agent, a hormone, a hormone analogue, an antibiotic agent, an anti-emetic agent, or an analgetic agent.

12. Use of a compound, a pharmaceutically acceptable salt thereof, a stereoisomer of the compound or of a pharmaceutically acceptable salt thereof according to any of claims 1 to 8 in the manufacture of a pharmaceutical composition inhibiting PIk1.

13. Use of a compound, a pharmaceutically acceptable salt thereof, a stereoisomer of the compound or of a pharmaceutically acceptable salt thereof according to any of claims 1 to 8 in the manufacture of a pharmaceutical composition for the treatment or prophylaxis of neoplasms or other proliferative diseases.

14. Use according to claim 13, wherein the neoplasm is a cancer of the colon including colorectal carcinoma, ovaries, breast, prostate, bladder, or lung, gastrointestinal carcinoma, gastrointestinal stromal tumor, small cell lung cancer, head and neck cancer, a cancer of cervix, pancreas, esophagus, kidney, larynx and hypopharynx, liver, endocrine glands, soft tissue, testis, retinoblastoma and Wilms tumor, endometrial tumors, malignant melanoma, non-Hodgekins lymphoma, Hodgekins lymphoma or other lymphomas, chronic and acute myeloic leukemia, acute lymphoblastic leukemia, T-cell lymphoma, plasma cell neoplasia, and leukemias, hematological malignancies including multiple myeloma, soft tissue sarcoma, osteosarcoma, fibrosarcoma, and other tumors of mesothelial origin, glioma, astrocytoma, cancers of unknown primary site, glioblastoma and other brain tumors and tumors of neuronal origin, germ cell cancers, myelodysplastic syndromes, myeloproliferative syndromes, disorders such as polycythemia vera, essential thrombocytopenia, myelofibrosis, hypereosinophilic syndrome, or other paraneoplastic syndromes.

15. Method of treating or preventing a neoplasm or another proliferative disease comprising administering to a patient in need thereof a therapeutically effective amount of a compound, a pharmaceutically acceptable salt thereof, a stereoisomer of the compound or of a pharmaceutically acceptable salt thereof according to any of claims 1 to 8.

16. Method of treating or preventing a neoplasm, in which the neoplasm is is a cancer of the colon including colorectal carcinoma, ovaries, breast, prostate, bladder, or lung, gastrointestinal carcinoma, gastrointestinal stromal tumor, small cell lung cancer, head and neck cancer, a cancer of cervix, pancreas, esophagus, kidney, larynx and hypopharynx, liver, endocrine glands, soft tissue, testis, retinoblastoma and Wilms tumor, endometrial tumors, malignant melanoma, non-Hodgekins lymphoma, Hodgekins lymphoma or other lymphomas, chronic and acute myeloic leukemia, acute lymphoblastic leukemia, T-cell lymphoma, plasma cell neoplasia, and leukemias, hematological malignancies including multiple myeloma, soft tissue sarcoma, osteosarcoma, fibrosarcoma, and other tumors of mesothelial origin, glioma, astrocytoma, cancers of unknown primary site, glioblastoma and other brain tumors and tumors of neuronal origin, germ cell cancers, myelodysplastic syndromes, myeloproliferative syndromes, disorders such as polycythemia vera, essential thrombocytopenia, myelofibrosis, hypereosinophilic syndrome, or other paraneoplastic syndrome.
